(19) Europäisches Patentamt
European Patent Office
Office européen des brevets

(11) Veröffentlichungsnummer: **0 300 249 B1**

(12) **EUROPÄISCHE PATENTSCHRIFT**

(45) Veröffentlichungstag der Patentschrift: **03.02.93**

(21) Anmeldenummer: **88110533.2**

(22) Anmeldetag: **01.07.88**

(51) Int. Cl.5: **C07D 207/337**, C07D 207/333, C07D 405/06, C07F 9/54, A61K 31/40

Die Akte enthält technische Angaben, die nach dem Eingang der Anmeldung eingereicht wurden und die nicht in dieser Patentschrift enthalten sind.

(54) **7-[1H-Pyrrol-3-yl]-substituierte 3,5-Dihydroxy-heptansäuren, ihre entsprechenden delta-Lactone und Salze, Verfahren zu ihrer Herstellung, ihre Verwendung als Arzneimittel, pharmazeutische Präparate und Zwischenprodukte.**

(30) Priorität: **10.07.87 DE 3722806**

(43) Veröffentlichungstag der Anmeldung: **25.01.89 Patentblatt 89/04**

(45) Bekanntmachung des Hinweises auf die Patenterteilung: **03.02.93 Patentblatt 93/05**

(84) Benannte Vertragsstaaten: **AT BE CH DE ES FR GB GR IT LI LU NL SE**

(56) Entgegenhaltungen:
**EP-A- 0 179 559**
**EP-A- 0 221 025**
**EP-A- 0 287 890**

**CHEMICAL ABSTRACTS, Band 93, Nr. 25, 22. Dezember 1980, Columbus, Ohio, US; SANKYO CO. LTD.: "Hypolipemic alkyl 3,5-dihydroxypentanoates", Seite 821, Spalte 1, Zusammenfassung-Nr. 239 089t**

(73) Patentinhaber: **HOECHST AKTIENGESELLSCHAFT**
**Postfach 80 03 20**
**W-6230 Frankfurt am Main 80(DE)**

(72) Erfinder: **Jendralla, Heiner, Dr.**
**Pfortengartenweg 38**
**W-6230 Frankfurt am Main 80(DE)**
Erfinder: **Beck, Gerhard, Dr.**
**Gustav-Freytag-Strasse 24**
**W-6000 Frankfurt am Main(DE)**
Erfinder: **Baader, Ekkehard, Dr.**
**Amselweg 14**
**W-6240 Königstein/Taunus(DE)**
Erfinder: **Kerekjarto, Bela, Dr.**
**Weilbächer Wälder FA8**
**W-6238 Hofheim am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF MEDICINAL CHEMISTRY, Band 28, Nr. 3, 1985; G.E. STOKKER et al.: "3-hydroxy-3-methylglutaryl-coenzyme A reductase inhibitors. 1. Structural modification of 5-substituted 3,5-dihydroxypentanoic acids and their lactone derivatives", Seiten 347-358

JOURNAL OF MEDICINAL CHEMISTRY, Band 29, Nr. 2, Februar 1986, W.F. HOFFMAN et al.: "3-hydroxy-3-methylglutaryl-coenzyme A reductase inhibitors. 2. Structural modification of 7-(substituted aryl)-3,5-dihydroxy-6-heptenoic acids and their lactone derivatives", Seiten 159-169

JOURNAL OF MEDICINAL CHEMISTRY, Band 29, Nr.2, Februar 1986; G.E. STOKKER et al.: "3-hydroxy-3-methylglutaryl-coenzyme A reductase inhibitors. 3. 7-(3,5-disubstituted[1,1'-biphenyl]-2-yl)-3,5-dihydroxy-6-heptenoic acids and their lactone derivatives", Seiten 170-181

PHOSPHORUS, Band 2, Nr. 1, Juli 1972, Gordon and Breach Science Publishers Ltd., Oxford, GB; E. ZBIRAL et al.: "Ueber eine neuartige Synthese von Pyrrol- und Imidazol-abkömmlingen mit Hilfe von Triphenyl-beta-acylvinylphosphoniumsalzen-", Seiten 29-34

JOURNAL OF MEDICINAL CHEMISTRY, Band 33, Seiten 61-70, 1990

**Beschreibung**

Hypercholesterolämie ist einer der primären Risikofaktoren für cardiovasculäre Erkrankungen wie Arteriosklerose. Es ist bekannt, daß das Enzym 3-Hydroxy-3-methylglutaryl-Coenzym A-Reduktase (HMG-CoA-Reduktase) die Bildung von Mevalonsäure aus 3-Hydroxy-3-methyl-glutaryl-Coenzym A (HMG-CoA) katalysiert. Diese Reaktion spielt eine zentrale Rolle bei der Biosynthese des Cholesterins. Derivate der 3-Hydroxy-3-methylglutarsäure (HMG) und der Mevalonsäure sind als Hemmer der Cholesterinbiosynthese beschrieben worden. Solche Verbindungen liegen zum Beispiel in Form der natürlichen Fermentations-Produkte Compactin und Mevinolin, einer Reihe daraus hergestellter semisynthetischer Derivate und verschiedener vollsynthetischer Analoger vor.

So beschreiben z.B. G. E. Stokker et al. (J. Med. Chem. 28, 347-358 (1985)) in 7-Stellung substituierte 3,5-Dihydroxy-hept-6(E)-ensäuren und 3,5-Dihydroxyheptansäuren, bei denen der 7-Substituent Phenyl, Phenanthryl, Dekalinyl und Adamantyl ist. W. F. Hoffmann et al. (J. Med. Chem. 29, 159-69 (1986)) beschreiben in 7-Stellung durch substituierte Phenylgruppen substituierte 3,5-Dihydroxy-hept-6(E)-ensäuren. Ferner berichten G. E. Stokker et al. (J. Med. Chem. 29, 170-181 (1986) über in 7-Stellung durch Biphenyle substituierte 3,5-Dihydroxy-hept-6(E)-ensäuren und deren δ-Lactone. Die von den genannten Autoren beschriebenen Verbindungen haben nur schwache Hemmwirkung auf die HMG-CoA-Reduktase.

In der europäischen Patentanmeldung mit der Veröffentlichungs-Nr. 179.559 werden 7-substituierte 3,5-Dihydroxy-heptansäuren beschrieben, bei denen der 7-Substituent substituiertes 1H-Pyrrol ist; der Pyrrol-Stickstoff ist an C-7 der Dihydroxyheptansäure gebunden (also 1H-Pyrrol-1-yl).

Schließlich werden in der europäischen Patentanmeldung mit der Veröffentlichungs-Nr. 221 025 u.a. 3,5-Dihydroxy-hept-6-ensäuren und 3,5-Dihydroxy-heptansäuren beansprucht, die in 7-Stellung eine gegebenenfalls substituierte 1H-Pyrrol-2-yl oder 1H-Pyrrol-3-yl Gruppe tragen. Es werden aber nur 1H-Pyrrol-2-yl substituierte Verbindungen beschrieben. Weder 1H-Pyrrol-3-yl substituierte Verbindungen noch Verfahren zu ihrer Herstellung werden offenbart.

Die Erfindung betrifft daher neue 7-[1H-Pyrrol-3-yl]-substituierte 3,5-dihydroxy-heptansäuren und deren Derivate der allgemeinen Formel I

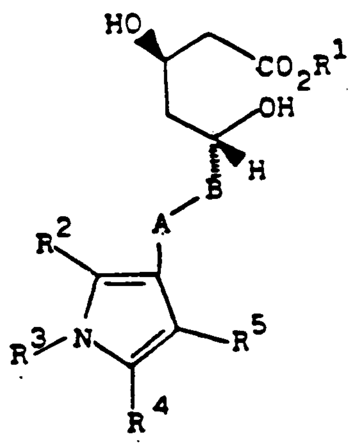

(I)

sowie die entsprechenden δ-Lactone der Formel II

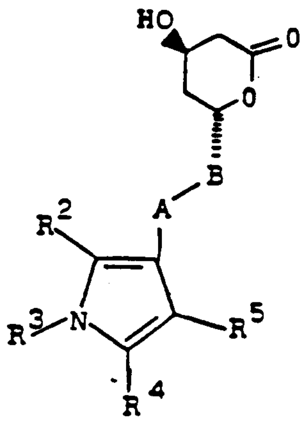

(II)

sowie Verfahren zu ihrer Herstellung .

In den Formeln bedeuten:

A-B eine Ethandiyl-Gruppierung $-CH_2-CH_2-$,

$R^1$ H, Alkyl mit 1 bis 4 C-Atomen, Phenyl, Benzyl oder 2,3-Dihydroxypropyl, oder ein pharmakologisch verträgliches Alkali- oder Erdalkalimetallkation, $NH_4^+$ oder ein mit 1 bis 4 Alkylgruppen mit jeweils 1-4 C-Atomen substituiertes Ammoniumion,

$R^2$, $R^3$, $R^4$, $R^5$ unabhängig voneinander

1) Wasserstoff
2) geradkettiges oder verzweigtes Alkyl mit 1-12 Kohlenstoff-Atomen, Cycloalkyl mit 5-7 C-Atomen, das gegebenenfalls über eine geradkettige oder verzweigte Alkylkette von 1-5 Kohlenstoffatomen an den heterocyclischen Aromaten gebunden ist,
3) Phenyl oder Benzyl, unsubstituiert oder 1-3 fach substituiert mit
   a) Fluor, Chlor, Brom oder Trifluormethyl,
   b) Alkyl oder Alkenyl mit bis zu 5 Kohlenstoffatomen, wobei orthoständige Alkyl- oder Alkenyl-Substituenten unter Ausbildung eines anellierten carbocyclischen Rings verbunden sein können,
   c) Alkoxy mit 1-5 Kohlenstoffatomen
   d) Alkoxycarbonyl mit 2-6 Kohlenstoffatomen,

wobei die gleichzeitige Bedeutung $R^1$ Methyl, $R^2$ i-Propyl, $R^3$ Methyl, $R^4$ Phenyl und $R^5$ 4-Fluorphenyl ausgenommen ist.

Beispiele für wie unter 3)b) definierte Gruppen $R^2$ bzw. $R^5$ haben folgende Formeln:

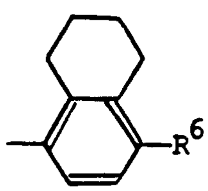

,

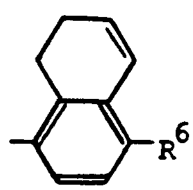

$R^6$=H oder F

Die Reste haben bevorzugt folgende Bedeutungen:

A-B $-CH_2-CH_2-$

$R^1$ Methyl, Ethyl oder Na

$R^2$, $R^3$, $R^4$, $R^5$ unabhängig voneinander

1) Wasserstoff
2) Methyl, Ethyl, Isopropyl, tert.-Butyl, Cyclohexyl
3) Phenyl, das 1-2fach substituiert sein kann mit
   a) Fluor, Chlor
   b) Methyl

wobei die gleichzeitige Bedeutung $R^1$ Methyl, $R^2$ i-Propyl, $R^3$ Methyl, $R^4$ Phenyl und $R^5$ 4-Fluorphenyl ausgenommen ist.

Unter den zu den allgemeinen Formeln I und II genannten Resten sind besonders bevorzugt

$R^1$ Methyl, Ethyl oder Na

$R^2$, $R^5$ unabhängig voneinander Isopropyl, p-Fluorphenyl, p-Fluor-m-methylphenyl
$R^3$, $R^4$ unabhängig voneinander Wasserstoff, Isopropyl, Cyclohexyl, Phenyl,
wobei die gleichzeitige Bedeutung $R^1$ Methyl, $R^2$ i-Propyl, $R^3$ Methyl, $R^4$ Phenyl und $R^5$ 4-Fluorphenyl ausgenommen ist.

Die Erfindung betrifft die reinen Enantiomeren sowie die Racemate der Formel I und Mischungen aus diesen, also die Racemate mit der absoluten Konfiguration

3R/5R bzw. 3S/5S für A-B gleich $-CH_2-CH_2-$

sowie die reinen Enantiomeren

3R/5R für A-B gleich $-CH_2-CH_2-$.

Die Erfindung betrifft ferner die reinen Enantiomeren sowie die Racemate der allgemeinen Formel II, die aus den oben genannten stereoisomeren offenkettigen Dihydroxycarbonsäuren der allgemeinen Formel I hervorgehen. Im einzelnen sind das die Racemate mit den absoluten Konfigurationen

3R/5R bzw. 3S/5S für A-B gleich $-CH_2-CH_2$

sowie die reinen Enantiomeren

3R/5R für A-B gleich $-CH_2-CH_2-$.

Die Verbindungen der Formeln I und II sind starke Hemmstoffe der HMG-CoA-Reduktase.

Die Erfindung betrifft daher ferner die Verwendung dieser Verbindungen, insbesondere zur Behandlung der Hypercholesterinämie, sowie pharmazeutische Präparate.

Die Verfahren zur Herstellung der Verbindungen der Formeln I und II sind dadurch gekennzeichnet, daß man

A)

1. einen Carbaldehyd der Formel III

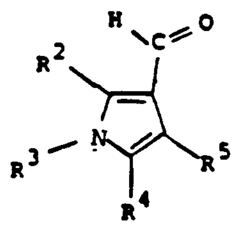

III

reduziert zu einem Alkohol der Formel IV

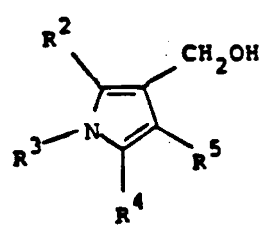

IV

2. den Alkohol IV in das Halogenderivat der Formel V

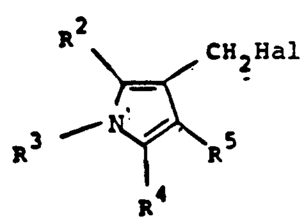

V

worin Hal Chlor, Brom oder Jod bedeutet, überführt

3. die Halogenverbindungen der Formel V in das Phosphoniumsalz der Formel VI

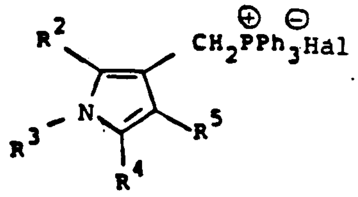

VI

worin Hal Chlor, Brom oder Jod bedeutet, überführt,
4. das Phosphoniumsalz der Formel VI mit dem Compactinaldehyd der Formel VII

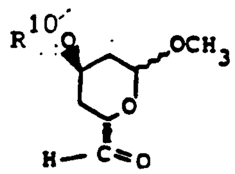

VII

worin $R^{10}$ eine gegen Basen und schwache Säuren stabile Schutzgruppe bedeutet, z.B. t-Butyldiphenylsilyl, umsetzt zu dem Lactolether der Formel VIII

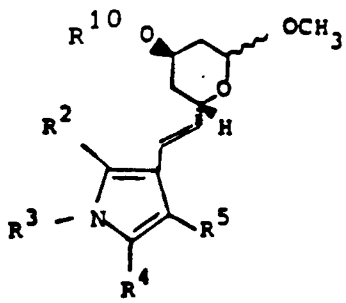

VIII

5. in dem Lactolether der Formel VIII die Methylacetalfunktion sauer hydrolysiert zu einem Lactol der Formel IX

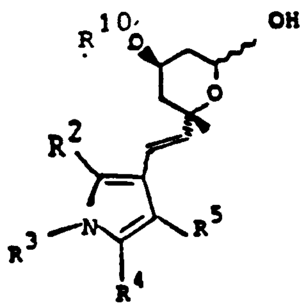

IX

6. das Lactol der Formel IX oxydiert zu einem Lacton der Formel X

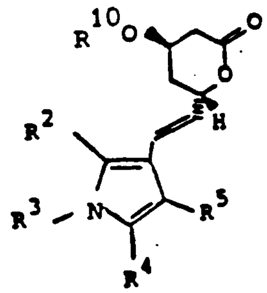

X

7. in dem Lacton der Formel X die Schutzgruppe $R^{10}$, z.B.

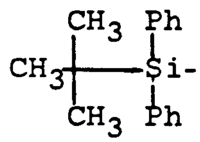

abspaltet zu einer Verbindung der Formel IIa

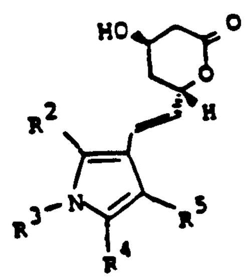

IIa

worin $R^2$ bis $R^5$ die angegebenen Bedeutungen haben und

8. gegebenenfalls eine erhaltene Verbindung der Formel IIa, worin $R^2$ bis $R^5$ die angegebenen Bedeutungen haben in die entsprechende Dihydroxy-hept-6-ensäure der Formel Ia

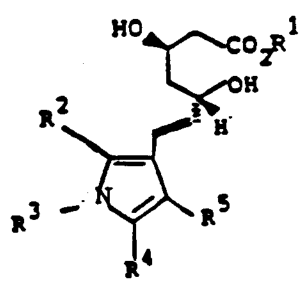

Ia

worin $R^1$ bis $R^5$ die angegebenen Bedeutungen haben, überführt und

9. in einer erhaltenen Verbindung der Formel Ia oder IIa mit -CH=CH- die Doppelbindung hydriert zu einer Verbindung der Formel I oder II mit A-B gleich $-CH_2-CH_2-$, wobei die Hydrierung auch bei den Zwischenstufen VIII, IX oder X sowie auch vor der Überführung von Verbindungen IIa nach Ia erfolgen kann, oder

B)

1. den $\alpha$, $\beta$ ungesättigten Aldehyd der Formel XI

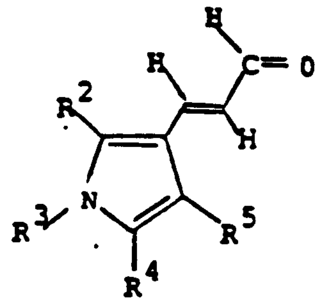

XI

einer Aldolreaktion mit dem Dianion des Acetessigsäuremethylesters unterwirft, wobei eine Verbindung der Formel XII

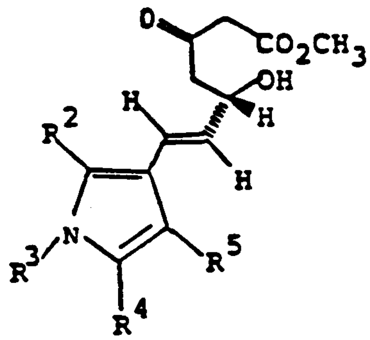

XII

entsteht,
2. in einer Verbindung der Formel XII die Ketogruppe stereoselektiv reduziert zu einer Verbindung der Formel Ia

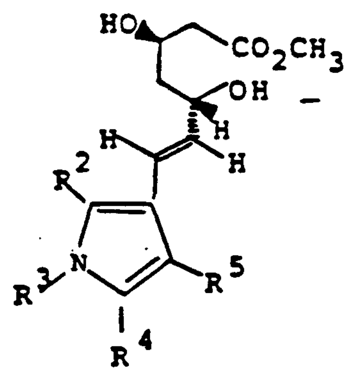

Ia

worin $R^2$ bis $R^5$ die angegebenen Bedeutungen haben, $R^1$ die Methylgruppe ist,
3. gegebenenfalls eine erhaltene Verbindung der Formel Ia, worin $R^1$ die $CH_3$-Gruppe bedeutet, in eine Verbindung Ia überführt, worin $R^1$ bis $R^5$ die zu Formel I angegebenen Bedeutungen haben und
4. in einer erhaltenen Verbindung der Formel Ia die Doppelbindung hydriert zu einer Verbindung der Formel I mit A-B gleich $-CH_2-CH_2-$, und
5. gegebenenfalls eine erhaltene Verbindung der Formel I mit $R^1 = H$ unter Abspaltung von Wasser in ein Lacton der Formel II überführt, oder

C)
1. das Enolat eines Essigsäureesters der Formel XIII

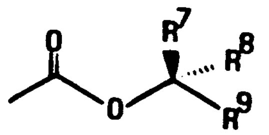

XIII

worin $R^7$ einen Phenylring, der unsubstituiert ist oder substituiert ist mit Fluor, Chlor, Brom, verzweigtem oder unverzweigtem Alkyl mit 1-4 Kohlenstoffatomen, $R^8$ ein H-Atom oder Methyl, und $R^9$ Diphenylhydroxymethyl $Ph_2COH$, wobei die Phenylringe unsubstituiert sind oder substituiert sind mit Fluor, Chlor, Brom, verzweigtem oder unverzweigtem Alkyl mit 1-4 Kohlenstoffatomen, bedeuten, in einer Aldol-Reaktion mit dem $\alpha$, $\beta$-ungesättigten Aldehyd der Formel XI

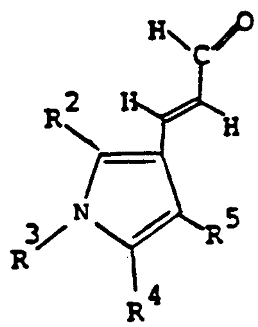

XI

zum Addukt der Formel XIV

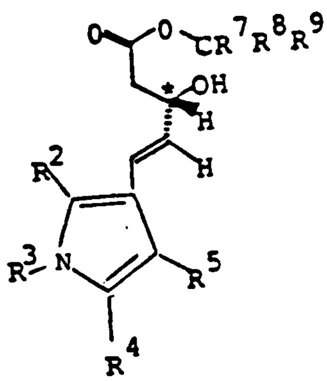

XIV

worin $R^2$ bis $R^5$ die zu Formel I und $R^7$, $R^8$, $R^9$ die zu Formel XIII angegebenen Bedeutungen haben, umsetzt,

2. das Addukt der Formel XIV durch Umesterung in den $\beta$-Hydroxymethylester der Formel XV

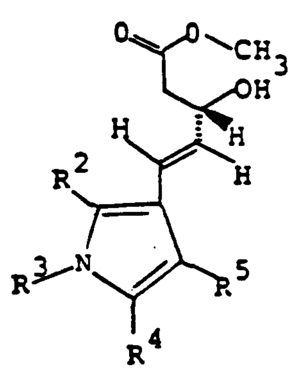

XV

überführt,

3. den erhaltenen Methylester der Formel XV mit dem Enolat eines Essigsäurealkylesters in den β-Keto-δ-(S)-hydroxyester der Formel XVI

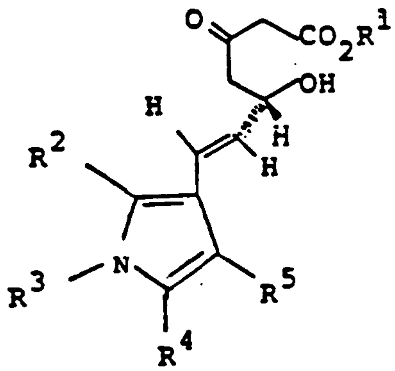

XVI

worin $R^2$ bis $R^5$ die zu Formel I angegebenen Bedeutungen haben und $R^1$ eine Alkylgruppe mit 1-4 C-Atomen ist, überführt und

4. in einer erhaltenen Verbindung der Formel XVI die Ketogruppe stereoselektiv reduziert zu einer Verbindung der Formel Ia

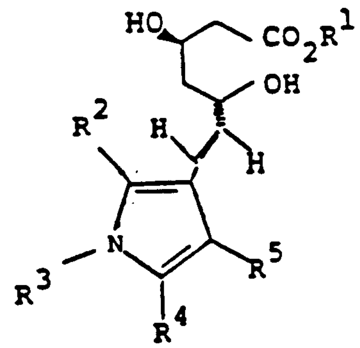

Ia

worin $R^2$ bis $R^5$ die zu Formel I angegebenen Bedeutungen haben, $R^1$ eine Alkylgruppe mit 1-4 C-Atomen ist

5. gegebenenfalls eine erhaltene Verbindung der Formel Ia, worin $R^1$ eine Alkylgruppe mit 1-4 C-Atomen ist, in eine Verbindung der Formel Ia überführt, worin $R^1$ bis $R^5$ die zu Formel I angegebenen Bedeutungen haben und

6. in einer erhaltenen Verbindung der Formel Ia die Doppelbindung hydriert zu einer Verbindung der Formel I mit A-B gleich $-CH_2-CH_2-$, und

7. gegebenenfalls eine erhaltene Verbindung der Formel I mit $R^1$ = Wasserstoff unter Abspaltung von Wasser in ein Lacton der Formel II überführt.

Verfahren A, dargestellt in Schema 1, wobei einige Verbindungen enger definiert werden, beruht auf der Wittig-Kupplung des aus dem Phosphoniumsalz VI erzeugten Ylids mit dem geschützten, optisch reinen "Compactinaldehyd" VII. Zur Synthese von VII aus tri-O-Acetyl-D-glucal siehe J. R. Falck, Tetrahedron Lett. 23, 4305 (1982) und die europäische Patentanmeldung mit der Veröffentlichungs-Nr. 217 092. Vorteil dieses Verfahrens ist, daß die Endprodukte der allgemeinen Formel I direkt optisch aktiv in der gewünschten Absolutkonfiguration erhalten werden. Bei der Kupplung von VI mit VII werden in der Regel Gemische von cis- und trans-Isomer erhalten. Außerdem erfordert die Hydrolyse des Lactolethers VIII zum Lactol IX mäßig bis stark saures Milieu. Unter diesen Bedingungen werden 1H-Pyrrole weitgehend hydrolytisch angegriffen. Nach diesem Verfahren erhält man die Verbindungen der Formel I und II.

Schema 1: Verfahren A zur Synthese von Verbindungen der allgemeinen Formeln I und II aus heterocyclischen Aldehyden III

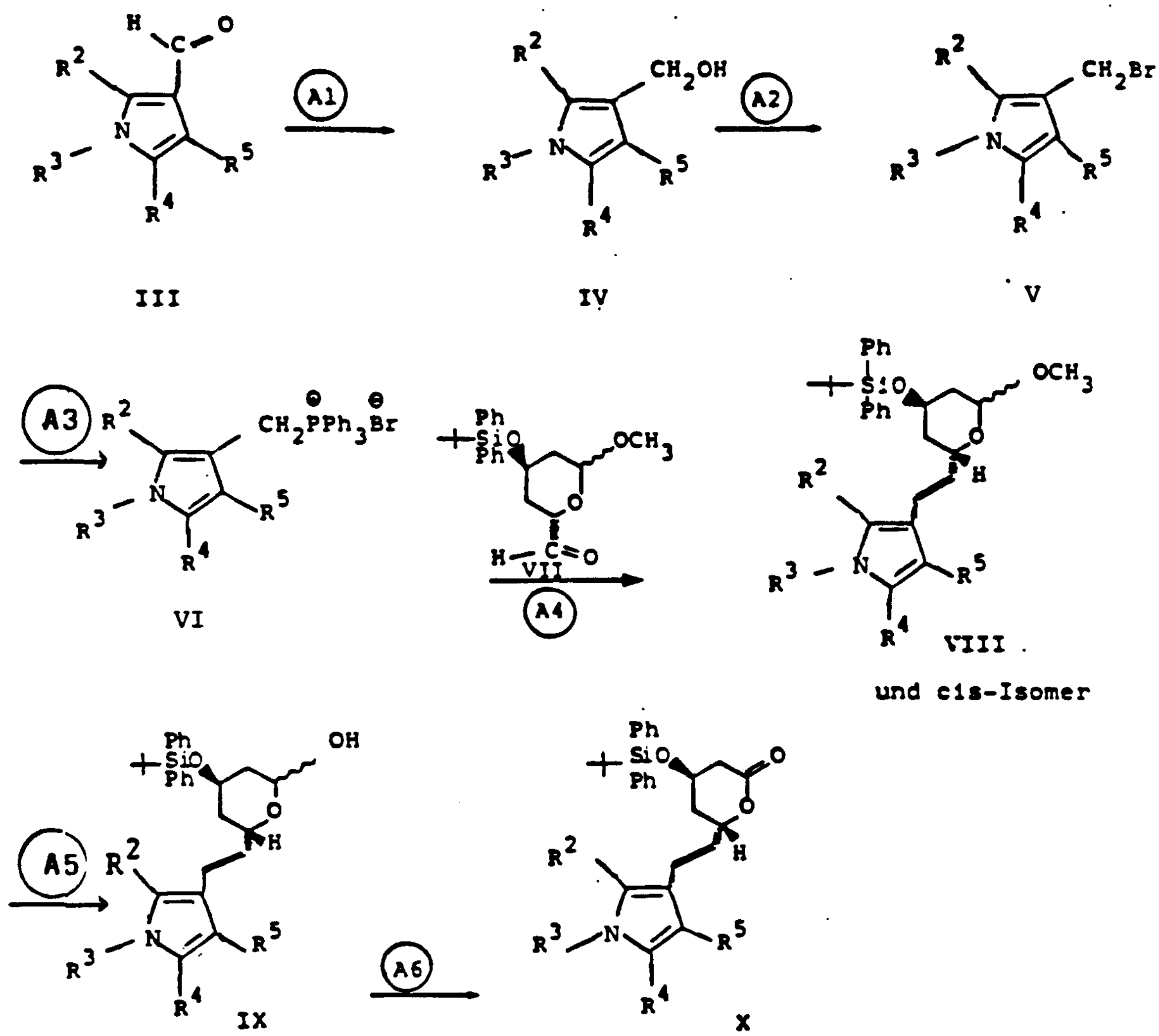


III IV V

VI VII VIII und cis-Isomer

IX X

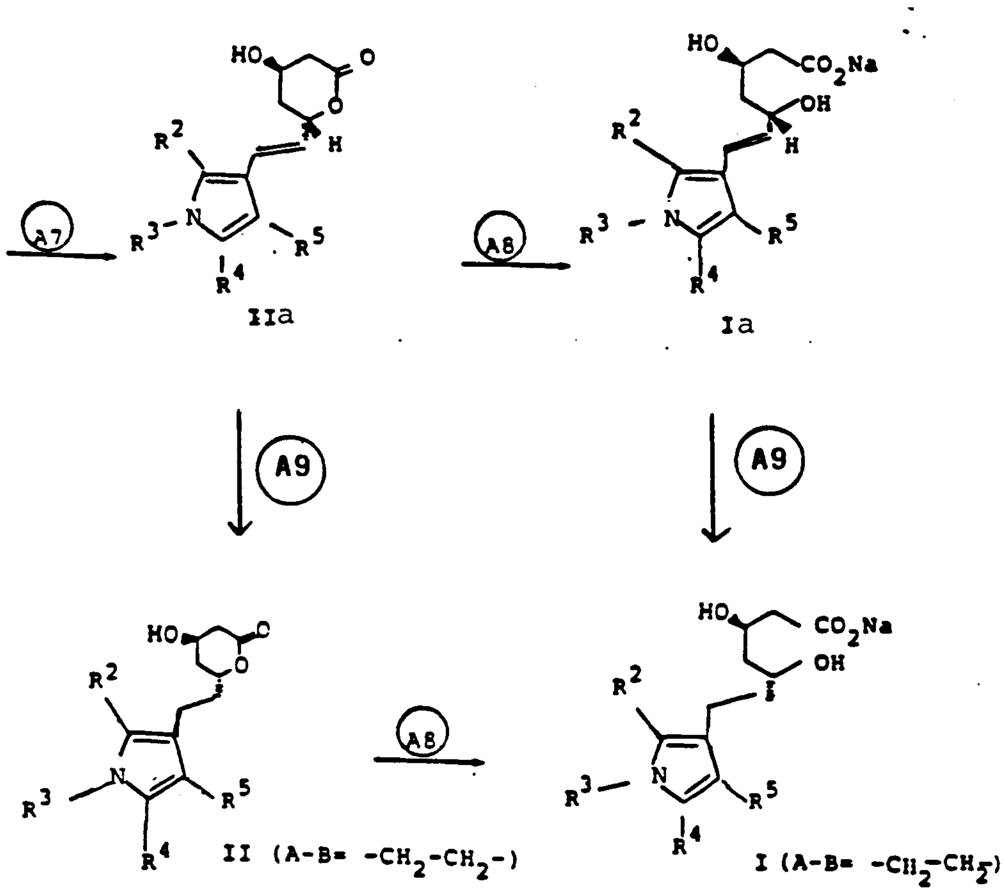


IIa

Ia

II (A-B= $-CH_2-CH_2-$)

I (A-B= $-CH_2-CH_2-$)

Verbindungen der Formel I worden vorteilhaft mit den Verfahren B und C hergestellt. Bei Verfahren B und C geht man aus vom $\alpha$, $\beta$-ungesättigten Aldehyd der Formel XI. Diesen erhält man z.B. durch Verlängerung des Carbaldehyds III mit Hilfe des "Wollenberg-Reagenzes" (R. H. Wollenberg et al., J. Am. Chem. Soc. 99, 7365 (1977). Die Reaktion folgt der Gleichung:

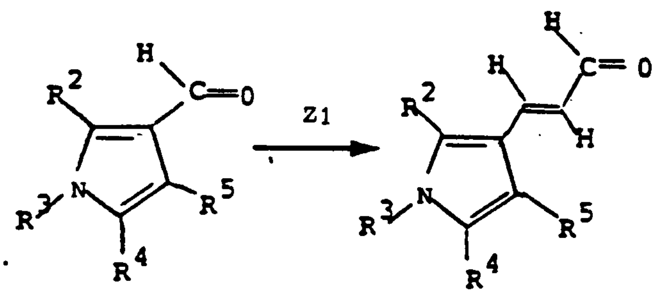


III

XI

nur trans

Ein weiteres Verfahren zur Herstellung der Verbindung XI basiert auf der basen-katalysierten Emmons-Horner-Kupplung des Carbaldehyds III mit einem Cyanomethylphosphonat, bevorzugt Diisopropylcyanomethylphosphonat (kommerziell erhältlich). Die entstehenden 3-Heteroaryl-substituierten Acrylnitrile XVIIkönnen dann mit verschiedenen Reduktionsmitteln, bevorzugt Diisobutylaluminiumhydrid, zu den Aldehyden XI reduziert werden. Für die Reaktion gilt das Formelschema:

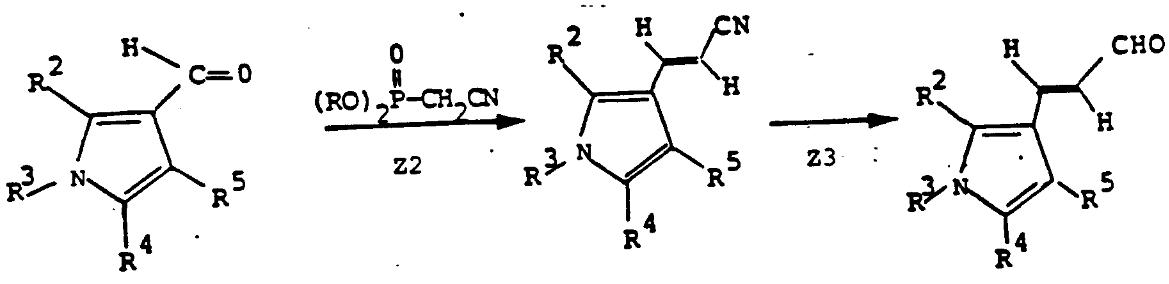


III XVII XI

und wenig cis-Isomer

Die Ausbeuten bei Herstellung von XI sind bei diesem Verfahren niedriger. Die trans/cis- Selektivität ist hoch.

Bei Verfahren B geht der Aldehyd XI eine Aldolreaktion mit dem Dianion des Acetessigsäuremethylesters ein. Die anschließende stereoselektive Reduktion der Ketogruppe führt zu Verbindungen der Formel Ia. Vorteile dieses Verfahrens sind, daß die Einführung der Doppelbindung mit über 95 %iger Ausbeute erfolgt und daß die Doppelbindung im Rahmen der analytischen Genauigkeit nur die gewünschte trans-Konfiguration aufweist. Ein weiterer Vorteil des Verfahrens ist, daß alle Verfahrensschritte unter basischen bis neutralen Bedingungen bei oder unterhalb Raumtemperatur durchgeführt werden können.

Die Endprodukte der allgemeinen Formel I können optisch rein in der gewünschten Absolutkonfiguration erhalten werden, indem man die racemischen Produkte I einer Racematspaltung unterwirft, z.B. unter Verwendung von d(+)-α-Methylbenzylamin gemäß US-Patent 4.567.289.

Verfahren C, dargestellt in Schema 2 (B2, A9 beziehen sich auf Schritte, die in Verfahren B bzw. A erwähnt werden), ist dadurch gekennzeichnet, daß das Enolat eines Essigsäureesters, bevorzugt das Magnesiumenolat eines Essigsäureesters mit optisch aktiver Alkohol-Komponente, besonders bevorzugt das Magnesiumenolat von S(+)-2-Acetoxy-1,1,2-triphenylethanol (Diss. R. M. Devant, Univ. Karlsruhe (1985), Seite 96-98), nach der Prozedur von Braun und Devant (Tetrahedron Lett. 25, 5031 (1984) bzw. von Lynch et al. (Tetrahedron Lett. 28, 1385 (1987)) in einer Aldol-Reaktion mit dem α,β-ungesättigten Aldehyd XI zum Addukt XIV umgesetzt wird. Bei Verwendung des Magnesiumenolats von S(+)-2-Acetoxy-1,1,2-triphenylethanol ist diese Addition hoch-diastereoselektiv und das Chiralitätszentrum von XIV entsteht zu 90 - 97 % in der gewünschten S-Konfiguration. Nach Umesterung zum Methylester XV liefert die Claisen-Kondensation mit dem Enolat eines Essigsäurealkylester, bevorzugt dem Lithiumenolat von Essigsäuretert.-butylester, den β-Keto-δ-(S)-hydroxyester XVI, der (in Analogie zu Verfahren B) stereoselektiv zum 3 (R), 5 (S)-Dihydroxy-hept-6 (E)-ensäureester Ia reduziert werden kann. Basische Verseifung und Hydrierung der erhaltenen Verbindungen liefern die Produkte der allgemeinen Formel I mit einem hohen Überschuß des gewünschten Enantiomeren (> 80 % ee). Die optisch reinen Verbindungen sind daraus durch Umkristallisieren zu erhalten.

Schema 2: Verfahren C zur Synthese von Verbindungen der allgemeinen Formel I aus 3-Heteroaryl-substituierten E-Propenalen XI

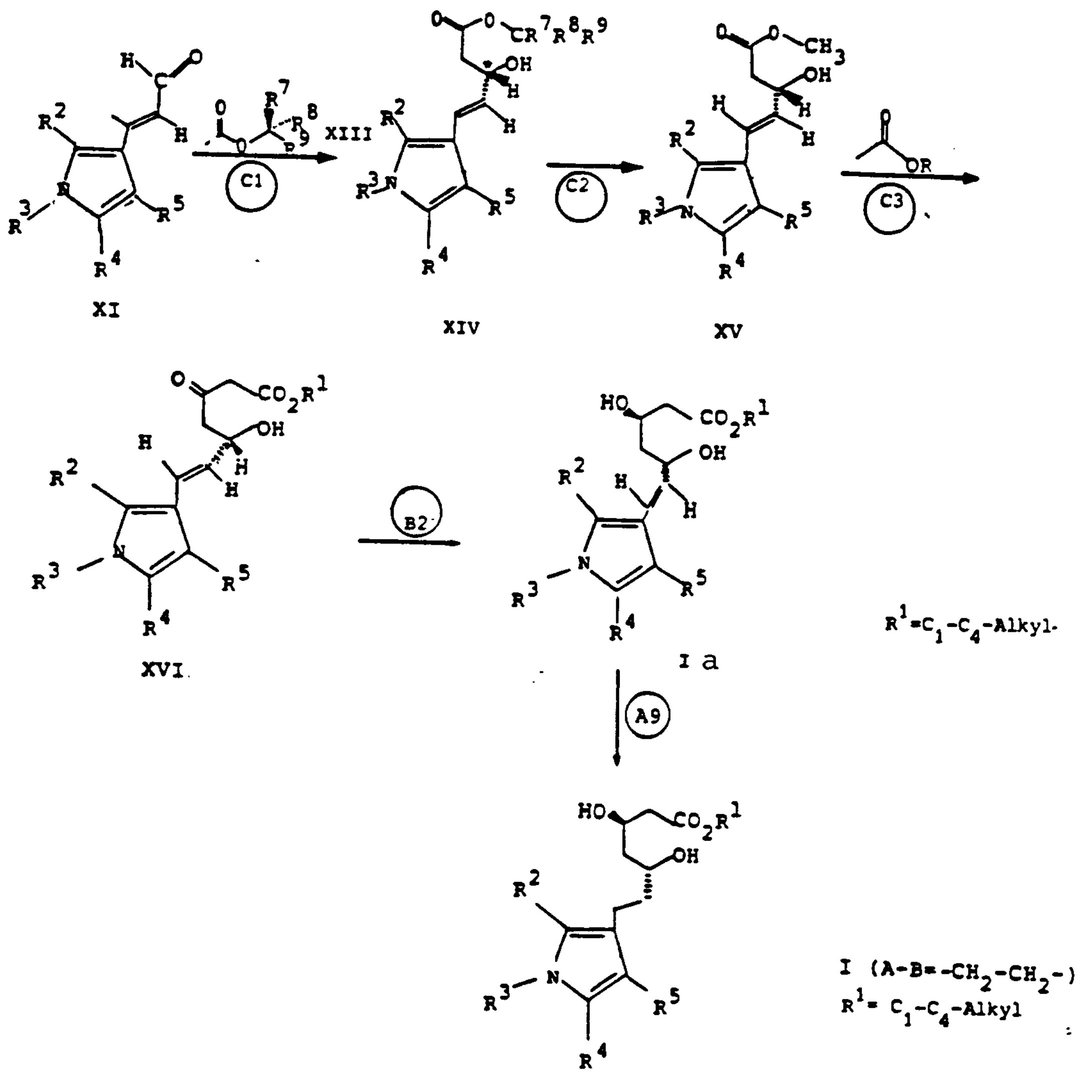


XI XIV XV

XVI Ia

$R^1 = C_1-C_4$-Alkyl.

I

I $(A-B = -CH_2-CH_2-)$
$R^1 = C_1-C_4$-Alkyl ;

In den Formel III-XVII haben $R^2$, $R^3$, $R^4$ $R^5$, die gleichen Bedeutungen, wie für die Formeln I bzw. II angegeben (siehe oben).

$R^7$ bedeutet einen Phenylring, der unsubstituiert ist oder substituiert sein kann mit Fluor, Chlor, Brom, verzweigtem oder unverzweigtem Alkyl mit 1-4 Kohlenstoffatomen. Bevorzugt bedeutet $R^7$-unsubstituiertes Phenyl. $R^8$ bedeutet ein H-Atom oder Methyl, bevorzugt ein H-Atom. $R^9$ bedeutet Diphenylhydroxymethyl $Ph_2COH$, wobei die Phenylringe bevorzugt unsubstituiert sind, aber auch substituiert sein können mit Fluor, Chlor, Brom, verzweigtem oder unverzweigtem Alkyl mit 1-4 Kohlenstoffatomen.

Als Ausgangsmaterialien für Verfahren A zur Synthese von Produkten der allgemeinen Formeln I und II sowie zur Herstellung der $\alpha$, $\beta$-ungesättigten Aldehyde der Formel XI werden heterocyclische Aldehyde der allgemeinen Formel III benötigt.

Heterocyclische Aldehyde der allgemeinen Formel III, in der die Substituenten $R^2$-$R^5$ die gleiche Bedeutung haben, wie für die allgemeinen Formeln I und II angegeben, können auf verschiedenen Wegen erhalten werden, die im Schema 3 zusammengestellt sind.

Schema 3: Verfahren zur Synthese der Aldehyde der allgemeinen Formel III

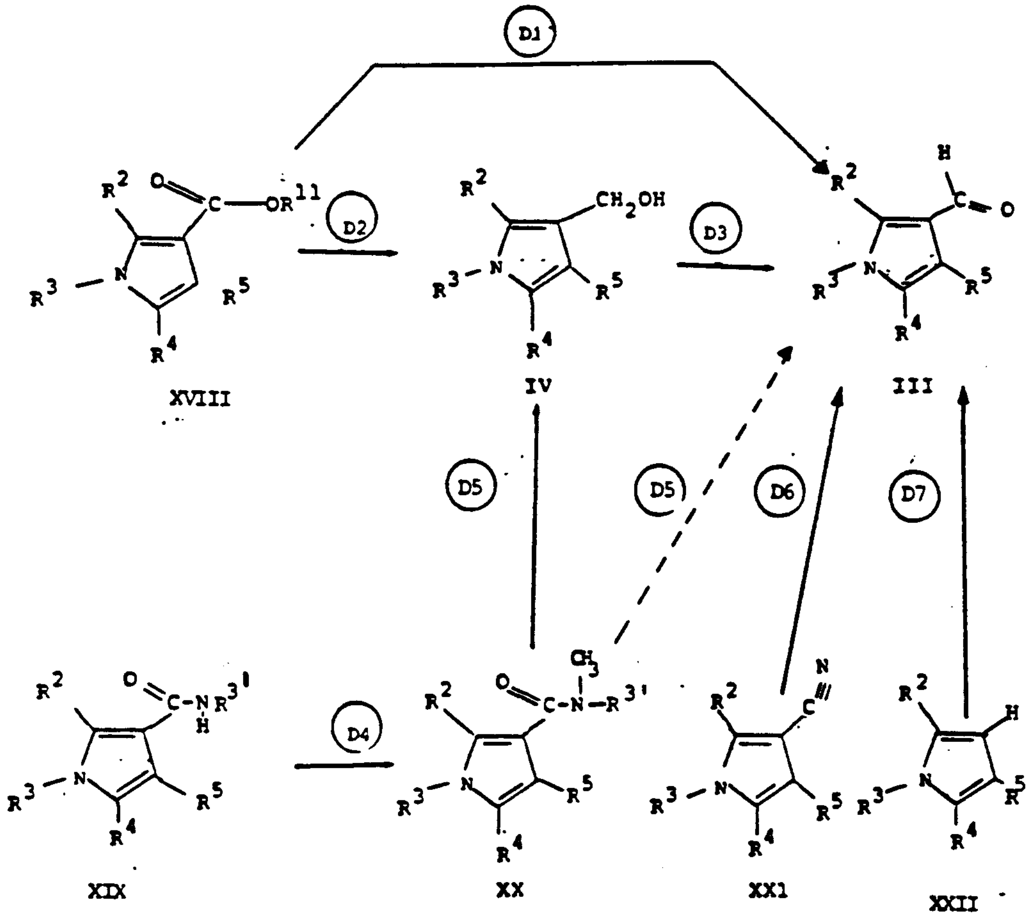


XVIII IV III XIX XX XXI XXII

In den Formeln XIX und XX bedeutet $R^{3'}$Phenyl oder durch die zu Formel I unter a) bis d) genannten Gruppen substituiertes Phenyl.

Heterocyclische Carbonsäurealkylester der allgemeinen Formel XVIII ($R^{11}$ = $C_1$-$C_4$-Alky), bevorzugt die Methylester ($R^{11}$ = $CH_3$), können unter bestimmten Bedingungen direkt zum Aldehyd der allgemeinen Formel III reduziert werden (D1). (siehe z.B. G. Benz in Houben-Weyl Band E3, Thieme Verlag (1983), S. 437 - 450). Bevorzugt wird jedoch, den Ester XVIII zunächst zum Alkohol IV zu reduzieren und dann zum Aldehyd III zu oxidieren. Sowohl für den Reduktionsschritt D2 als auch den Oxidationsschritt D3 existieren eine Vielzahl von Methoden (siehe z.B. A. Hajós in Houben-Weyl Bd. IV/1d Teil II, Thieme Verlag (1981), S. 195 - 199; H. Bornowski in Houben-Weyl Bd. E3, Thieme Verlag (1983), S. 265 ff und E. Campaigne, G. M. Shutske, J. Heterocyclic Chem. 12, 317 (1975).

Bevorzugt wird die Reduktion D2 mit Lithiumaluminiumhydrid oder mit Diisobutylaluminiumhydrid durchgeführt, die Oxidation D3 mit Mangandioxid oder mit Chromtrioxid in Pyridin. Alternativ kann die Oxidation D3 mit dem Fetizon-Reagenz ($Ag_2CO_3$ auf Celite, siehe M. Fetizon et al., Compt. Rend. Acad. Sc. Paris, Ser. C, 267, 900 (1968) und J. Heterocyclic Chem. 13, 525 (1976)) oder mit Pyridiniumchlorochromat (G. Piancatelli et al. Synthesis, 245 (1982)) oder mit Pyridiniumdichromat (E. J. Corey et al., Tetrahedron Lett. 20, 399 (1979)), durch Swern-Oxidation (D. Swern et al., Synthesis, 165 (1981)) oder mit N-Methylmorpholin-N-oxid unter Ruthenium-Katalyse (K. B. Sharpless et al., Tetrahedron Lett. 17, 2503 (1976); H. Tomioka et al., Tetrahedron Lett. 22, 1605 (1981)) durchgeführt werden.

Besonders bevorzugt wird der Reduktionsschritt D2 mit Lithiumaluminiumhydrid und der Oxidationsschritt D3 mit Mangandioxid oder mit N-Methylmorpholin-N-oxid/Tris(triphenylphosphin)-ruthenium(II)chlorid durchgeführt.

Aldehyde der allgemeinen Formel III sind auch aus Aniliden der allgemeinen Formel XIX erhältlich, indem man diese in die N-Methylanilide XX überführt und letztere mit Lithiumaluminiumhydrid zu Alkoholen der allgemeinen Formel IV reduziert (D4, D5). In Abhängigkeit von der Natur der Substituenten $R^2$ - $R^5$ kann diese Reduktion bei einigen Systemen auch direkt zum Aldehyd III führen. (F. Weygand, Angew. Chem. 65, 525 (1953)). Heterocyclische Nitrile der allgemeinen Formel XXI können durch komplexe Metallhydride,

bevorzugt mit Diisobutylaluminiumhydrid zu Aldehyden der Formel III reduziert werden.

Elektronenreiche Heterocyclen der allgemeinen Formel XXII können in einigen Fällen mittels einer Vilsmeier-Formylierung zu Aldehyden der Formel III umgewandelt werden (D7) (siehe z.B. G. Simchen in Houben-Weyl, Bd. E3, Thieme Verlag (1983), S. 57 ff).

Die Reaktionsbedingungen zur Durchführung der in den Verfahren A, (Schema 1), Verfahren B (einschließlich Herstellung der Ausgangsverbindung nach Schritt Z1, Z2, Z3), Verfahren C (Schema 2) und Schema 3 angegebenen Verfahrensschritte können Tabelle 1 und den Beispielen (siehe hinten) entnommen werden.

**Tabelle 1:** Verfahrensschritte zur Synthese von Verbindungen der allgemeinen Formel I

| Verfahrensschritte | Reagenz (Äquiv.) | Temperatur | Lösungsmittel | Inert-Atmosphäre |
|---|---|---|---|---|
| A1 | komplexes Metallhydrid (Überschuß), bevorzugt $LiAlH_4$ | -20 °C bis Rückfl., bevorzugt 25°C | Ether, THF, Benzol oder Toluol, bevorzugt Ether | ja |
| A2 | $PBr_3$ (0.5) | 0 - 25 °C | Benzol oder Toluol | - |
| A3 | $PPh_3$ (Überschuß) | 60 - 120 °C, bevorzugt 80 °C | Benzol oder Toluol | - |
| A4 | a) n-BuLi (1.0)<br>b) VII (0.9) | a) VI auf ~ 120 °C/ 2 h heizen, n-BuLi bei 5 - 10 °C, dann 25 °C/ 30 min<br>b) VII bei 25 °C | Ether, THF, Benzol, Toluol bevorzugt THF | ja |

| Verfahrensschritte | Reagenz (Äquiv.) | Temperatur | Lösungsmittel | Inert-Atmosphäre |
|---|---|---|---|---|
| A5 | $H_2O$/AcOH (Überschuß) | 25 °C-Rückfluß bevorzugt ca. 60°C | $H_2O$/THF bevorzugt ca. 1:1 | ja |
| | oder | | | |
| | $CF_3CO_2H$ (Überschuß) | 0 - 50 °C, bevorzugt 0 - 20 °C | | |
| A6 | $(n\text{-}Bu)_4NI$ (1.0) | 0 - 50 °C, bevorzugt 25 °C | inertes Lsgsm., | ja |
| | N-Iodsuccinimid (~5.0) | | bevorzugt $CH_2Cl_2$ | ja |
| | oder | | | |
| | $CrO_3$ (10.0), Pyridin (20.0) | 0 - 30 °C, bevorzugt 25 °C | $CH_2Cl_2$ | - |
| A7 | $(n\text{-}Bu)_4NF \cdot 3H_2O$ (~3.0) AcOH (~4.0) | -30 °C - +50 °C bevorzugt 0 - 25 °C | Ether, THF, Benzol oder Toluol bevorzugt THF | ja |
| | oder | | | |
| | 48 % HF/$H_2O$ (Überschuß) | 0 - 80 °C bevorzugt 25 °C | $CH_3CN$ | - |

| Verfahrens-schritte | Reagenz (Äquiv.) | Temperatur | Lösungsmittel | Inert-Atmosphäre |
|---|---|---|---|---|
| A8 | NaOH (1.0) | 0 - 50 °C, bevorzugt 25 °C | $H_2O$, $CH_3OH$ oder EtOH | - |
| A9 | Pd/C, $H_2$, 1 atm | 0 - 50 °C bevorzugt 25 °C | inertes Lsgsm., bevorzugt $CH_3OH$, Spur $NEt_3$ | |
| Z1 | 1-Tri-n-butylstannyl-2-ethoxy-ethylen (1.2) n-BuLi (1.25) | -78 °C - -40 °C bevorzugt -70 °C | Ether oder THF, bevorzugt THF | ja |
| B1 | $CH_3-\overset{O}{C}-CH_2-CO_2CH_3$ (1.75)<br>NaH (1.80)<br>n-BuLi (1.70) | a) NaH bei -15 °C/ 50 min<br>b) n-BuLi bei -15 °C/ 20 min<br>c) Aldehyd bei -15 °C → 0 °C/ 45 min<br>d) $NaH_2PO_4$/ $H_2O$ (Überschuß -10 °C → 25 °C | Ether oder THF, bevorzugt THF | ja |

| Verfahrens-schritte | Reagenz (Äquiv.) | Temperatur | Lösungsmittel | Inert-Atmosphäre |
|---|---|---|---|---|
| B2 | $Et_3B$ (1.2)<br>$NaBH_4$ (1.3) | a) $Et_3B$ bei 25°C/ 20 min<br>b) $NaBH_4$ bei ~ 70°C/ 12 h<br>c) $NaH_2PO_4/H_2O$ (Überschuß) bei -10°C | Ether oder THF, bevorzugt THF | ja |
| Z2 | NaH (1.8)<br>$((CH_3)_2CHO)_2POCH_2CN$ (1.5) | a) Phosphonat bei 0°C zum NaH; 40 min 0°C<br>b) Aldehyd bei 0°C; 1-3 h 25°C | Ether oder THF bevorzugt THF | ja |
| Z3 | $((CH_3)_2-CH-CH_2)_2AlH$ (3.0) | -20 - +50°C, bevorzugt 0°C | inertes Lsgm., bevorzugt THF | - |

| Verfahrensschritte | Reagenz (Äquiv.) | Temperatur | Lösungsmittel | Inert-Atmosphäre |
|---|---|---|---|---|
| C1 | $S(+)CH_3CO_2CH(Ph)C(OH)Ph_2$ (1.1)<br>LDA (2.5)<br>$MgI_2 \cdot Et_2O$ (1.1) | -135°C bis -60°C, bevorzugt -78°C<br>siehe Diss R. M. Devant, Univ. Karlsruhe (1985), S. 99 - 101 | Ether oder THF bevorzugt THF | ja |
| C2 | $CH_3ONa$ (1.05) | 0-65°C, bevorzugt 25°C | $CH_3OH$ | - |
| C3 | $CH_3-\overset{\overset{O}{\|}}{C}-O$-tert.Butyl (3.0)<br>n-Buli (2.9) | -10 - -50°C, bevorzugt -30°C/1 h | Ether oder THF bevorzugt THF | ja |
| D1 | $((CH_3)_2CHCH_2)_2AlH$ (1.0) | -40 - -78°C, bevorzugt -78°C | inertes Lsgm., bevorzugt THF | ja |
| D2 | komplexes Metallhydrid, bevorzugt $LiAlH_4$ (Überschuß)<br>oder<br>$((CH_3)_2CHCH_2)_2AlH$ (4.0) | -20°C bis Rückfl., bevorzugt 25°C<br><br>-40°C | Ether, THF, Benzol, Toluol bevorzugt Ether<br><br>inertes Lsgm., bevorzugt Toluol | ja<br><br>ja |

| Verfahrensschritte | Reagenz (Äquiv.) | Temperatur | Lösungsmittel | Inert-Atmosphäre |
|---|---|---|---|---|
| D3 | $MnO_2$ (Überschuß) | 0 - 35°C, bevorzugt 25°C | Ether | - |
| | oder | | | |
| | $CrO_3$ (10.0), Pyridin (20.0) | 0 - 25°C, bevorzugt 25°C | $CH_2Cl_2$ | - |
| D4 | NaH (Überschuß) | a) NaH 25°C → 110°C | Toluol | - |
| | $CH_3I$ (Überschuß) | b) $CH_3I$ 25°C → Rückfluß | | |
| D5 | $LiAlH_4$ (3.0) | Rückfluß | THF | ja |
| D6 | $((CH_3)_2CHCH_2)_2AlH$ (1.3) | -50°C - 0°C, bevorzugt -30°C | THF oder Toluol | ja |
| D7 | $HCON(CH_3)_2$ (3.0), $POCl_3$ (3.2) | -40°C - +50°C bevorzugt 0°C | inertes Lsgm., z. B. $CH_3CN$ | ja |

Heterocyclische Alkylester der allgemeinen Formel XVIII, Anilide der allgemeinen Formel XIX, Nitrile der allgemeinen Formel XXI und Heterocyclen der allgemeinen Formel XXII sind nach einer Reihe allgemeiner Heterocyclensynthesen zugänglich. Diese sind Stand der Technik und können Monographien über die jeweiligen Heterocyclen entnommen werden.

Im Folgenden ist eine Auswahl von Synthesemethoden angegeben, die besonders zur Herstellung

solcher Verbindungen der allgemeinen Formeln XVIII, XIX, XXI oder XXII geeignet sind, die bezüglich ihrer Substituenten $R^2$, $R^3$, $R^4$, $R^5$ bevorzugt oder besonders bevorzugt sind.

Schema 4: Synthese von Zwischenprodukten des 1H-Pyrrol-3-yl-Typs

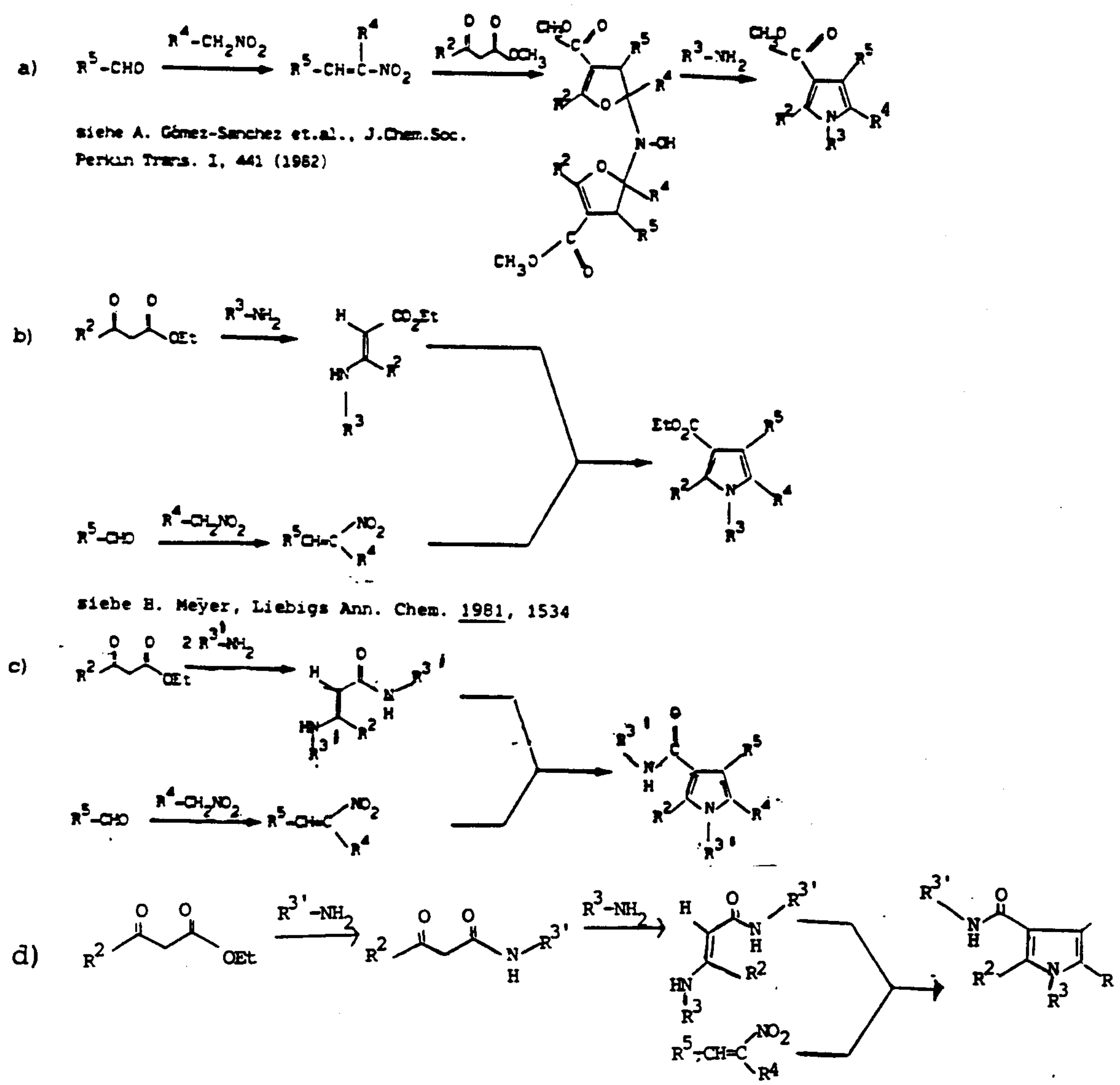


wobei $R^{3'}$ Phenyl oder durch die zu Formel I unter a) - d) genannten Gruppen substituiertes Phenyl bedeutet,

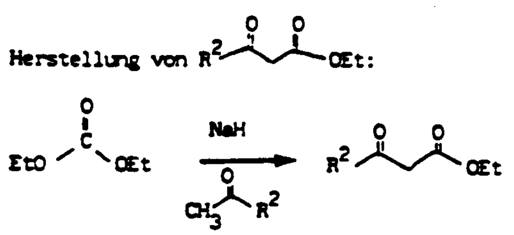


siehe M. Jackman et.al. J. Am. Chem. Soc 70, 2885 (1948)

Bei Methode b) setzt sich der $\beta$-Ketoester mit 1 Äquivalent des Amins zu Enaminoester um, wenn $R^2$ nicht sterisch anspruchsvoll ist (z. B. $R^2$ = $CH_3$). Ist $R^2$ hingegen ein sperriger Substituent (z. B. $R^2$ = Isopropyl,

Methode c), so erfolgt im Falle des Anilins der Angriff der Esterfunktion rascher als der der Ketofunktion. Ist $R^3$ ein substituierter oder unsubstituierter Phenylring, so erhält man durch Einsatz von zwei Äquivalenten des Anilins $R^{3'}$-$NH_2$ direkt das Enaminosäureanilid ($R^3 = R^{3'}$) (Methode C), das mit der Nitroverbindung zum 3-Pyrrolcarbonsäureanilid kondensiert. Ist $R^3$ hingegen ein Alkylrest und somit notwendigerweise unterschiedlich von $R^{3'}$ (Methode d), so muß man den $\beta$-Ketoester zunächst mit einem Äquivalent des Anilins $R^{3'}$-$NH_2$ zum $\beta$-Ketoanilid umsetzen, das dann mit einem Äquivalent des Alkylamins $R^3$-$NH_2$ zum Enaminosäureanilid ($R^3 \neq R^{3'}$) kondensiert.

Die Inhibierung der HMG-CoA-Reduktase-Aktivität durch die Verbindungen der allgemeinen Formeln I und II wurde an solubilisierten Enzympräparationen aus Ratten-Lebermikrosomen bestimmt.

Nach Umstellung der Ratten im Tag-Nacht-Rhythmus wurde die Enzymbildung mit Cholestyramin ((R)Cuemid) induziert. Als Substrat diente (S,R) $^{14}$C-HMG-CoA, die Konzentration von NADPH wurde während der Inkubation durch ein regenerierendes System aufrechterhalten. Die Abtrennung von $^{14}$C-Mevalonat von Substrat und anderen Produkten (z. B. $^{14}$C-HMG) erfolgte über Säulenelution, wobei das Elutionsprofil jeder Einzelprobe ermittelt wurde. Auf die ständige Mitführung von $^3$H-Mevalonat wurde verzichtet, weil es sich bei der Bestimmung um die Relativangabe der Hemmwirkung handelt. In einer Versuchsreihe wurden jeweils die enzymfreie Kontrolle, der enzymhaltige Normalansatz (= 100 %) und solche mit Präparatezusätzen zusammen behandelt. Jeder Einzelwert wurde als Mittelwert aus 3 Parallelproben gebildet. Die Signifikanz der Mittelwertsunterschiede zwischen präparatefreien und präparatehaltigen Proben wurde nach dem t-Test beurteilt.

Nach der oben beschriebenen Methode wurden von den erfindungsgemäßen Verbindungen z. B. folgende Hemmwerte auf die HMG-CoA-Reduktase ermittelt ($IC_{50}$-Wert M = molare Konzentration der Verbindung, die für eine 50 %ige Hemmung erforderlich ist. Angegeben sind jeweils die $IC_{50}$-Werte für die optisch reinen Verbindungen I in der bevorzugten Absolutkonfiguration)

Tabelle 2

| Beispiel | $IC_{50}$ (M) | Beispiel | $IC_{50}$ (M) |
|---|---|---|---|
| 2 | $8{,}0 \times 10^{-8}$ | 9 | $3{,}0 \times 10^{-9}$ |
| 3 | $8{,}7 \times 10^{-9}$ | 12 | $1{,}0 \times 10^{-7}$ |
| 5 | $8{,}0 \times 10^{-9}$ | 13 | $5{,}0 \times 10^{-8}$ |
| 6 | $2{,}5 \times 10^{-9}$ | 16 | $1{,}5 \times 10^{-9}$ |
| 8 | $1{,}5 \times 10^{-8}$ | 19 | $3{,}0 \times 10^{-9}$ |

Mit ausgewählten Verbindungen wurde außerdem die Cholesterin-Biosynthese-Hemmung in Zellkulturen durch $^{14}$C-Präkursoreinbau in Cholesterin getestet.

Monolayers von HEP G2-Zellen in lipoproteinfreiem Nährmedium wurden mit verschiedenen Konzentrationen der Prüfsubstanzen 1 Stunde vorinkubiert. Nach Zugabe des $^{14}$C-markierten Präkursors $^{14}$C-Natriumacetat wurde die Inkubation 3 Stunden fortgesetzt. Anschließend wurde $^3$H-Cholesterin als interner Standard zugesetzt und ein Teil der Zellen alkalisch verseift. Die Lipide der verseiften Zellen wurden mit Chloroform-Methanol extrahiert. Dieses Lipidgemisch wurde nach Zusatz von Träger-Cholesterin präparativ dünnschichtchromatographisch getrennt, die Cholesterin-Bande nach dem Sichtbarmachen mit Jod-Dämpfen isoliert und die aus dem $^{14}$C-Präkursor gebildete Menge $^{14}$C-Cholesterin szintigraphisch bestimmt. In einem aliquoten Teil der Zellen wurde Zellprotein bestimmt, so daß die in der Zeiteinheit /mg Zellprotein aus $^{14}$C-Präkursor gebildete Menge $^{14}$C-Cholesterin berechnet werden kann. Zum Vergleich für die Hemmwirkung eines zugesetzten Prüfpräparates dient die Lösungsmittelkontrolle, so daß direkt die Hemmung der Cholesterin-Biosynthese bei einer bestimmten molaren Konzentration des Prüfpräparates im Medium angegeben werden kann. In einem aliquoten Anteil der Zellkulturen wurde die Intaktheit der Zellkultur und die fehlende Zellschädigung durch Präparate-Einwirkung morphologisch (Lichtmikroskop) sichergestellt. Angegeben in Tabelle 3 ist die relative Wirkstärke der Testverbindungen bezogen auf Mevinolin-Natriumsalz. Die relative Wirkstärke wurde ermittelt durch Vergleich der $IC_{70}$-Werte bzw. $IC_{50}$-Werte für die Testverbindung und für Mevinolin-Natriumsalz (interner Standard). Die Absolutwerte für $IC_{70}$ und $IC_{50}$ (Mevinolin-Natrium) variieren etwas von Zellkultur zu Zellkultur. Sie liegen im Mittel bei $IC_{70} = 1{,}5 \times 10^{-7}$ M, $IC_{50} = 5 \times 10^{-8}$ M.

Tabelle 3

| rel. Wirkstärke (%) bezogen auf Mevinolin-Natrium | | |
|---|---|---|
| Beispiel | bei $IC_{70}$ | bei $IC_{50}$ |
| 3<br>9 | 51<br>150 | 450 |

Die Verbindungen der allg. Formel I bzw. II zeichnen sich aus durch starke Hemmung der HMG-CoA-Reduktase, des geschwindigkeitsbestimmenden Enzyms der Cholesterinbiosynthese. Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterinbiosynthese (vgl. J. R. Sabine in CRC Series in Enzyme Biology: 3-Hydroxy-3-methylglurary1 Coenzym A Reduktase, CRC Press Inc. Boca Raten , Florida 1983 (ISBN 0-8493-6551-1)).

Hohe Cholesterinspiegel werden mit einer Reihe von Erkrankungen, wie z.B. koronarer Herzkrankheit oder Arteriosklerose in Verbindung gebracht. Daher ist die Senkung erhöhter Cholesterinspiegel zur Vorbeugung und Behandlung solcher Erkrankungen ein therapeutisches Ziel.

Ein Ansatzpunkt dazu liegt in der Hemmung bzw. Verminderung der endogenen Cholesterinbiosynthese. Hemmstoffe der HMG-CoA-Reduktase blockieren die Cholesterinbiosynthese auf einer frühen Stufe.

Die Verbindungen der allg. Formel I bzw. II eignen sich daher als Hypolipidemika und zur Behandlung bzw. Prophylaxe arteriosklerotischer Veränderungen.

Die Erfindung betrifft daher auch pharmazeutische Präparate auf Basis dieser Verbindungen sowie ihre Verwendung als Arzneimittel, insbesondere als Hypolipidemika und zur Prophylaxe arteriosklerotischer Veränderungen.

Die Anwendung der Verbindungen der Formel I bzw. II als Hypolipidemika oder Anti-Arteriosklerotika erfolgt in oralen Dosen von 3 bis 2 500 mg, vorzugsweise jedoch im Dosisbereich von 10 - 500 mg. Diese Tagesdosen können nach Bedarf auch in zwei bis vier Einzeldosen aufgeteilt werden oder in Retardform verarbeitet werden. Das Dosierungsschema kann vom Typ, Alter, Gewicht, Geschlecht und medizinischen Zustand des Patienten abhängen.

Ein zusätzlicher cholesterinsenkender Effekt läßt sich durch gleichzeitige Gabe der erfindungsgemäßen Verbindungen mit gallensäurebindenden Stoffen, wie z.B. Anionenaustauscherharzen, erzielen. Die erhöhte Gallensäureausscheidung führt zu einer verstärkten Neusynthese und damit zu einem erhöhten Cholesterinabbau (vgl. M. S. Brown, P. T. Koranen u. J. C. Goldstein, Science 212, 628 (1981); M. S. Brown, J. C. Goldstein, Spektrum der Wissenschaft 1, 96 (1985)).

Die erfindungsgemäßen Verbindungen der Formel I bzw. II können in Form der δ-Lactone, als freie Säuren oder in Form ihrer physiologisch unbedenklichen anorganischen oder organischen Salze oder als Ester zu Anwendung kommen. Säuren und Salze bzw. Ester können in Form ihrer wässrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetaldehyddiacetalgemischen, Ölen wie z. B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon oder in festen Zubereitungen zur Anwendung gelangen.

Für die Verbindungen der Formel I bzw. II sind feste, oral verabreichbare Zubereitungsformen bevorzugt, die die üblichen Hilfsstoffe enthalten können. Sie werden nach üblichen Methoden hergestellt.

Als Zubereitungen für die orale Anwendung eignen sich insbesondere Tabletten, Dragees oder Kapseln. Eine Dosierungseinheit enthält vorzugsweise 10 bis 500 mg Wirkstoff.

**Beispiel 1:**

Synthese von 3(RS), 5(SR)-Dihydroxy-7-[1-phenyl-2,5- dimethyl-4-(4-fluorphenyl)-1H-pyrrol-3-yl]-hept-6(E)-ensäure-methylester

[Formel Ia mit trans-HC=CH, $R^1=CH_3$, $R^2=CH_3$, $R^3=$ Phenyl, $R^4=CH_3$, $R^5=$p-Fluorphenyl]

**Beispiel 1.1.**

3-Phenylamino-but-2(E)-ensäureethylester (N-Phenylamino-crotonsäureethylester) (Schema 4 b)

Die Lösung aus 46.5 g (45.5 ml, 0.5 mol) Anilin, 65 g (63,5 ml, o.5 mol) Acetessigsäureethylester und 1 ml Eisessig in 100 ml Toluol wurde 4 Std. am Wasserabscheider unter Rückfluß gekocht. Das Lösungsmittel wurde im Vakuum abdestilliert und der Rückstand im Hochvak. destilliert.
57.9 g farbloses Öl. Sdp. 118-120 ° C/1.5 Torr.
MS: $C_{12}H_{15}NO_2$ (205), m/e = 205 ($M^+$)

**Beipiel 1.2.**

1-(p-Fluorphenyl)-2-nitro-propen (Schema 4 a und 4 b)

Die Lösung aus 84 g p-Fluorbenzaldehyd, 69.4 g Nitroäthan und 4 ml n-Butylamin in 110 ml Xylol wurde 20 Std. am Wasserabscheider unter Rückfluß gekocht. Beim Abkühlen auf 0 ° C kristallisierten 21.7 g des Produkts, Schmp. 64-5 ° C. Dieser Feststoff wurde abgesaugt und mit kaltem Xylol gewaschen.

Das Filtrat wurde mit 41.4 g Nitroäthan und 3 ml n-Butylamin versetzt und nochmals 14 Std. am Wasserabscheider unter Rückfluß gekocht. Das Lösungsmittel wurde abgezogen, der Rückstand wurde bei 0 ° C mit Methanol verrieben, wobei Kristallisation eintrat. Der Feststoff wurde abgesaugt und mit kaltem Methanol gewaschen. 53.8 g, Schmp. 65-66 ° C.

**Beispiel 1.3.**

1-Phenyl-2,5-dimethyl-4-(4-fluorphenyl)-1H-pyrrol-3-carbonsäureethylester

[Formel XVIII mit $R^2 = CH_3$, $R^3$ = Phenyl, $R^4 = CH_3$, $R^5$ = p-Fluorphenyl, $R^{11}$ = Ethyl]

Die Lösung aus 23.1 g (113 mmol) N-Phenylaminocrotonsäureethylester (Bsp. 1.1.) und 205 g (113 mmol) des Nitroolefins aus Bsp. 1.2. in 250 ml Ethanol wurde 30 Std. unter Rückfluß gekocht. Das Lösungsmittel wurde im Vak. abdestilliert und der Rückstand mit Cyclohexan/Essigester 95:5 über 1 kg Kieselgel chromatographiert. 26 g farbloses Öl.
$^1$H-NMR($CDCl_3$): $\delta$ = 1.05(t, 3H, Ester-$CH_3$), 1.85(s, 3H, $CH_3$), 2.3(s, 3H, $CH_3$), 4.1(qua, 2H, $CH_2$), 6.9-7.6(m, 9H, arom.-H)
MS: $C_{21}H_{20}FNO_2$ (337) m/e = 337 ($M^+$), 308, 292, 77

**Beispiel 1.4. Verfahrensschritt D2 (Schema 3)**

1-Phenyl-2,5-dimethyl-4-(4-fluorphenyl)-3- hydroxymethyl-1H-pyrrol

[Formel IV mit $R^2 = CH_3$, $R^3$ = Phenyl, $R^4 = CH_3$, $R^5$ = p-Fluorphenyl]

Zur Suspension von 7.8 g (0.2 Mol) Lithiumaluminiumhydrid in 300 ml Ether tropft man bei 5-10 ° C die Lösung von 27.8 g des Esters aus Beispiel 1.3. in 300 ml Ether. Man rührt 4 Std. bei Raumtemperatur und tropft dann bei 0 ° C nacheinander vorsichtig 35 ml Essigester, 16 ml Wasser und 24 ml 2N Natronlauge zu. Man rührt 30 min bei Raumtemperatur, saugt den Feststoff ab und engt das Filtrat im Vakuum ein. Der Rückstand wird mit Cyclohexan/Essigester 2:1 + 0.2 % Triethylamin über 1 kg Kieselgel chromatographiert.
20.7 g farbloses Öl, das sehr langsam kristallisiert.
1H-NMR($CDCl_3$): $\delta$ = 1.3(s, breit, 1H, OH), 2.0(s, 3H, $CH_3$), 2.1(s, 3H, $CH_3$), 4.55(s, 2H, $CH_2$), 6.9-7.65(m, 9H, arom.-H)
MS: $C_{19}H_{18}FNO$ (295) m/e = 295 ($M^+$), 278 ($M^+$-OH), 77

**Beispiel 1.5. Verfahrensschritt D3 (Schema 3)**

1-Phenyl-2,5-dimethyl-4-(4-fluorphenyl)-1H-pyrrol-3-carboxaldehyd

[Formel III mit $R^2 = CH_3$, $R^3$ = Phenyl, $R^4 = CH_3$, $R^5$ = p-Fluorphenyl]

Zur Lösung von 20.7 g des Alkohols aus Beispiel 1.4. in 1.2 l abs. Ether und 12 ml Triethylamin gibt man im Abstand von 24 Std. 2 mal 182.5 g Mangandioxid. Man rührt 2 Tage unter $N_2$ bei Raumtemperatur,

saugt den Feststoff ab und wäscht mit Ether.

Das Filtrat wird im Vak. eingeengt und der Rückstand mit Cyclohexan/Essigester 6:1 +0.1 % Triethylamin über Kieselgel chromatographiert. 13.2 g gelblicher Feststoff.
$^1$H-NMR ($CDCl_3$): $\delta$ = 1.94 (s, 3H, $CH_3$), 2.35 (s, 3H, $CH_3$), 6.95 - 7.7 (m, 9H, arom.-H), 9.85 (s, 1H, CHO)
MS: $C_{19}H_{16}FNO$ (293), m/e = 293 ($M^+$), 77

**Beispiel 1.6. Verfahrensschritt Z1**

3-[1-Phenyl-2,5-dimethyl-4-(4-fluorphenyl)-1H-pyrrol-3-yl]-(E)-propenal

[Formel XI mit $R^2 = CH_3$, $R^3$ = Phenyl, $R^4 = CH_3$, $R^5$ = p-Fluorphenyl]

Zur Lösung von 3.46 g (9.6 mmol) 1-Ethoxy-2-(tri-n-butylstannyl)-ethylen(A. J. Leusink et. al. J. Organomet. Chem. 9, 289 (1967) in 110 ml abs. THF gibt man bei -70 °C unter Stickstoff 10 mmol n-Butyllithium (6.25 ml einer 1.6 M Lsg. in n-Hexan). Nach 2 Std. bei -73 °C tropft man innerhalb 5 min die Lösung von 2.34 g (8 mmol) des Aldehyds aus Bsp. 1.5. in 12 ml THF zu. Die Innentemp. steigt dabei bis auf -66 °C. Man rührt 2 Std. bei -73 °C, 10 min bei -50 °C und tropft dann bei -40 °C 18.6 ml einer gesättigten wäßrigen Ammoniumchlorid-Lösung zu. Man läßt auf Raumtemp. kommen, trennt die organ. Phase ab, extrahiert die wäßrige Phase 2 mal mit Ether, wäscht die vereinigten Organ. Phasen mit gesättigter Kochsalzlösung und engt ein. Der Rückstand wird in 93 ml THF, 18 ml Wasser und p-Toluolsulfonsäure aufgenommen und 1 Std. bei Raumtemp. gerührt. Die organische Phase wird abgetrennt, die wäßrige Phase mit Ether extrahiert. Die vereinigten organ. Phasen werden mit gesätt. Kochsalzlösung gewaschen, getrocknet und eingeengt. Der Rückstand wird mit Cyclohexan/Essigester/Triethylamin 3:1:0.1 über 450 g Kieselgel chromatographiert.
2.25 g amorpher Feststoff
$^1$H-NMR($CHCl_3$): $\delta$ = 1.9(s, 3H, $CH_3$), 2.2(s, 3H, $CH_3$), 6.07 (dd, 1H, =C-H), 6.9-7.7(m, 10 H, =C-H und 9 arom.-H), 9.45(d, 1H, CHO)
MS: $C_{21}H_{18}FNO$ (319) m/e = 319 ($M^+$), 290 ($M^+$-CHO), 77

**Beipiel 1.7. Verfahrensschritt B1**

5(RS)-Hydroxy-3-oxo-7-[1-phenyl-2,5-dimethyl-4-(4-fluorphenyl)-1H-pyrrol-3-yl]-hept-6(E)-ensäure-methylester

[Formel XII mit $R^1 = CH_3$, $R^2 = CH_3$, $R^3$ = Phenyl, $R^4 = CH_3$, $R^5$ = p-Fluorphenyl, X,Z = C, Y = N]

Zur Suspension von 12.7 mmol Natriumhydrid (609 mg einer 50 %igen Suspension von NaH in Mineralöl) in 86 ml THF wird bei -15 °C innerhalb 5 min. die Lösung von 1,43 g (12.33 mmol) Acetessigsäuremethylester in 10 ml THF getropft. Die Lösung wird 50 min. bei -15 °C gerührt. Dann wird innerhalb 10 min. 7.68 ml (12.26 mmol) einer 1,6 M Lösung von n-Butyllithium in Hexan zugetropft und 20 min. bei -15 °C nachgerührt. Bei dieser Temperatur wird innerhalb 10 min. die Lösung von 2,25 g (7.05 mmol) des Aldehydes aus Bsp. 1.6. in 25 ml THF zugetropft und die Reaktionslösung 45 min. bei -15 °C nachgerührt. Bei -10 °C werden 13 ml einer gesättigten Natriumdihydrogenphosphatlösung zugetropft. Es wird 5 min bei 0 °C gerührt und das Reaktionsgemisch zwischen Ether und gesättigter Kochsalzlösung verteilt. Die organische Phase wird abgetrennt und die wäßrige Phase 1 mal mit Ether extrahiert. Die vereinigten organ. Phasen werden mit gesätt. Kochsalzlösung gewaschen, getrocknet, filtriert und eingeengt. Kieselgel-Chromatographie mit Cyclohexan/Essigester/Triethylamin (2:1:0.1) gibt 2.33 g gelbes Öl.
$^1$H-NMR($CDCl_3$, 270 MHz): $\delta$ = 1.6(s, 1H, OH), 1.9(s, 3H, $CH_3$), 2.13(s, 3H, $CH_3$), 2.36(s, 2H, $CH_2$), 3.57 (sehr enges AB-System 2 H, $CH_2$) 3.73(s, 3H, $OCH_3$), 5.99(d, 1H, CH), 6.16(dd, J = 15.2 und 11.2 Hz, 1H, =C-H), 6.94(d, J = 15.2Hz, 1 H, =C-H), 7.08-7.33(m, 5H, arom.-H), 7.44-7.58 (m, 4H, arom.-H)
MS: $C_{26}H_{26}FNO_4$ (435) m/e = 435($M^+$), 417($M^+$-$H_2O$), 320, 319, 316, 290

**Beispiel 1.8. Verfahrensschritt B2**

3(RS), 5(SR)-Dihydroxy-7-[1-phenyl-2,5-dimethyl-4-(4-fluorphenyl)-1H-pyrrol-3-yl] -hept-6(E)-ensäure-methylester

[Formel Ia mit trans-HC = CH, $R^1 = CH_3$, $R^2 = CH_3$, $R^3$ = Phenyl, $R^4 = CH_3$, $R^5$ = p-Fluorphenyl]

Zur Lösung von 2,3 g (5.28 mmol) des Ketoesters aus Beispiel 1.7. in 70 ml absolutem THF wird bei 20 ° C innerhalb 5 min. 6.33 ml (6.33 mmol) einer 1 M Lösung von Triethylboran in THF getropft. Es wird 20 min. bei Raumtemperatur gerührt, dann innerhalb 5 min. 14.8 ml trockene Luft mittels einer Spritze in die Reaktionslösung eingeleitet. Es wird weitere 2 Std. bei Raumtemp. gerührt. Das Reaktionsgemisch wird auf -75 ° C gekühlt und 261 mg (6.864 mmol) Natriumborhydrid auf einmal zugegeben. Es wird 12 Std. bei -75 ° C gerührt. Bei -10 ° C werden 37 ml gesättigte Natriumdihydrogenphosphatlösung zugetropft. Das Reaktionsgemisch wird zwischen Ether und gesättigter Kochsalzlösung verteilt. Die organische Phase wird mit Kochsalzlösung gewaschen, getrocknet, filtriert, eingeengt. Der Rückstand wird mit 370 ml absol. Methanol versetzt und 3 Std. bei Raumtemperatur gerührt, dann eingeengt und der Rückstand mit Cyclohexan/Essigester/Triethylamin 1:1:0.1 über Kieselgel chromatographiert. 1.9 g hellgelbes Öl.
$^1$H-NMR($C_6D_6$, 270 MHz): $\delta$ = 1.37(dt, 1H, $CH_2$), 1.67(dt, 1H, $CH_2$), 1.90(s, 3H, $CH_3$), 2.08(s, 3H, $CH_3$), 2.05-ca. 2.12 (dd, verdeckt, 1H, $CH_2$), 2.26(dd, 1H, $CH_2$), 2.40(d, 1H, OH), 3.26(s, 3H, $OCH_3$), 3.48(d, 1H, OH), 4.11(m, 1H, CH), 4.30(m, 1H, CH), 5.72(dd, J = 16.0 und 6.0 Hz, 1H, = C-H), 6.72(d, J = 16.0 Hz, 1H, = C-H), 6.85-6.91 (m, 2H, arom.-H), 6.95-7.17(m, 5H, arom.-H), 7.32-7.40 (m, 2H, arom.-H)
MS: $C_{26}H_{28}FNO_4$ (437) m/e = 437($M^+$), 419($M^+$-$H_2O$), 320, 302, 278
$^1$H-NMR($D_2O$, $\delta$ HOD = 4.8, 270 MHz):$\delta$ = 1.6(s, 3H, $CH_3$), 2.0 (s, 3H, $CH_3$), 2.1(m, 2H, $CH_2$), 2.3(m, 2H, $CH_2$), 3.97(m, 1H, CH), 4.68(d, 1H, CH), 5.62(m, 2H, = CH), 6.8-7.4(m, 9H, arom.-H)

**Beispiel 2: Verfahrensschritt A9 (Schema 1)**

3(RS), 5(RS)-Dihydroxy-7- [1-phenyl-2,5-dimethyl-4-(4-fluorphenyl)-1H-pyrrol-3-yl] -heptansäure-methylester

[Formel I mit AB = $CH_2$-$CH_2$, $R^1$ = $CH_3$, $R^2$ = $CH_3$, $R^3$ = Phenyl, $R^4$ = $CH_3$, $R^5$ = p-Fluorphenyl]

Zur Lösung von 75 mg des Esters aus Beispiel 1.8. in 20 ml Methanol und 0,1 ml Triethylamin gibt man ca. 50 mg 10 % Palladium auf Kohle und schüttelt 10 min. bei Normaldruck und Raumtemperatur, in einer Wasserstoffatmosphäre. Der Katalysator wird abfiltriert und mit Methanol gewaschen. Das Filtrat wird im Vakuum eingeengt und der Rückstand mit Toluol/Essigester/Triethylamin 2:1:0.01 über Kieselgel chromatographiert.
47 mg amorpher Feststoff
$^1$H-NMR($C_6D_6$, 270 MHz): $\delta$ = 1.10(dt, 1H, $CH_2$), 1.38(dt, 1H, $CH_2$), 1.50-1.76(m, 2H, $CH_2$), 1.97(s, 3H, $CH_3$), 2.01 (dd, z. T. verdeckt, 1H, $CH_2$), 2.08(s, 3H, $CH_3$), 2.17 (dd, 1H, $CH_2$), 2.77(m, 2H, $CH_2$), 2.86(d, 1H, OH), 3.27(s,3H, $OCH_3$), 3.50(d, 1H, OH), 3.72(m, 1H, CH), 3.95 (m, 1H, CH), 6.90-7.13(m, 7H, arom.-H), 7.28-7.36(m, 2H, arom.-H)
MS: $C_{26}H_{30}FNO_4$ (439): m/e = 439($M^+$), 407 ($M^+$-$CH_3OH$), 279, 278, 264, 118

**Beispiel 3:** Verfahrensschritt A8 (Schema 1)

3(RS), 5(RS)-Dihydroxy-7-[1-phenyl-2,5-dimethyl-4-(4-fluorphenyl)-1H-pyrrol-3-yl]-heptansäure-Natriumsalz

[Formel I mit AB = $CH_2$-$CH_2$, $R^1$ = Na, $R^2$ = $CH_3$, $R^3$ = Phenyl, $R^4$ = $CH_3$, $R^5$ = p-Fluorphenyl]

Zur Lösung von 41 mg (0.093 mmol) des Esters aus Bsp. 3 in 10 ml Methanol gibt man bei 0 ° C 0.93 ml (0.093 mmol) 0.1 N wäßrige Natronlauge. Nach 90 min engt man zur Trockne ein und trocknet das Pulver weiter im Hochvakuum. 42 mg amorpher Feststoff.

**Beispiel 4:**

Synthese von 3(RS),5(SR)-Dihydroxy-7-[1-phenyl-2-isopropyl-4-(4-fluorphenyl)-5-methyl-1H-pyrrol-3-yl-] hept-6(E)-ensäure-methylester

[Formel Ia mit trans -CH = CH, $R^1$ = $CH_3$, $R^2$ = Isopropyl, $R^3$ = Phenyl, $R^4$ = $CH_3$, $R^5$ = p-Fluorphenyl]

**Beispiel 4.1.**

3-Phenylamino-4-methyl-pent-2(E)-ensäure-anilid (Schema 4 c)

Die Lösung von 37 ml (0,41 Mol) Anilin, 31 ml (0,2 Mol) 3-Oxo-4-methylpentansäureethylester und 1,0

ml Eisessig in 50 ml Toluol wird 6 Stunden am Wasserabscheider unter Rückfluß gekocht. Das Lösungsmittel wird im Vakuum abgezogen.

Beim Erkalten kristallisiert der Rückstand. Beim Umkristallisieren aus ca. 300 ml heißem Toluol/Petrolether (80-110 °C) 2:1 (heiß dekantieren) erhält man 38,7 g farbloses Pulver, Schmp. 147-148 °C. Weiteres Produkt kann aus der Mutterlauge gewonnen werden.
$^1$H-NMR ($CDCl_3$): $\delta$ = 1,1 (d + m, 7H, $C(CH_3)_2$ + Amin-H), 2,9 (sept., 1H, CH), 4,75 (s, 1H, =CH), 6,8 - 7,6 (m, 10H, arom.-H), 11,1 (s, breit, 1H, Amid-H)
MS: $C_{18}H_{20}N_2O$ (280) m/e = 280 ($M^+$), 237 ($M^+$ - $CH(CH_3)_2$), 188 ($M^+$ - PhNH)

**Beispiel 4.2.**

1-Phenyl-2-isopropyl-4-(4-fluorphenyl)-5-methyl-1H-pyrrol-3-carbonsäureanilid

[Formel XIX mit $R^2$ = Isopropyl, $R^3$ = Phenyl, $R^4$ = $CH_3$, $R^5$ = p-Fluorphenyl, $R^{12}$ = H,]

Die Lösung von 12,4 g (60 mmol) 1-(p-Fluorphenyl)-2-nitro-propen (Beispiel 1.2) und 17.1 g (61 mmol) des Anilids aus Bsp. 4.1 in 130 ml Ethanol wird unter Stickstoff 12 Std. unter Rückfluß gekocht. Das Ethanol wird im Vakuum abgezogen und der gelbe, feste Rückstand mit Cyclohexan/Essigester 4:1 über Kieselgel chromatographiert. 19.7 g gelber Feststoff, Schmp. 186 - 188 °. Umkristallisieren aus Cyclohexan/Essigester ergibt einen farblosen Feststoff mit Schmp. 190-192 °C.
$^1$H-NMR ($CDCl_3$): $\delta$ = 1,3 (d, 6H, $C(CH_3)_2$), 1,83 (s, 3H, $CH_3$), 3,2 (sept., 1H, CH), 6,8 - 7,6 (m, 15H, Amid-H + 14 arom.-H)
MS: $C_{27}H_{25}N_2OF$ (412) m/e = 412 ($M^+$), 320 ($M^+$-PhNH)

**Beispiel 4.3.** Verfahrensschritt D4 (Schema 3)

1- Phenyl -2 -isopropyl-4-(4-fluorphenyl)-5-methyl -1H-pyrrol-3-carbonsäure-N-methylanilid

[Formel XX mit $R^2$ = Isopropyl, $R^3$ = Phenyl, $R^4$ = $CH_3$, $R^5$ = p-Fluorphenyl, $R^{12}$ = H]

Zur Lösung von 4,5 g des Anilids aus Beispiel 4.2 in 50 ml Toluol gibt man unter mechan. Rühren unter Stickstoff portionsweise 1,2 g 50 % Natriumhydrid/Mineralöl.

Man erwärmt 30 min auf 60 °C und 10 min. auf 110 °C. Man kühlt auf 30 °C ab und gibt auf einmal 5.8 ml Methyl-jodid zu. Dann kocht man 16 Std. unter Rückfluß bei 75 °C Badtemp. Man kühlt mit Methanol/Trockeneis und tropft langsam 16 ml Wasser zu. Man läßt bis auf 5 °C erwärmen, gibt ca. 200 ml Ether zu, trennt die organ. Phase ab, wäscht mit gesättigter Kochsalzlösung, trocknet und engt im Vakuum ein. Der Rückstand kristallisiert beim Verreiben mit n-Hexan. 3,9 g schwachgelber Feststoff, Schmp. 60 - 63 °C.
$^1$H-NMR ($CDCl_3$): $\delta$ = 1,2 (d, 3H, $CH_3$), 1,3 (d, 3H, $CH_3$), 1,8 (s, 3H, $CH_3$), 2,8 (sept., 1H, CH), 3,17 (s, 3H, N-$CH_3$), 6,5 - 7,5 (m, 14H, arom.-H)
MS: $C_{28}H_{27}N_2OF$ (426) m/e = 426 ($M^+$), 320 ($M^+$ -$PhNCH_3$)

**Beispiel 4.4.** Verfahrensschritt D5 (Schema 3)

1-Phenyl-2-isopropyl-3-hydroxymethyl-4-(4-fluorphenyl)-5-methyl-1H-pyrrol

[Formel IV mit $R^2$ = Isopropyl, $R^3$ = Phenyl, $R^4$ = $CH_3$, $R^5$ = p-Fluorphenyl]

Zur Suspension von 1.02 g (27 mmol) $LiAlH_4$ in 40 ml abs. THF gibt man die Lösung von 3.83 g (9mmol) des N-Methylanilids aus Bsp. 5.3. in 30 ml THF. Man kocht unter Stickstoff 18 Std. unter Rückfluß. Bei 0 °C tropft man nacheinander 5 ml Essigester, 2 ml Wasser und 3 ml 2N Natronlauge zu und rührt 30 min. bei Raumtemperatur. Der Feststoff wird abgesaugt und das Filtrat im Vak. eingeengt. Der Rückstand wird mit Pentan verrieben, worauf das Produkt kristallisiert. Die pentanische Mutterlauge enthält das Zweitprodukt N-Methylanilin.

Alternativ kann man das Rohprodukt mit Toluol/Essigester 20:1 + 1 % Triethylamin über Kieselgel filtrieren.1.82 g farbloser Feststoff
$^1$H-NMR($CDCl_3$): $\delta$ = 1.25(d, 6H, $C(CH_3)_2$),1.9(s, 3H, $CH_3$), 2.8(m, 1H, CH), 4.35(s, 1H, OH), 4.55 (s, 2H, $CH_2$), 6.85-7.75(m, 9H, arom.-H)

MS: $C_{21}H_{22}NOF$ (323) m/e = 323 ($M^+$), 308 ($M^+$-$CH_3$), 290 ($M^+$-$CH_3$-$H_2O$)

**Beispiel 4.5.** Verfahrensschritt D3 (Schema 3)

1-Phenyl-2-isopropyl-4-(4-fluorphenyl)-5-methyl-1H-pyrrol-3-carbaldehyd

[Formel III mit $R^2$ = Isopropyl, $R^3$ = Phenyl, $R^4$ = $CH_3$, $R^5$ = p-Fluorphenyl]

Zur Suspension von 10 g Celite und 5 g (50 mmol) fein gepulvertem Chromtrioxid in 100 ml absol. Methylenchlorid tropft man bei 15-20 °C die Lösung von 7.9 g (100 mmol) abs. Pyridin in 50 ml Methylenchlorid. Man rührt 20 min. bei Raumtemperatur und tropft rasch die Lösung von 1.61 g (5 mmol) des Alkohols aus Beispiel 5.4 in 50 ml Methylenchlorid zu. Man rührt 45 Min. bei Raumtemperatur und saugt rasch durch einen Celitefilter ab. Der Feststoff wird gut mit Methylenchlorid gewaschen und die vereinigten Filtrate im Vakuum eingeengt. Der Rückstand wird in Cyclohexan/Essigester/Triethylamin 1:1:0.01 aufgenommen und erneut durch einen Celitefilter abgesaugt. Der Feststoff wird gut gewaschen und die vereinigten Filtrate im Vakuum eingeengt. Der Rückstand wird mit Cyclohexan/Essigester/Triethylamin 3:1:0.01 über 800 g Kieselgel chromatographiert. 720 mg gelblicher Feststoff
$^1$H-NMR ($CDCl_3$): $\delta$ = 1.3 (d, 6H, $C(CH_3)_2$), 2.1 (s, 3H, $CH_3$), 3.1 (sept., 1H, CH), 6.9 - 7.6 (m, 9H, arom.-H), 10.0 (s, 1H, CHO)
MS: $C_{21}H_{20}NOF$ (321) m/e = 321 ($M^+$)

**Beispiel 4.6.** Verfahrensschritt Z1

3- [1-Phenyl-2-isopropyl-4-(4-fluorphenyl)-5-methyl-1H-pyrrol-3-yl -(E)-propenal]

[Formel XI mit $R^2$ = Isopropyl, $R^3$ = Phenyl, $R^4$ = $CH_3$, $R^5$ = p-Fluorphenyl]

In Analogie zu Beispiel 1.6 erhält man aus 700 mg des Aldehyds aus Bsp. 4.5 686 mg gelblichen Feststoff.
$^1$H-NMR($CDCl_3$): $\delta$ = 1.3 (d, 6H, $C(CH_3)_2$), 2.0 (s, 3H, $CH_3$), 3.1 (sept., 1H, CH), 6.1 (dd, J = 16 und 8Hz, 1H, =C-H), 7.0 - 7.8 (m, 10H, =C-H und 9 arom.-H), 9.5 (d, J = 8Hz, 1H, CHO)
MS: $C_{23}H_{22}NOF$ (347) DCI m/e = 348,2 (M + $H^+$)

**Beispiel 4.7.** Verfahrensschritt B1

5(RS)-Hydroxy-3-oxo-7-[1-phenyl-2-isopropyl-4-(4-fluorphenyl) -5 -methyl-1H-pyrrol-3-yl]-hept-6(E)-ensäure-methylester

[Formel XII mit $R^1$ = $CH_3$, $R^2$ = Isopropyl, $R^3$ = Phenyl, $R^4$ = $CH_3$, $R^5$ = p-Fluorphenyl]

In Analogie zu Beispiel 1.7 erhält man aus 660 mg des Aldehydes aus Bsp. 4.6 672 mg gelbes Öl.
MS: $C_{28}H_{30}FNO_4$ (463) m/e = 463 ($M^+$), 446 ($M^+$-OH)

**Beispiel 4.8.** Verfahrensschritt B2

3(RS), 5(SR)-Dihydroxy-7-[1-phenyl-2-isopropyl-4-(4-fluorphenyl)-5-methyl-1H-pyrrol-3-yl] -hept-6(E)-ensäure-methylester

[Formel Ia mit trans-HC = CH, $R^1$ = $CH_3$, $R^2$ = Isopropyl, $R^3$ = Phenyl, $R^4$ = $CH_3$, $R^5$ = p-Fluorphenyl]

In Analogie zu Beispiel 1.8. erhält man aus 640 mg des Ketoesters aus Bsp. 4.7 605 mg blaßgelbes zähes Öl.
$^1$H-NMR ($C_6D_6$): $\delta$ = 1.3 (d + m, 7H, $C(CH_3)_2$ und $CH_2$), 1.6 (m, 1H, $CH_2$), 1.95 (s, 3H, $CH_3$), 2.0 - 2.3 (m, 2H, $CH_2$), 2.5 (s, br., 1H, OH), 3.1 (sept., 1H, CH), 3.3 (s, 3H, $OCH_3$), 3.5 (s, breit, 1H, OH), 4.1 (m, 1H, CH), 4.3 (m, 1H, CH), 5.7 (dd, J = 16 und 6Hz, 1H, =C-H), 6.8 - 7.5 (m, 10H, =C-H und 9 arom.-H)
MS: $C_{28}H_{32}FNO_4$ (465) m/e = 465 ($M^+$), 447 ($M^+$-$H_2O$)

**Beispiel 5:** Verfahrensschritt A9 (Schema 1)

3 (RS), 5(RS)-Dihydroxy-7- [1-phenyl-2-isopropyl-4-(4-fluorphenyl)-5 - methyl - 1H -pyrrol-3-yl]-heptansäure-methylester

[Formel I mit AB = $CH_2$-$CH_2$, $R^1$ = $CH_3$, $R^2$ = Isopropyl, $R^3$ = Phenyl, $R^4$ = $CH_3$, $R^5$ = p-Fluorphenyl]

wird aus dem Produkt aus Beispiel 4.8 in Analogie zu Beispiel 2 erhalten.
$^1$H-NMR ($C_6D_6$): $\delta$ = 1.1 - 1.5 (m, 2H, $CH_2$), 1.3 (d, 6H, $C(CH_3)_2$), 1.6 - 2.2 (m + s, 7H, 2 x $CH_2$ und $CH_3$), 2.9 - 3.2 (m, 4H, $CH_2$, CH und OH), 3.3 (s, 3H, $OCH_3$), 3.45 (s, breit, 1H, OH), 3.8 - 4.1 (m, 2H, 2 x CH), 6.8 - 7.5 (m, 9H, arom.-H)
MS: $C_{28}H_{34}FNO_4$ (467) FAB m/e = 468 (M + $H^+$)

**Beispiel 6:** Verfahrensschritt A8 (Schema 1)

3(RS), 5(RS)-Dihydroxy-7-[1-phenyl-2-isopropyl-4-(4-fluorphenyl)-5-methyl-1H-pyrrol-3-yl] -heptansäure-Natriumsalz

[Formel I mit AB = $CH_2$-$CH_2$, $R^1$ = Na, $R^2$ = Isopropyl, $R^3$ = Phenyl, $R^4$ = $CH_3$, $R^5$ = p-Fluorphenyl]

wird aus dem Produkt aus Beispiel 5 in Analogie zu Bsp. 3 erhalten.

**Beispiel 7:**

Synthese von 3(RS), 5(SR)-Dihydroxy-7-[1-phenyl-2-isopropyl-4-(4-fluorphenyl)-1H-pyrrol-3-yl]-hept-6(E)-ensäure-methylester

[Formel Ia mit trans -CH = CH, $R^1$ = $CH_3$, $R^2$ = Isopropyl, $R^3$ = Phenyl, $R^4$ = H, $R^5$ = p-Fluorphenyl]

**Beispiel 7.1.**

[1-Phenyl-2-isopropyl-4-(4-fluorphenyl) -1H-pyrrol-3-carbonsäureanilid](Schema 4 c)

Die Lösung von 12.5 g (75 mmol) 1-p-Fluorphenyl-2-nitroethylen (Gattermann-Wieland, 43. Aufl., Seite 361) und 23.1 g (82.5 mmol) 3-Phenylamino-4-methyl-pent-2(E)-ensäure-anilid (Beispiel 4.1.) in 200 ml Ethanol wird 18 Std. unter Rückfluß gekocht. Das Lösungsmittel wird abgezogen und der Rückstand in wenig Cyclohexan/Essigester 9:1 + 1 % $NEt_3$ aufgenommen. Dabei geht ein großer Teil des Reaktionsprodukts nicht in Lösung. Der Feststoff wird abgesaugt und aus Methanol umkristallisiert. 11.94 g farbloser Feststoff, Schmp. 192-194 °C.

Das obige Filtrat wird mit dem gleichen Laufmittel über Kieselgel chromatographiert und ergibt weitere 2.8 g des Produkts.
$^1$H-NMR ($CD_2Cl_2$):$\delta$ = 1.30(d, 6H, $C(CH_3)_2$), 3.14(sept., 1H, CH), 6.73(s, 1H, arom.-H), 7.00-7.70(m, 10H, Amid-H und 9 arom.-H)

**Beispiel 7.2..** Verfahrensschritt D4

[1-Phenyl-2-isopropyl-4-(4-fluorphenyl) 1H-pyrrol-3-carbonsäure-N-methylanilid]

[Formel XX mit $R^2$ = Isopropyl, $R^3$ = Phenyl, $R^4$ = H, $R^5$ = p-Fluorphenyl, $R^{12}$ = H]

In Analogie zu Beispiel 4.3. erhält man aus 14.4 g des Anilids aus Bsp. 7.1. nach Chromatographie über Kieselgel mit Cyclohexan/Essigester/Triethylamin 8:2:0.01 14.5 g farblosen Feststoff, Schmp. 126-127 °C.
MS: $C_{27}H_{25}FN_2O$(412) m/e = 412 ($M^+$), 306, 262

**Beispiel 7.3.** Verfahrensschritt D5 (Schema 3)

[1-Phenyl-2-isopropyl-3-hydroxymethyl-4-(4-fluorphenyl)-1H-pyrrol]

[Formel IV mit $R^2$ = Isopropyl, $R^3$ = Phenyl, $R^4$ = H, $R^5$ = p-Fluorphenyl]

In Analogie zu Beispiel 4.4. erhält man aus 14.5 g des N-Methylanilids aus Bsp. 7.2. 10.4 g zähes, farbloses Öl.
1H-NMR($CDCl_3$): δ = 1.28(d, 7H, $C(CH_3)_2$ + OH), 3.03(sept., 1H, CH), 4.70(s, 2H, $CH_2$), 6.73(s, 1H, arom.-H), 6.90-7.70(m, 9H, arom.-H)
MS: $C_{20}H_{20}FNO$ (309) m/e = 309 ($M^+$), 294, 276, 77

**Beispiel 7.4.** Verfahrensschritt D3 (Schema 3)

[1-Phenyl-2-isopropyl-4-(4-fluorphenyl)] -1H-pyrrol-3-carbaldehyd

[Formel III mit $R^2$ = Isopropyl, $R^3$ = Phenyl, $R^4$ = H, $R^5$ = p-Fluorphenyl]

Zur Suspension von 25.9 g (0.258 Mol) fein gepulvertem Chromtrioxid und 15 g Celite in 250 ml absol. Methylenchlorid tropft man bei 15-20 °C unter Stickstoff die Lesung von 40.9 g (0.517 Mol) Pyridin in 50 ml absol. Methylenchlorid. Man rührt 20 Min. bei Raumtemp. und tropft dann sehr rasch die Lösung von 8 g (25.8 mmol) des Alkohols aus Beispiel 7.3. in 150 ml absol. Methylenchlorid zu. Die Innentemp. wird mit Hilfe von Eiskühlung zwischen 20 und 24 °C gehalten. Nach 15 Min. gibt. man 500 ml Cyclohexan zu, saugt durch einen Celite-Filter ab und wäscht mit Methylenchlorid/Cyclohexan 3:7. Das Filtrat wird eingeengt, der Kolbenrückstand in Cyclohexan/Essigester 6:4 aufgenommen und erneut durch einen Celite-Filter abgesaugt. Es wird gut nachgewaschen, das Filtrat eingeengt und der Kolbenrückstand mit Cyclohexan/Essigester 4:1 über 500 g Kieselgel chromatographiert.
2.4 g gelbliches Öl, das langsam kristallisiert.

Alternative Prozedur

Zur Lösung von 46.8 g (400 mmol) N-Methylmorpholin-N-oxid in 400 ml Aceton (über $K_2CO_3$ getrocknet) gibt man 3.8 g (4.0 mmol) Tris(triphenylphosphin)-ruthenium(II)dichlorid. Die Mischung wird 20 min. bsi 20 °C gerührt. Die Lösung des Alkohols aus Bsp. 7.3. (30.9 g, 100 mmol) in 600 ml trockenem Aceton wird zugetropft und das Reaktionsgemisch 20 Std. bei Raumtemperatur gerührt. Man filtriert durch eine kurze, dicke Kieselgel-Schicht (ca. 500 g) und wäscht mit 3 1 Ether nach. Das Filtrat wird in Vak. eingeengt und mit Cyclohexan/Essigester/Triethylamin 4:1:0.1 über 800 g Kieselgel chromatographiert. 26.6 g (87% Ausb.) blaßgelbe Kristalle, Schmp. 119-120 °C.
1H-NMR($CDCl_3$): δ = 1.36(d, 6H, $C(CH_3)_2$), 3.16(sept., 1H, CH), 6.65(s, 1H, arom.-H), 7.0-7.7(m, 9H, arom.-H), 10.1(s, 1H, CHO)
MS: $C_{20}H_{18}FNO$ (307) m/e = 307 ($M^+$), 292, 77

**Beispiel 7.5.** Verfahrensschritt Z1

3- [1-Phenyl-2-isopropyl-4-(4-fluorphenyl)-1H-pyrrol-3-yl]-(E)-propenal]

[Formel XI mit $R^2$ = Isopropyl, $R^3$ = Phenyl, $R^4$ = H, $R^5$ = p-Fluorphenyl]

In Analogie zu Beispiel 1.6 erhält man aus 2.4 g des Aldehyds aus Beispiel 7.4 neben 1.1 g unumgesetzen Ausgangsmaterial 1.3 g des Produkts als Öl, das langsam kristallisiert. Quantitativer Umsatz sollte sich durch verlängerte Reaktionszeit und (oder) etwas höhere Reaktionstemperatur erzielen lassen.
$^1$H-NMR ($CDCl_3$): δ = 1.35 (d, 6H, $C(CH_3)_2$), 3.16 (sept., 1H, CH), 6.05 (dd, J = 16Hz und 7,6Hz, 1H, =CH), 6.63 (s, 1H, arom.-H), 7.0 - 7.5 (m, 9H, arom.-H), 7.75 (d, J = 16Hz, 1H, =CH), 9.50 (d, J = 7,6Hz, 1H, CHO)
MS: $C_{22}H_{20}FNO$ (333) DCI m/e = 334.2 ($M + H^+$), 290.2

**Beispiel 7.6.** Verfahrensschritt B1

5(RS)-Hydroxy-3-oxo-7-[1-phenyl-2-isopropyl-4-(4-fluorphenyl)-1H-pyrrol-3-yl]-hept-6(E)-ensäure-methylester

[Formel XII mit $R^1$ = $CH_3$, $R^2$ = Isopropyl, $R^3$ = Phenyl, $R^4$ = H, $R^5$ = p-Fluorphenyl]

In Analogie zu Beispiel 1.7 erhält man aus 1.3 g des Aldehydes aus Beispiel 7.5 1.4 g des Produkts (Öl)

MS: $C_{27}H_{28}FNO_4$ (449) m/e = 449 ($M^+$), 432 ($M^+$-OH), 373, 334, 290

**Beispiel 7.7.** Verfahrensschritt B2

3 (RS), 5 (SR)-Dihydroxy-7-[1-phenyl-2-isopropyl-4-(4-fluorphenyl)-1H-pyrrol-3-yl]-hept-6(E)-ensäure-methylester

[Formel I a mit trans-HC=CH, $R^1$ = $CH_3$, $R^2$ = Isopropyl, $R^3$ = Phenyl, $R^4$ = H, $R^5$ = p-Fluorphenyl]

In Analogie zu Beispiel 1.8 erhält man aus 1.4 g des $\beta$-Ketoesters aus Beispiel 7.6. 1.28 g Produkt (zähes Öl).
$^1$H-NMR ($C_6D_6$): $\delta$ = 1.30 (d+m, 7H, $C(CH_3)_2$ und $CH_2$), 1.57 (dt, 1H, $CH_2$), 2.03 (dd, 1H, $CH_2$), 2.18 (dd, 1H, $CH_2$), 2.70 (s, breit, 1H, OH), 3.09 (sept., 1H, CH), 3.27 (s, 3H, $OCH_3$), 3.45 (s, breit, 1H, OH), 4.03 (m, 1H, CH), 4.34 (m, 1H, CH), 5.67 (dd, J=16 und 6Hz, 1H, =CH), 6.50 (s, 1H, arom.-H), 6.87 - 7.15 (m, 8H, =CH und 7 arom.-H), 7.45 (dd, 2H, arom.-H).
MS: $C_{27}H_{30}FNO_4$ (451) m/e = 451 ($M^+$), 433 ($M^+$-$H_2O$), 334, 292, 290, 276

**Beispiel 8:** Verfahrensschritt A9 (Schema 1)

3 (RS), 5 (RS)- Dihyroxy-7-[1-phenyl-2-isopropyl-4-(4-fluorphenyl)-1H-pyrrol-3-yl]-heptansäure-Methylester

[Formel I mit AB = $CH_2$-$CH_2$, $R^1$ = $CH_3$, $R^2$ = Isopropyl, $R^3$ = Phenyl, $R^4$ = H, $R^5$ = p-Fluorphenyl]

wird aus dem Produkt aus Beispiel 7.7. in Analogie zu Beispiel 2 erhalten.
$^1$H-NMR ($C_6D_6$, 270 MHz): $\delta$ = 1.03 (dt, 1H, $CH_2$), 1.28 - 1.43 (m, z.T. verdeckt, 1H, $CH_2$), 1.32 (d, 3H, $CH_3$), 1.33 (d, 3H, $CH_3$), 1.60 - 1.85 (m, 2H, $CH_2$), 1.94 (dd, 1H, $CH_2$), 2.12 (dd, 1H, $CH_2$), 2.90 - 3.02 (m, 1H, $CH_2$), 3.03 - 3.22 (m, 3H, $CH_2$, CH, 1 OH), 3.24 (s, 3H, $OCH_3$), 3.43 (s, breit, 1H, 1 OH), 3.75 (m, 1H, CH), 3.88 (m, 1H, CH), 6.58 (s, 1H, arom.-H), 6.94 (AA'BB', 2H, arom.-H), 7.03 - 7.15(m, 5H, arom.-H), 7.42 (AA'BB', 2H, arom.-H)
MS: $C_{27}H_{32}FNO_4$ (453) FAB m/e = 454 (M + $H^+$), 292

**Beispiel 9:** Verfahrensschritt A8 (Schema 1)

3 (RS), 5 (RS)- Dihydroxy-7-[(1-phenyl-2-isopropyl-4-(4-fluorphenyl)-1H-pyrrol-3-yl ]-heptansäure-Natriumsalz

[Formel I mit AB = $CH_2$-$CH_2$, $R^1$ = Na, $R^2$ = Isopropyl, $R^3$ = Phenyl, $R^4$ = H, $R^5$ = p-Fluorphenyl]

Zur Lösung von 3.43 mmol des Methylesters aus Bsp. 8 in 100 ml Methanol gibt man bei 0 °C 3.45 ml 1N Natronlauge und rührt 2 Std. Das Lösungsmittel wird im Vakuum abgezogen, 20 ml Methanol zugesetzt und wieder im Vakuum abgezogen. Der Rückstand wird mit Ether verrieben, dekantiert und nochmals mit Ether verrieben. Dabei tritt Kristallisation ein. Lösungsmittel werden im Hochvakuum entfernt.

Das Produkt hat einen Schmp. von 230-233 °C (Zersetz.).

**Beispiel 10:**

Synthese von 3 (RS), 5 (SR)-Dihydroxy-7- 1-isopropyl-2-methyl-4-(4-fluorphenyl)-1H-pyrrol-3-µl -hept-6(E)-ensäure-methylester

[Formel Ia mit trans-HC=CH, $R^1$ = $CH_3$, $R^2$ = $CH_3$, $R^3$ = Isopropyl, $R^4$ = H, $R^5$ = p-Fluorphenyl]

**Beispiel 10.1.**

[1-Isopropyl-2-methyl-3-methoxycarbonyl-4-(4-fluorphenyl)-1H-pyrrol

[Formel XVIII mit $R^2$ = $CH_3$, $R^3$ = Isopropyl, $R^4$ = H, $R^5$ = p-Fluorphenyl]

a) N,N-Bis[3-(4-fluorphenyl)-4-methoxycarbonyl-5-methyl-2,3-dihydrofuran-2-yl)hydroxylamin (Schema 4

a)
Zur Lösung von 2.92 g (54 mmol) Natriummethanolat in 54 ml Methanol gibt man bei 0 °C unter Rühren 20.9 g (180 mmol) Acetessigsäuremethylester, dann portionsweise 30.1 g (180 mmol) 4-Fluor-β-nitrostyrol (1-p-fluorphenyl-2-nitroethylen, hergestellt gemäß Gattermann-Wieland "Die Praxis des organischen Chemikers", 43. Aufl., W.de Gruyter Berlin 1962, Seite 361). Nach 15 min. Rühren bei 0 °C entsteht ein dicker Brei, der 2 Std. bei 0 °C stehen gelassen wird. Der Niederschlag wird abgesaugt, mit eiskaltem Methanol gewaschen und im Vakuum über Phosphorpentoxid getrocknet. 22.0 g farbloser Feststoff, Schmp. 135-137 °C. Die Mutterlauge wird auf ca. 75 ml eingeengt. Das auskristallisierte Produkt wird abgesaugt, gewaschen, getrocknet. 7.0 g farbloser Feststoff, Schmp. 139 - 141 °C,
$^1$H-NMR (DMSO-$d_6$): δ = 2.25 (s, 6H, 2 x C-$CH_3$), 3.32 (s, 3H, $OCH_3$), 3.50 (s, 3H, $OCH_3$), 4.30 (dd, 2H, CH), 5.40 (d, 2H, CH), 7.16 (d, 8H, arom.-H), 8.72 (s, 1H, OH)
MS: $C_{26}H_{25}F_2NO_7$ (501) FAB m/e = 502 (M + $H^+$), 458, 235
b) 15 g (30 mmol) des Hydroxylamins aus a) werden in 500 ml Methanol suspendiert, 3.6 g (60 mmol) Isopropylamin zugegeben und 2 Std. bei 40 °C, 5 Std. bei 50 °C gerührt. Dabei geht die Suspension in eine klare Lösung über. Das Lösungsmittel wird im Vakuum abdestilliert und der Rückstand mit n-Hexan/Diethylether 4:1 über Kieselgel chromatographiert. 7.3 g leicht rötlich gefärbte Kristalle, Schmp. 97 - 99 °C.
$^1$H-NMR ($CDCl_3$): δ = 1.42 (d, 6H, $C(CH_3)_2$, 2.53 (s, 3H, $CH_3$), 3.65 (s, 3H, $OCH_3$), 4.37 (sept., 1H, CH), 6.60 (s, 1H, arom.-H), 6.80 - 7.46 (m, 4H, arom.-H)
MS: $C_{16}H_{18}FNO_2$ (275.3) m/e = 275 ($M^+$), 244, 202, 201

**Beispiel 10.2.** Verfahrensschritt D2 (Schema 3)

[1-Isopropyl-2-methyl-3-hydroxymethyl-4-(4-fluorphenyl) 1H-pyrrol]

[Formel IV mit $R^2$ = $CH_3$, $R^3$ = Isopropyl, $R^4$ = H, $R^5$ = p-Fluorphenyl]

Zur Suspension von 1.13 g (29.75 mmol) Lithiumaluminiumhydrid in 150 ml trock. Ether gibt man bei 0 °C innerhalb 5 min. die Lösung von 2.75 g (11.9 mmol) des Methylesters aus Bsp.10.1 in 75 ml Ether. Man rührt 1 Stunde bei 0 °C und tropft dann bei dieser Temperatur nacheinander 10 ml Essigester, 2.5 ml Wasser und 5 ml 2N Natronlauge zu. Man rührt 15 min., saugt den anorgan. Niederschlag ab, versetzt das Filtrat mit 2 ml Triethylamin und engt im Vakuum ein. Der Rückstand wird mit Cyclohexan/Essigester 5:2 +0.1 % Triethylamin über Kieselgel chromatographiert.
MS: $C_{15}H_{18}FNO$ (247.3) m/e = 247 ($M^+$-OH), 188

**Beispiel 10.3.** Verfahrensschritt D3 (Schema 3)

[1-Isopropyl-2-methyl-4-(4-fluorphenyl)]-1H-pyrrol-3-carboxaldhyd

[ Formel III mit $R^2$ = $CH_3$, $R^3$ = Isopropyl, $R^4$ = H, $R^5$ = p-Fluorphenyl]

In Analogie zu Beispiel 1.5 erhält man aus 1.5 g des Alkohols aus Bsp. 10.2 1.3 g farbloses Öl.
$^1$H-NMR ($CDCl_3$): δ = 1.43 (d, 6H, $C(CH_3)_2$), 2.60 (s, 3H, $CH_3$), 4.30 (sept., 1H, CH), 6.68 (s, 1H, arom.-H), 6.9-7.56 (m, 4H, arom.-H), 9.92 (s, 1H, CHO)
MS: $C_{15}H_{16}FNO$ (245) m/e = 245 ($M^+$), 202

**Beispiel 10.4.** Verfahrensschritt Z1

3- [1-Isopropyl-2-methyl-4-(4-fluorphenyl)-1H-pyrrol-3-yl]-(E)-propenal

[Formel XI mit $R^2$ = $CH_3$, $R^3$ = Isopropyl, $R^4$ = H, $R^5$ = p-Fluorphenyl]

In Analogie zu Beispiel 1.6 erhält man aus 1.3 g des Aldehyds aus Beispiel 10.3. 1.35 g farblose Kristalle.
$^1$H-NMR ($CDCl_3$): δ = 1.47 (d, 6H, $C(CH_3)_2$), 2.43 (s, 3H, $CH_3$), 4.42 (sept., 1H, CH), 6.20 (dd, J = 16 und 7,8Hz, 1H, =CH), 6.72 (s, 1H, arom.-H), 6.9 - 7.5 (m, 4H, arom.-H), 7.50 (d, J = 16Hz, 1H, =CH), 9.48 (d, 1H, CHO)
MS: $C_{17}H_{18}FNO$ (271) m/e = 271 ($M^+$), 256, 242, 200

**Beispiel 10.5.** Verfahrensschritt B1

5(RS)-Hydroxy-3-oxo-7-[1-isopropyl-2-methyl-4-(4-fluorphenyl)-1H-pyrrol-3-yl]-hept-6(E)-ensäure-methylester

[Formel XII mit $R^1 = CH_3$, $R^2 = CH_3$, $R^3 =$ Isopropyl, $R^4 =$ H, $R^5 =$ p-Fluorphenyl]

In Analogie zu Bsp. 1.7 erhält man aus 1.2 g des Aldehyds aus Beispiel 10.4 1.42 g blaßgelbes Öl.
$^1$H-NMR ($CDCl_3$, 270MHz): $\delta$ = 1.44 (d + verd. Signal, 8H, $C(CH_3)_2$ + $CH_2$), 1.58 (s, breit, 1H, OH), 2.37 (s, 3H, $CH_3$), 3.58 (s, 2H, $CH_2$), 3.75 (s, 3H, $OCH_3$), 4.35 (sept., 1H, CH), 6.02 (d, J = 16,0Hz, 1H, =CH), 6.27 (dd, J = 16.0 und 11.2Hz, 1H, =CH), 6.67 (s, 1H, arom.-H), 7.06 (AA'BB', 2H, arom.-H), 7.28 (AA'BB', 2H, arom.-H)
MS: $C_{22}H_{26}FNO_4$ (387.4) m/e = 387 ($M^+$), 369, 272

**Beispiel 10.6.** Verfahrensschritt B2

3 (RS), 5 (SR)- Dihydroxy-7-[1-isopropyl-2-methyl-4-(4-fluorphenyl)-1H-pyrrol-3-yl]-hept-6(E)-ensäure-methylester

[Formel Ia mit trans-HC=CH, $R^1 = CH_3$, $R^2 = CH_3$, $R^3 =$ Isopropyl, $R^4 =$ H, $R^5 =$ p-Fluorphenyl]

In Analogie zu Bsp. 1.8 erhält man aus 1.42 g des $\beta$-Ketoesters aus Bsp. 10.5 803 mg blaßgelbes Öl.
$^1$H-NMR ($C_6D_6$, 270MHz): $\delta$ = 0.98 (d, 6H, $C(CH_3)_2$), 1.40 (dt, 1H, $CH_2$), 1.68 (dt, 1H, $CH_2$), 2.05 (s, 3H, $CH_3$), 2.09 (dd, z.T. verdeckt, 1H, $CH_2$), 2.27 (dd, 1H, $CH_2$), 3.27 (s, 3H, $OCH_3$), 3.73 (sept., 1H, CH), 4.14 (m, 1H, CH), 4.34 (m, 1H, CH), 5.72 (dd, J = 16.0 und 7.0 Hz, 1H, =CH), 6.50 (s, 1H, arom.-H), 6.73 (d, J = 16.0Hz, 1H, =CH), 6.98 (AA'BB', 2H, arom.-H), 7.43 (AA'BB', 2H, arom.-H)
MS: $C_{22}H_{28}FNO_4$ (389.5) m/e = 389 ($M^+$), 272, 230

**Beispiel 11:** Verfahrensschritt A8 (Schema 1)

3 (RS), 5 (SR) - Dihydroxy-7-[1-isopropyl-2-methyl-4-(4- fluorphenyl)-1H-pyrrol-3-yl]-hept-6(E)-ensäure-Natriumsalz

[Formel Ia mit trans -CH=CH, $R^1 =$ Na, $R^2 = CH_3$, $R^3 =$ Isopropyl, $R^4 =$ H, $R^5 =$ p-Fluorphenyl]

wird aus dem Produkt aus Beispiel 10.6 in Analogie zu Beispiel 3 erhalten.

**Beispiel 12:** Verfahrensschritt A9 (Schema 1)

3 (RS), 5 (RS)-Dihydroxy-7-[1-isopropyl-2-methyl-4-(4-fluorphenyl)-1H-pyrrol-3-yl]-heptansäure-methylester

[Formel I mit AB = $CH_2$-$CH_2$, $R^1 = CH_3$, $R^2 = CH_3$, $R^3 =$ Isopropyl, $R^4 =$ H, $R^5 =$ p-Fluorphenyl]

wird aus dem Produkt aus Beispiel 10.6 in Analogie zu Beispiel 2 erhalten.
$^1$H-NMR ($C_6D_6$, 270MHz): $\delta$ = 1.02 (2 x d , 6H, $C(CH_3)_2$), 1.38 (dt, 2H, $CH_2$), 1.50 - 1.75 (m, 2H, $CH_2$), 1.97 (dd, 1H, $CH_2$), 2.10 (s, 3H, $CH_3$), 2.15 (dd, 1H, $CH_2$), 2.82 (m, 2H, $CH_2$), 3.27 (s, 3H, $OCH_3$), 3.70 (m, 1H, CH), 3.78 (sept., 1H, CH), 3.93 (m, 1H, CH), 6.58 (s, 1H, arom.-H), 6.98 (AA'BB', 2H, arom.-H), 7.39 (AA'BB', 2H, arom.-H)
MS: $C_{22}H_{30}FNO_4$ (391) DCI m/e = 392.2 (M + $H^+$), 391 ($M^+$), 360.2, 331.1, 230

**Beispiel 13:** Verfahrensschritt A8 (Schema 1)

3 (RS), 5 (RS)-Dihydroxy-7-[1-isopropyl-2-methyl-4-(4-fluorphenyl)-1H-pyrrol-3-yl]heptansäure-Natriumsalz

[Formel I mit AB = $CH_2$-$CH_2$, $R^1 = CH_3$, $R^2 = CH_3$, $R^3 =$ Isopropyl, $R^4 =$ H, $R^5 =$ p-Fluorphenyl]

wird aus dem Methylester aus Beispiel 12 in Analogie zu Beispiel 3 erhalten.

**Beispiel 14**

Synthese von 3(RS), 5(SR)-Dihydroxy-7-[1-cyclohexyl-2-isopropyl-4-(4-fluorphenyl)-1H-pyrrol-3-yl]-hept-6-(E)-ensäure-methylester

[Formel Ia mit trans-HC=CH, $R^1$ = $CH_3$, $R^2$ = Isopropyl, $R^3$ = Cyclohexyl, $R^4$ = H, $R^5$ = p-Fluorphenyl]

**Beispiel 14.1**

3-Oxo-4-methyl-pentansäureanilid

Die Lösung aus 47.4 g (0.3 mol) 3-Oxo-4-methyl-pentansäureethylester, 27.93 g (0.3 mol) Anilin und 0.6 ml Eisessig in 360 ml Toluol wird 4 Stunden am Wasserabscheider unter Rückfluß gekocht. Das erkaltete Reaktionsgemisch wird zweimal mit 0.5 N Salzsäure, dann zweimal mit gesättigter Natriumbicarbonat-Lösung, dann mit gesättigter Kochsalzlösung gewaschen, getrocknet, im Vakuum eingeengt. Der Rückstand wird mit Toluol/Essigester 10:1 über 1 kg Kieselgel chromatographiert. 40.5 g (66 % Ausbeute) blaßrosafarbenes Öl.
$^1$H-NMR ($CDCl_3$): $\delta$ = 1.2 (d,6H), 2.8 (s, 2H), 3.65 (s, 2H), 7.0-7.75 (m, 5H), 9.1-9.4 (s, br., 1H)
MS: $C_{12}H_{15}NO_2$ (205) m/e = 205 ($M^+$), 93

**Beispiel 14.2**

3-Cyclohexylamino-4-methyl-pent-2(E)-ensäure-anilid (Schema 4d)

Die Lösung von 31.6 g (154 mmol) des Anilids aus Bsp. 14.1, 30.55 g (308 mmol) Cyclohexylamin, 1.5 ml Eisessig in 750 ml Toluol wird 20 Stunden am Wasserabscheider unter Rückfluß gekocht. Das Lösungsmittel wird im Vakuum abgezogen und der Rückstand mit 150 ml Diisopropylether gerührt, wobei Kristallisation einsetzt. Der Feststoff wird abgesaugt und mit Petrolether gewaschen. 27.1 g farbloser Feststoff, weitere 8.9 g aus der Mutterlauge (Ausbeute 82 %). Der Schmp. war nicht scharf (123-132 °C), aber NMR zeigte ausreichende Reinheit an.
$^1$H-NMR ($CDCl_3$): $\delta$ = 1.15 (d, 6H), 1.0-2.1 (m, 10H), 2.7 (sept., 1H), 3.45 (m, 1H), 4.4 (s, 1H), 6.55 (m, 1H), 6.9-7.6 (m, 5H), 9.5 (s, br., 1H)
MS: $C_{18}H_{26}N_2O$ (286) m/e = 286 ($M^+$), 194, 93

**Beispiel 14.3**

1-Cyclohexyl-2-isopropyl-4-(4-fluorphenyl)-1H-pyrrol-3-carbonsäureanilid

[Formel XIX mit $R^2$ = Isopropyl, $R^3$ = Cyclohexyl, $R^{3'}$ = Phenyl, $R^4$ = H, $R^5$ = p-Fluorphenyl

In Analogie zu Beispiel 7.1 erhält man in 52 %iger Ausbeute einen farblosen Feststoff, Schmp. 215-216 °C (nicht umkristallisiert).
$^1$H-NMR ($CDCl_3$): $\delta$ = 0.9-2.2 (d+m, 16H), 3.5-4.3 (m, 2H), 6.65 (s, 1H), 6.8-7.6 (m, 10H)
MS: $C_{26}H_{29}FN_2O$ (404) CI m/e = 405 ($M+H^+$), 312, 230

**Beispiel 14.4** Verfahrensschritt D.4 (Schema 3)

1-Cyclohexyl-2-isopropyl-4-(4-fluorphenyl)-1H-pyrrol-3-carbonsäure-N-methylanilid

[Formel XX mit $R^2$ = Isopropyl, $R^3$ = Cyclohexyl $R^{3'}$ = Phenyl, $R^4$ = H, $R^5$ = p-Fluorphenyl]

In Analogie zu Beispiel 4.3 erhält man in 98 %iger Ausbeute einen farblosen harzigen Feststoff, der beim längeren Stehen kristallisiert. Schmp. 102-105 °C (nicht umkristallisiert)
$^1$H-NMR ($CDCl_3$): $\delta$ = 1.35 (d, 3H), 1.50 (d, 3H), 1.1-2.2 (m, 11H), 3.25 (s, br., 3H), 3.95 (m, 1H), 6.4-7.4 (m, 10H)
MS: $C_{27}H_{31}FN_2O$ (418) CI m/e = 419 ($M+H^+$), 312

**Beispiel 14.5** Verfahrensschritt D5 (Schema 3)

1-Cyclohexyl-2-isopropyl-3-hydroxymethyl-4-(4-fluorphenyl)-1H-pyrrol

[Formel IV mit $R^2$ = Isopropyl, $R^3$ = Cyclohexyl, $R^4$ = H, $R^5$ = p-Flurophenyl]

In Analogie zu Beispiel 4.4 erhält man in 67 %iger Ausbeute einen farblosen Feststoff, Schmp. 114-116 °C (nicht umkristallisiert)
$^1$H-NMR ($CDCl_3$): $\delta$ = 1.37 (d, 6H), 1.2-2.1 (m, 10H), 3.30 (sept., 1H), 3.96 (m, 1H), 4.38 (s, 2H), 6.70 (s, 1H), 6.95 (m, 2H), 7.47 (m, 2H)
MS: $C_{20}H_{26}FNO$ (315) m/e = 315 ($M^+$), 300, 282, 200

**Beispiel 14.6** Verfahrensschritt D3 (Schema 3)

[1-Cyclohexyl-2-isopropyl-4-(4-fluorphenyl)[-1H-pyrrol-3-carbaldehyd

[Formel III mit $R^2$ = Isopropyl, $R^3$ = Cyclohexyl, $R^4$ = H, $R^5$ = p-Fluorphenyl]

Zur Lösung von 12.9 g (110 mmol) N-Methylmorpholin-N-oxid (97 %ig) in 110 ml Aceten (über Kaliumcarbonat getrocknet) gibt man bei Raumtemperatur 1.06 g (1.1 mmol) Tris(triphenylphosphin)-ruthenium(II)chlorid. Man rührt 20 Minuten und tropft dann die Lösung von 8.67 g (27.5 mmol) des Pyrrolalkohols aus Beispiel 19.5 in 200 ml Aceton zu. Man rührt 19 Std. unter Luftausschluß, filtriert das Reaktionsgemisch über eine kurze, dicke Kieselgelsäule (ca. 500 ml Kieselgel) und wäscht die Säule gut mit Ether. Die vereinigten Filtrate werden eingeengt. Man erhält 8.4 g (97 % Ausbeute) farblose Kristalle, Schmp. 134-135 °C (nicht umkristallisiert).
$^1$H-NMR ($CDCl_3$): $\delta$ = 1.45 (d, 6H), 1.1-2.2 (m, 10H), 3.55-4.35 (m + sept., 2H), 6.65 (s, 1H), 6.9-7.6 (m, 4H), 9.95 (s, 1H)
MS: $C_{20}H_{24}FNO$ (313) m/e = 313 ($M^+$), 298, 231, 216

**Beispiel 14.7** Verfahrensschritt Z2

$\beta$-[1-Cyclohexyl-2-isopropyl-4-(4-fluorphenyl)-1H-pyrrol-3-yl]-(E)-acrylnitril

[Formel XVII mit $R^2$ = Isopropyl, $R^3$ = Cyclohexyl, $R^4$ = H, $R^5$ = p-Fluorphenyl]

Zur Suspension von 37.8 mg 50 %igem Natriumhydrid/Mineralöl (0.787 mmol) in 7 ml abs. THF tropft man bei 0 °C die Lösung von 135 mg (0.66 mmol) Diisopropylcyanomethylphosphonat in 2 ml THF. Man rührt 40 Min. bei 0 °C und tropft dann die Lösung von 0.437 mmol des Aldehyds aus Bsp. 14.6 in 5 ml THF zu. Man rührt 5 min bei 0 °C und 2 Std. bei Raumtemperatur.

Das Reaktionsgemisch wird in wäßrige Kochsalzlösung gegossen, mit Ether extrahiert und das etherische Extrakt mit Kochsalzlösung gewaschen, dann getrocknet, filtriert und eingeengt. Der Rückstand wird mit Cyclohexan/Essigester 6:1 + 0.1 % Triethylamin über Kieselgel chromatographiert.

Man erhält in 96 %iger Ausbeute einen blaßgelben Feststoff, Schmp. 130-132 °C (nicht umkristallisiert)
$^1$H-NMR ($CDCl_3$): $\delta$ = 1.40 (d, 6H), 1.2-2.1 (, 10H), 3.30 (sept., 1H), 4.00 (m, 1H), 4.95 (d, 1H), 6.60 (s, 1H), 6.9-7.4 (m, 4H), 7.55 (d, 1H)
MS: $C_{22}H_{25}FN_2$ (336) m/e 336 ($M^+$), 321, 239

**Beispiel 14.8** Verfahrensschritt Z3

3-[1-Cyclohexyl-2-isoproyl-4-(4-fluorphenyl)-1H-pyrrol-3-yl]-(E)-propenal

[Formel XI mit $R^2$ = Isopropyl, $R^3$ = Cyclohexyl, $R^4$ = H, $R^5$ = p-Fluorphenyl]

Zur Lösung von 0.24 mmol des obigen Nitrils (aus Bsp. 14.7) in 5 ml abs. THF tropft man bei 0 °C 0.6 ml einer 1.2 M Lösung von Diisobutylaluminiumhydrid in Toluol (0.72 mmol). Man rührt 1 Std. bei 0 °C und 1.5 Std. bei Raumtemperatur und tropft bei 0 °C vorsichtig 1 ml gesättigte Natriumdihydrogenphosphatlösung, dann 2 ml Wasser zu. Man rührt 30 Min. bei 0 °C, 1 Std. bei Raumtemperatur. Das Reaktionsgemisch wird mit gesättigter Kochsalzlösung versetzt und 2 mal mit Ether extrahiert. Die vereinigten Organ. Phasen werden mit $NaHCO_3$-Lösung gewaschen, getrocknet, filtriert und eingeengt. Der Rückstand wird mit

Cyclohexan/Essigester 5:1 + 0.1 % Triethylamin über Kieselgel chromatographiert.

Man erhält in 81 %iger Ausbeute blaßgelbe Kristalle, Schmp. 124 °C (nicht umkristallisiert)
$^1$H-NMR ($CDCl_3$): $\delta$ = 1.46 (d, 6H), 1.3-2.2 (m, 10H), 3.50 (sept., 1H), 4.00 (m, 1H), 6.05 (dd, 1H), 6.65 (s, 1H), 6.9-7.5 (m, 4H), 7.65 (d, 1H), 9.50 (d, 1H)
MS: $C_{22}H_{26}FNO$ (339) m/e = 339 ($m^+$), 296, 214

**Beispiel 14.9** Verfahrensschritt B1

5(RS)-Hydroxy-3-oxo-7-[1-cyclohexyl-2-isopropyl-4-(4-fluorphenyl)-1H-pyrrol-3-yl]-hept-6(E)-ensäure-methylester

[Formel XII mit $R^1$ = $CH_3$, $R^2$ = Isopropyl, $R^3$ = Cyclohexyl, $R^4$ = H, $R^5$ = p-Fluorphenyl]

erhält man in Analogie zu Beispiel 1.7 in 82 %iger Ausbeute
$^1$H-NMR ($CD_2Cl_2$): $\delta$ = 1.35 (d, 6H), ~1.3-2.3 (m,10H), 2.35 (d, 1H), 2.65 (d, 2H), 3.30 (sept., 1H), 3.50 (s, 2H), 3.70 (s, 3H), 4.00 (m, 1H), 4.60 (m, 1H), 5.35 (dd, 1H), 6.65 (s, 1H), 6.65 (d, 1H), 6.85-7.50 (m, 4H)
MS: $C_{27}H_{34}FNO_4$ (455) m/e 455 ($M^+$) 437, 340, 296, 214

**Beispiel 14.10** Verfahrensschritt B2

3(RS),5(SR)-Dihydroxy-7-[1-cyclohexyl-2-isopropyl-4-(4-fluorphenyl)-1H-pyrrol-3-yl]-hept-6(E)-ensäure-methylester

[Formel Ia mit trans-HC=CH, $R^1$ = $CH_3$, $R^2$ = Isopropyl, $R^3$ = Cyclohexyl, $R^4$ = H, $R^5$ = p-Fluorphenyl]

erhält man in Analogie zu Beispiel 1.8.

**Beispiel 15** Verfahrensschritt A9 (Schema 1)

3(RS),5(RS)-Dihydroxy-7-[1-cyclohexyl-2-isopropyl-4- (4-fluorphenyl)-1H-pyrrol-3-yl]-heptansäure-methylester

[Formel I mit AB = $CH_2$-$CH_2$, $R^1$ = $CH_3$, $R^2$ = Isopropyl, $R^3$ = Cyclohexyl, $R^4$ = H, $R^5$ = p-Fluorphenyl]

erhält man in Analogie zu Beispiel 2 aus dem Produkt von Beispiel 14.10.

**Beispiel 16** Verfahrensschritt A8 (Schema 1)

3(RS),5(RS)-Dihydroxy-7-[1-cyclohexyl-2-isopropyl-4-(4-fluorphenyl)-1H-pyrrol-3-yl]-heptansäure-Natriumsalz

[Formel I mit AB = $CH_2$-$CH_2$, $R^1$ = Na, $R^2$ = Isopropyl, $R^3$ = Cyclohexyl, $R^4$ = H, $R^5$ = p-Fluorphenyl]

erhält man in Analogie zu Beispiel 9 aus dem Produkt von Beispiel 15.

**Beispiel 17**

Synthese von 3(RS),5(SR)-Dihydroxy-7-[1,2-diisopropyl-4-(4-fluorphenyl)-1H-pyrrol-3-yl]-hept-6(E)-ensäure-methylester

[Formel Ia mit trans-HC=CH, $R^1$ = $CH_3$, $R^2$ = Isopropyl, $R^3$ = Isopropyl, $R^4$ = H, $R^5$ = p-Fluorphenyl]

**Beispiel 17.1**

3-Isopropylamino-4-methyl-pent-2(E)-ensäure-anilid (Schema 4d)

35.7 g (174 mmol) des Anilids aus Beispiel 14.1 und 0.6 ml Eisessig werden in 600 ml Toluol gelöst. In das am Wasserabscheider unter Rückfluß kochende Gemisch wurde sehr langsam 20.6 g (348 mmol) Isopropylamin getropft. Nach 16 stündigem Rückfluß wurde eingeengt und abgekühlt. Das Kristallisat wurde

mit Diisopropylether/Petrolether 1:1 verrührt. Der Feststoff wurde abgesaugt und mit Petrolether gewaschen. 27 g farbloser Feststoff, Schmp. 151-153 °C. Aus der Mutterlauge wurden weiter 1.9 g, Schmp. 148-150 °C, gewonnen.
$^1$H-NMR ($CDCl_3$): $\delta$ = 1.1 (d, 6H), 1.25 (d, 6H), 2.73 (sept., 1H), 3.8 (m, 1H), 4.43 (s, 1H), 6.7 (s, 1H), 6.9-7.6 (m, 5H), 9.1-9.6 (s, br., 1H)
MS: $C_{15}H_{22}N_2O$ (246) CI c/e = 247 ($M+H^+$), 154

**Beispiel 17.2**

1,2-Diisopropyl-4-(4-fluorphenyl)-1H-pyrrol-3-carbonsäureanilid

[Formel XIX mit $R^2$ = Isopropyl, $R^3$ = Isopropyl, $R^{3'}$ = Phenyl, $R^4$ = H, $R^5$ = p-Fluorphenyl]

In Analogie zu Beispiel 7.1 erhält man in 50 %iger Ausbeute einen farblosen Feststoff, Schmp. 131-133 °C (nicht umkristallisiert)
$^1$H-NMR ($CDCl_3$): $\delta$ = 1.45 (d, 6H), 1.55 (d, 6H), 3.75 (sept., 1H), 4.60 (sept., 1H), 6.70 (s, 1H), 6.9-7.6 (m, 10H)
MS: $C_{23}H_{25}FN_2O$ (364) m/e = 364 ($M^+$), 272, 230

**Beispiel 17.3** Verfahrensschritt D4 (Schema 3)

1,2-Diisopropyl-4-(4-fluorphenyl)-1H-pyrrol-3-carbonsäure-N-methylanilid

[Formel XX mit $R^2$ = Isopropyl, $R^3$ = Isopropyl, $R^{3'}$ = Phenyl, $R^4$ = H, $R^5$ = p-Fluorphenyl]

In Analogie zu Beispiel 4.3 erhält man in 73 %iger Ausbeute ein Öl.
$^1$H-NMR ($CDCl_3$): $\delta$ = 1.40 (d, 12H), 3.23 (s + sept., 4H), 4.40 (sept., 1H), 6.50 (s, 1H), 6.5-7.5 (m, 9H)
MS: $C_{24}H_{27}FN_2O$ (378) m/e = 378 ($M^+$), 272, 91

**Beispiel 17.4** Verfahrensschritt D5 (Schema 3)

1,2-Diisopropyl-3-hydroxymethyl-4-(4-fluorphenyl)-1H-pyrrol

[Formel IV mit $R^2$ = Isopropyl, $R^3$ = Isopropyl, $R^4$ = H, $R^5$ = p-Fluorphenyl]

In Analogie zu Beispiel 4.4 erhält man in 75 % Ausbeute ein blaßgelbes Öl, das langsam kristallisiert.
$^1$H-NMR ($CDCl_3$): $\delta$ = 1.2-1.6 (m, 12H), 2.35 (s, br., 1H), 3.33 (sept., 1H), 4.40 (s, 2H), 4.50 (sept., 1H), 6.70 (s, 1H), 6.8-7.65 (m, 4H)
MS: $C_{17}H_{22}FNO$ (275) CI m/e = 275 ($M^+$), 258, 242, 200

**Beispiel 17.5** Verfahrensschritt D3 (Schema 3)

[1,2-Diisopropyl-4-(4-fluorphenyl)]-1H-pyrrol-3-carbaldehyd

[Formel III mit $R^2$ = Isopropyl, $R^3$ = Isopropyl, $R^4$ = H, $R^5$ = p-Fluorphenyl]

In Analogie zu Beispiel 14.6 erhält man in 87 %iger Ausbeute ein gelbliches Öl.
$^1$H-NMR ($CDCl_3$): $\delta$ = 1.43 (d, 6H), 1.47 (d, 6H), 3.80 (sept., 1H), 4.57 (sept., 1H), 6.62 (s, 1H), 7.06 (m, 2H), 7.37 (m, 2H), 9.89 (s, 1H)
MS: $C_{17}H_{20}FNO$ (273) m/e = 273 ($M^+$), 258, 244

**Beispeil 17.6** Verfahrensschritt Z2

$\beta$-[1,2-Diisopropyl-4-(4-fluorphenyl)-1H-pyrrol-3-yl]-(E)-acrylnitril

[Formel XVII mit $R^2$ = Isopropyl, $R^3$ = Isopropyl, $R^4$ = H, $R^5$ = p-Fluorphenyl]

In Analogie zu Beispiel 14.7. erhält man in 91 % Ausbeute Kristalle vom Schmp. 121-123 °C (nicht

umkristallisiert)
$^1$H-NMR ($CDCl_3$): $\delta$ = 1.43 (2xd, 12H), 3.30 (sept., 1H), 4.50 (sept., 1H), 4.93 (d, 1H), 6.60 (s, 1H), 6.9-7.4 (m, 4H), 7.53 (d, 1H)
MS: $C_{19}H_{21}N_2F$ (296) m/e = 296 ($M^+$), 281, 256, 239

**Beispiel 17.7** Verfahrensschritt Z3

3-[1,2-Diisopropyl-4-(4-fluorphenyl)-1H-pyrrol-3-yl]-(E)-propenal

[Formel XI mit $R^2$ = Isopropyl, $R^3$ = Isopropyl, $R^4$ = H, $R^5$ = p-Fluorphenyl]

In Analogie zu Beispiel 14.8 erhält man in 70 %iger Ausbeute einen farblosen Feststoff, Schmp. 119-121 °C (nicht umkristallisiert)
$^1$H-NMR ($CDCl_3$): $\delta$ = 1.45 (2xd, 12H), 3.45 (sept., 1H), 4.53 (sept., 1H), 6.00 (dd, 1H), 6.65 (s, 1H), 6.9-7.5 (m, 4H), 7.63 (d, 1H), 9.45 (d, 1H)
MS: $C_{19}H_{22}FNO$ (299) m/e = 299 ($M^+$), 256, 214

**Beispiel 17.8** Verfahrensschritt B1

5(RS)-Hydroxy-3-oxo-7-[1,2-diisopropyl-4-(4-fluorphenyl)-1H-pyrrol-3-yl]-hept-6(E)-ensäure-methylester

[Formel XII mit $R^1$ = $CH_3$, $R^2$ = Isopropyl, $R^3$ = Isopropyl, $R^4$ = H, $R^5$ = p-Fluorphenyl]

erhält man in Analogie zu Beispiel 1.7 aus dem Produkt aus Beispiel 17.7 in 80 %iger Ausbeute.
$^1$H-NMR ($CD_2Cl_2$): $\delta$ = 1.36 (d, 6H), 1.42 (d, 6H), 2.37 (d, 1H), 2.68 (m, 2H), 3.30 (sept., 1H), 3.48 (s, 2H), 3.70 (s, 3H), 4.44 (sept., 1H), 4.59 (m, 1H), 5.32 (dd, 1H), 6.62 (d, 1H), 6.62 (s, 1H), 7.00 (m, 2H), 7.30 (m, 2H)
MS: $C_{24}H_{30}FNo_4$ (415) m/e = 415 ($M^+$), 397, 300, 256

**Beispiel 17.9** Verfahrensschritt B2

3(RS),5(SR)-Dihydroxy-7-[1,2-diisopropyl-4-(4-fluorphenyl)-1H-pyrrol-3-yl]- hept-6(E)-ensäure-methylester

[Formel Ia mit trans-HC=CH, $R^1$ = $CH_3$, $R^2$ = Isopropyl, $R^3$ = Isopropyl, $R^4$ = H, $R^5$ = p-Fluorphenyl]

erhält man in Analogie zu Beispiel 1.8.

**Beispiel 18** Verfahrensschritt A9 (Schema 1)

3(RS),5(RS)-Dihydroxy-7-[1,2-diisopropyl-4-(4-fluorphenyl)-1H-pyrrol-3-yl]-heptansäure-methylester

[Formel I mit AB = $CH_2$-$CH_2$, $R^1$ = $CH_3$, $R^2$ = Isopropyl, $R^3$ = Isopropyl, $R^4$ = H, $R^5$ = p-Fluorphenyl]

erhält man in Analogie zu Beispiel 2 aus dem Produkt von Beispiel 17.9.

**Beispiel 19** Verfahrensschritt A8 (Schema 1)

3(RS),5(RS)-Dihydroxy-7-[1,2-diisopropyl-4-(4-fluorphenyl)-1H-pyrrol-3-yl]-heptansäure-Natriumsalz

[Formel I mit AB = $CH_2$-$CH_2$, $R^1$ = Na, $R^2$ = Isopropyl, $R^3$ = Isopropyl, $R^4$ = H, $R^5$ = p-Fluorphenyl]

erhält man in Analogie zu Beispiel 9 aus dem Produkt aus Beispiel 18.

## Patentansprüche

## Patentansprüche für folgende Vertragsstaaten : AT, BE, CH, DE, FR, GB, IT, LI, LU, NL, SE

**1.** 7-[1H-Pyrrol-3-yl]-substituierte 3,5-Dihydroxyheptansäuren und deren Derivate der allgemeinen Formel I

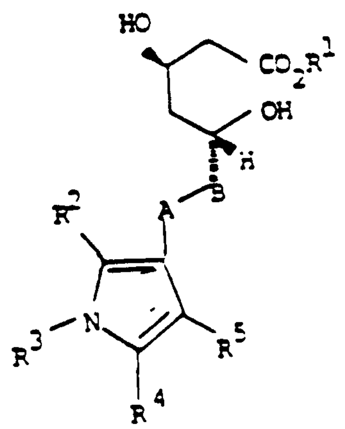

I

sowie entsprechenden δ-Lactone der Formel II

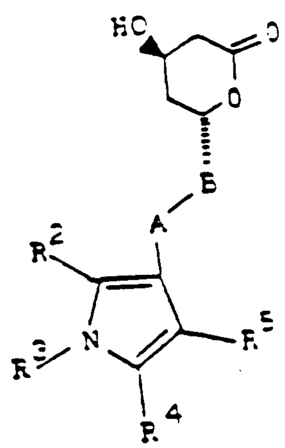

II

wobei in den Formeln

A-B eine Ethandiyl-Gruppierung $-CH_2-CH_2-$ bedeutet,

$R^1$ Wasserstoff, Alkyl mit 1 bis 4 Kohlenstoffatomen, Phenyl, Benzyl, 2,3-Dihydroxypropyl, ein pharmakologisch verträgliches Alkali- oder Erdalkalimetallkation, $NH_4$ oder ein mit 1-4 Alkylgruppen und jeweils 1-4 C-Atomen substituiertes Ammoniumion bedeutet,

$R^2$, $R^3$, $R^4$, $R^5$ unabhängig voneinander bedeuten

1) Wasserstoff

2) geradkettiges oder verzweigtes Alkyl mit 1-12 Kohlenstoffatomen, Cycloalkyl mit 5-7 C-Atomen, das gegebenenfalls über eine geradkettige oder verzweigte Alkylkette von 1-5- Kohlenstoffatomen an den heterocyclischen Aromaten gebunden ist.

3) Phenyl oder Benzyl, unsubstituiert oder 1-3 fach substituiert mit

a) Fluor, Chlor, Brom oder Trifluormethyl,

b) Alkyl oder Alkenyl mit bis zu 5 Kohlenstoffatomen, wobei orthoständige Alkyl- oder Alkenyl-Substituenten unter Ausbildung eines anellierten carbocyclischen Rings verbunden sein können,

c) Alkoxy mit 1-5 Kohlenstoffatomen

d) Alkoxycarbonyl mit 2-6 Kohlenstoffatomen,

ausgenommen die Verbindung der Formel I, worin $R^1$ Methyl, $R^2$ i-Propyl, $R^3$ Methyl, $R^4$ Phenyl und $R^5$ 4-Fluorphenyl bedeuten.

**2.** Verbindungen gemäß Anspruch 1, dadurch gekennzeichnet, daß in Formel I

A-B $-CH_2-CH_2-$ bedeutet

| | |
|---|---|
| $R^1$ | Methyl, Ethyl oder Natrium bedeutet |
| $R^2$, $R^3$, $R^4$, $R^5$ | unabhängig voneinander bedeuten |

1) Wasserstoff
2) Methyl, Ethyl, Isopropyl, tert.-Butyl, Cyclohexyl
3) Phenyl, das 1-2 fach substituiert sein kann mit
   a) Fluor, Chlor
   b) Methyl

sowie die entsprechenden δ-Lactone der allgemeinen Formel II.

**3.** Pharmazeutische Präparate enthaltend eine Verbindung gemäß Anspruch 1.

**4.** Verbindung gemäß Anspruch 1 zur Prophylaxe und Therapie der Hypercholesterinämie.

**5.** Verfahren zur Herstellung der Verbindungen der Formeln I und II, dadurch gekennzeichnet, daß man

A)

1. einen Carbaldehyd der Formel III

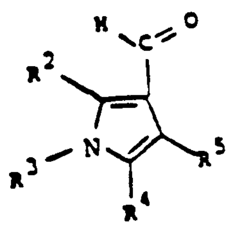

III

reduziert zu einem Alkohol der Formel IV

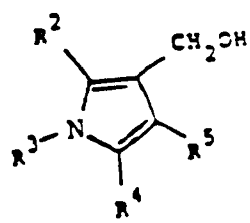

IV

2. den Alkohol IV in das Halogenderivat der Formel V

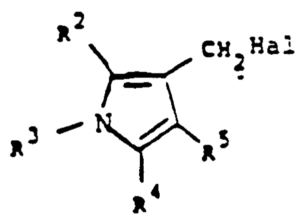

V

worin Hal Chlor, Brom oder Jod bedeutet, überführt

3. die Halogenverbindungen der Formel V in das Phosphoniumsalz der Formel VI

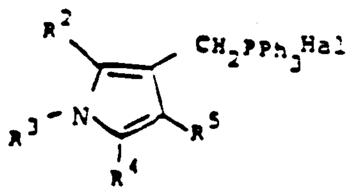

VI

worin Hal Chlor, Brom oder Jod bedeutet, überführt,

4. das Phosphoniumsalz der Formel VI mit dem Compactinaldehyd der Formel VII

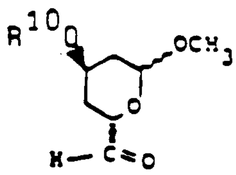

VII

worin $R^{10}$ eine gegen Basen und schwache Säuren stabile Schutzgruppe bedeutet, z.B. t-Butyldiphenylsilyl, umsetzt zu dem Lactolether der Formel VIII

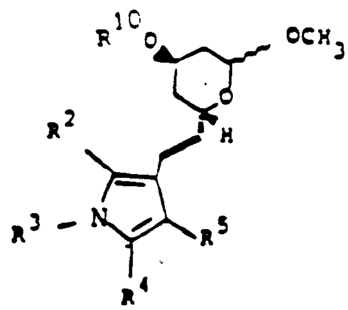

VIII

5. in dem Lactolether der Formel VIII die Methylacetalfunktion sauer hydrolysiert zu einem Lactol der Formel IX

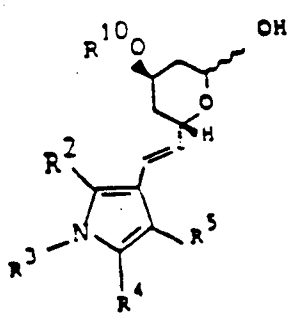

IX

6. das Lactol der Formel IX oxydiert zu einem Lacton der Formel X

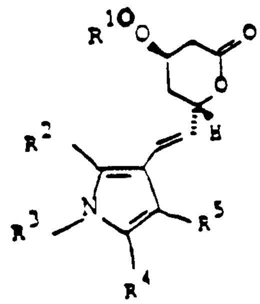

X

7. in dem Lacton der Formel X die Schutzgruppe $R^{10}$, z. B.

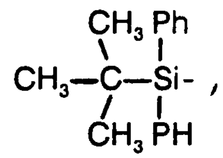

abspaltet zu einer Verbindung der Formel II a

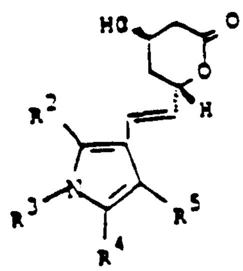

II a

worin $R^2$ bis $R^5$ die angegebenen Bedeutungen haben und

8. gegebenenfalls eine erhaltene Verbindung der Formel II a, worin $R^2$ bis $R^5$ die angegebenen Bedeutungen haben, in die entsprechende Dihydroxy-hept-6-ensäure der Formel Ia

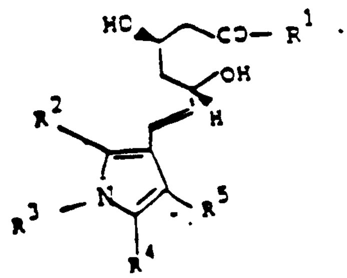

I a

worin $R^1$ bis $R^5$ die angegebenen Bedeutungen haben, überführt und

9. in einer erhaltenen Verbindung der Formel I a oder II a mit CH=CH die Doppelbindung hydriert zu einer Verbindung der Formel I oder II mit A-B gleich $CH_2$-$CH_2$, wobei die Hydrierung auch bei den Zwischenstufen VIII, IX oder X sowie auch vor der Überführung von Verbindungen II a nach I a erfolgen kann, oder

B)

1. den $\alpha,\beta$-ungesättigten Aldehyd der Formel XI

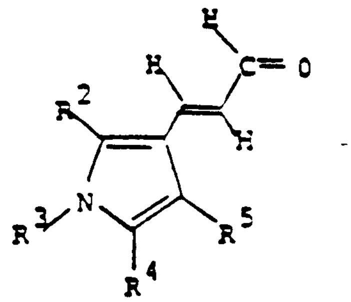

XI

einer Aldolreaktion mit dem Dianion des Acetessigsäuremethylesters unterwirft, wobei eine Verbindung der Formel XII

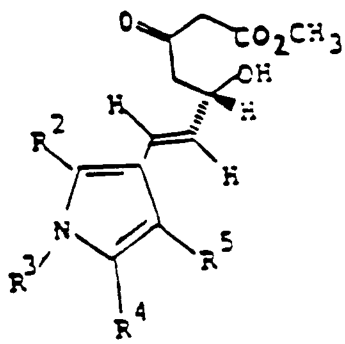

XII

entsteht,

2. in einer Verbindung der Formel XII die Ketogruppe stereoselektiv reduziert zu einer Verbindung der Formel I a

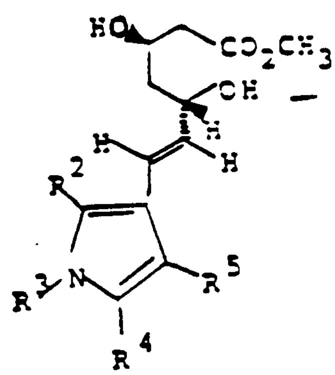

I a

worin $R^2$ bis $R^5$ die angegebenen Bedeutungen haben, $R^1$ die Methylgruppe ist,

3. gegebenenfalls eine erhaltene Verbindung der Formel Ia, worin $R^1$ die $CH_3$-Gruppe bedeutet, in eine Verbindung I a überführt, worin $R^1$ bis $R^5$ die zu Formel I angegebenen Bedeutungen haben und

4. in einer erhaltenen Verbindung der Formel Ia die Doppelbindung hydriert zu einer Verbindung der Formel I mit A-B gleich $-CH_2-CH_2-$, und

5. gegebenenfalls eine erhaltene Verbindung der Formel I mit $R^1 = H$ unter Abspaltung von Wasser in ein Lacton der Formel II überführt, oder

C)

1. das Enolat eines Essigsäureesters der Formel XIII

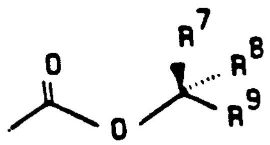

XIII

worin $R^7$ einen Phenylring, der unsubstituiert ist oder substituiert ist mit Fluor, Chlor, Brom, verzweigtem oder unverzweigtem Alkyl mit 1-4 Kohlenstoffatomen, $R^8$ ein H-Atom oder Methyl, und $R^9$ Diphenylhydroxymethyl $Ph_2COH$, wobei die Phenylringe unsubstituiert sind oder substituiert sind mit Fluor, Chlor, Brom, verzweigtem oder unverzweigtem Alkyl mit 1-4 Kohlenstoffatomen, bedeuten, in einer Aldolreaktion mit dem $\alpha,\beta$-ungesättigten Aldehyd der Formel XI

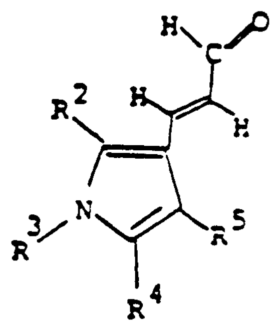

XI

zum Addukt der Formel XIV

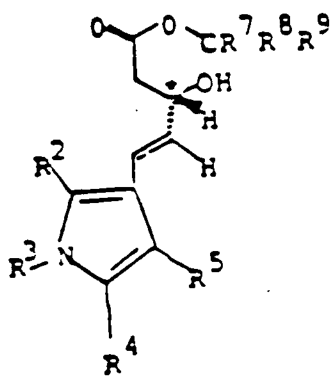

XIV

worin $R^2$ bis $R^5$ die zu Formel I und $R^7$, $R^8$, $R^9$ die zu Formel XIII angegebenen Bedeutungen haben, umsetzt,

2. das Addukt der Formel XIV durch Umesterung in den $\beta$-Hydroxymethylester der Formel XV

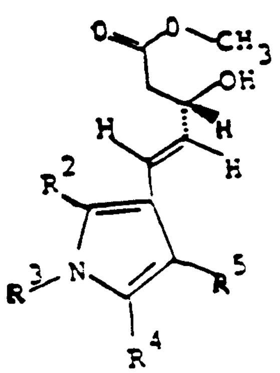

XV

überführt,

3. den erhaltenen Methylester der Formel XV mit dem Enolat eines Essigsäurealkylesters in den β-Keto-δ-(S)-hydroxyester der Formel XVI

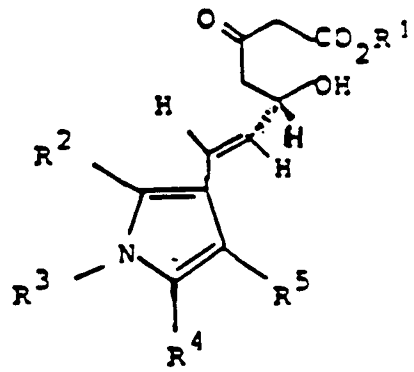

XVI

worin $R^2$ bis $R^5$ die zu Formel I angegebenen Bedeutungen haben und $R^1$ eine Alkylgruppe mit 1-4 C-Atomen ist, überführt und

4. in einer erhaltenen Verbindung der Formel XVI die Ketogruppe stereoselektiv reduziert zu einer Verbindung der Formel Ia

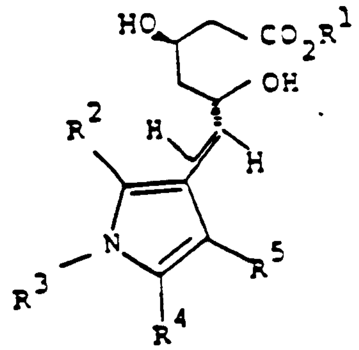

I a

worin $R^2$ bis $R^5$ die zu Formel I angegebenen Bedeutungen haben, $R^1$ eine Alkylgruppe mit 1-4 C-Atomen ist,

5. gegebenenfalls eine erhaltene Verbindung der Formel Ia worin $R^1$ eine Alkylgruppe mit 1-4 C-Atomen ist, in eine Verbindung der Formel Ia überführt, worin $R^1$ bis $R^5$ die zu Formel I angegebenen Bedeutungen haben und

6. in einer erhaltenen Verbindung der Formel Ia die Doppelbindung hydriert zu einer Verbindung der Formel I mit A-B- gleich $-CH_2-CH_2-$ und

7. gegebenenfalls eine erhaltene verbindung der Formel I mit $R^1$ = Wasserstoff unter Abspaltung von Wasser in ein Lacton der Formel II überführt.

**Patentansprüche für folgende Vertragsstaaten : ES, GR**

**1.** Verfahren zur Herstellung von 7-[1H-Pyrrol-3-yl]-substituierte 3,5-Dihydroxyheptansäuren und deren Derivaten der allgemeinen Formel I

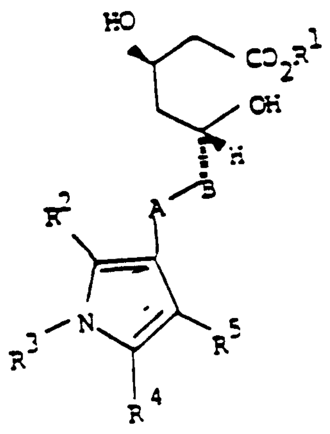

I

sowie den entsprechenden δ-Lactonen der Formel II

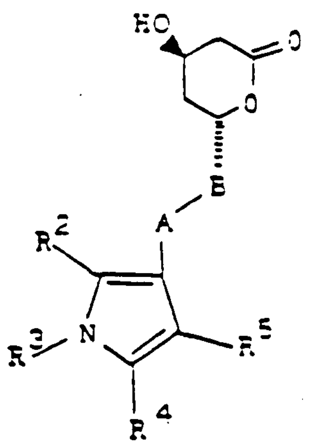

II

wobei in den Formeln

| | |
|---|---|
| A-B | eine Ethandiyl-Gruppierung $-CH_2-CH_2-$ bedeutet, |
| $R^1$ | Wasserstoff, Alkyl mit 1 bis 4 Kohlenstoffatomen, Phenyl, Benzyl, 2,3-Dihydroxypropyl, ein pharmakologisch verträgliches Alkali- oder Erdalkalimetallkation, $NH_4$ oder ein mit 1-4 Alkylgruppen und jeweils 1-4 C-Atomen substituiertes Ammoniumion bedeutet, |
| $R^2$, $R^3$, $R^4$, $R^5$ | unabhängig voneinander bedeuten |

1) Wasserstoff

2) geradkettiges oder verzweigtes Alkyl mit 1-12 Kohlenstoffatomen, Cycloalkyl mit 5-7 C-Atomen, das gegebenenfalls über eine geradkettige oder verzweigte Alkylkette von 1-5- Kohlenstoffatomen an den heterocyclischen Aromaten gebunden ist.

3) Phenyl oder Benzyl, unsubstituiert oder 1-3 fach substituiert mit

a) Fluor, Chlor, Brom oder Trifluormethyl,

b) Alkyl oder Alkenyl mit bis zu 5 Kohlenstoffatomen, wobei orthoständige Alkyl- oder Alkenyl-Substituenten unter Ausbildung eines anellierten carbocyclischen Rings verbunden sein können,

c) Alkoxy mit 1-5 Kohlenstoffatomen

d) Alkoxycarbonyl mit 2-6 Kohlenstoffatomen,

ausgenommen die Verbindung der Formel I, worin $R^1$ Methyl, $R^2$ i-Propyl, $R^3$ Methyl, $R^4$ Phenyl und $R^5$ 4-Fluorphenyl bedeuten,

dadurch gekennzeichnet, daß man

A)

1. einen Carbaldehyd der Formel III

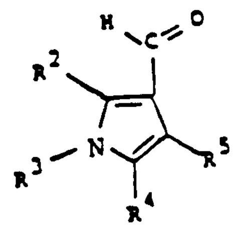

III

reduziert zu einem Alkohol der Formel IV

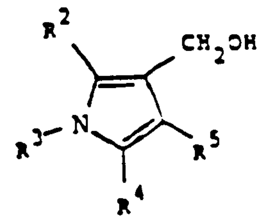

IV

2. den Alkohol IV in das Halogenderivat der Formel V

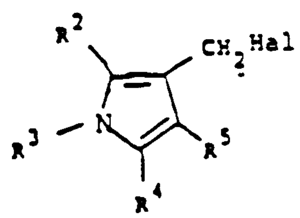

V

worin Hal Chlor, Brom oder Jod bedeutet, überführt
3. die Halogenverbindungen der Formel V in das Phosphoniumsalz der Formel VI

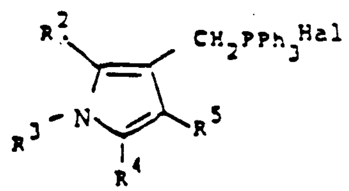

VI

worin Hal Chlor, Brom oder Jod bedeutet, überführt,
4. das Phosphoniumsalz der Formel VI mit dem Compactinaldehyd der Formel VII

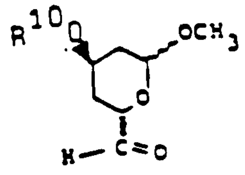

VII

worin $R^{10}$ eine gegen Basen und schwache Säuren stabile Schutzgruppe bedeutet, z.B. t-Butyldiphenylsilyl, umsetzt zu dem Lactolether der Formel VIII

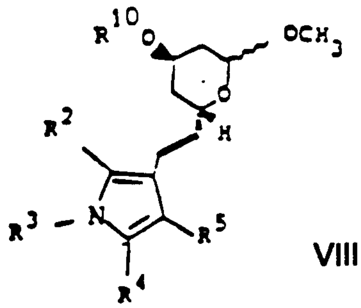

VIII

5. in dem Lactolether der Formel VIII die Methylacetalfunktion sauer hydrolysiert zu einem Lactol der Formel IX

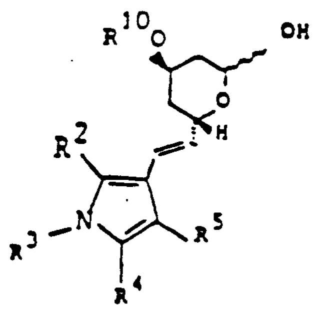

IX

6. das Lactol der Formel IX oxydiert zu einem Lacton der Formel X

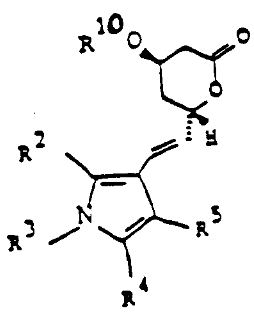

X

7. in dem Lacton der Formel X die Schutzgruppe $R^{10}$, z. B.

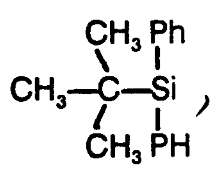

abspaltet zu einer Verbindung der Formel II a

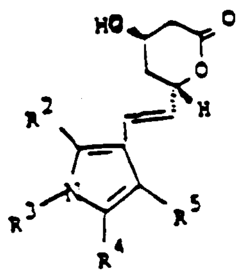

II a

worin $R^2$ bis $R^5$ die angegebenen Bedeutungen haben und

8. gegebenenfalls eine erhaltene Verbindung der Formel II a, worin $R^2$ bis $R^5$ die angegebenen Bedeutungen haben in die entsprechende Dihydroxy-hept-6-ensäure der Formel Ia

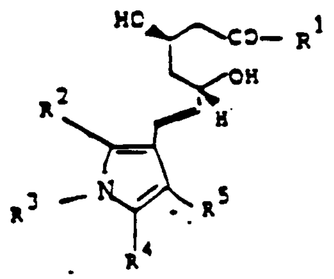

I a

worin $R^1$ bis $R^5$ die angegebenen Bedeutungen haben, überführt und

9. in einer erhaltenen Verbindung der Formel I a oder II a die Doppelbindung hydriert zu einer Verbindung der Formel I oder II mit A-B gleich $CH_2$-$CH_2$, wobei die Hydrierung auch bei den zwischenstufen VIII, IX oder X sowie auch vor der Überführung von Verbindungen II a nach I a erfolgen kann, oder

B)

1. den $\alpha,\beta$-ungesättigten Aldehyd der Formel XI

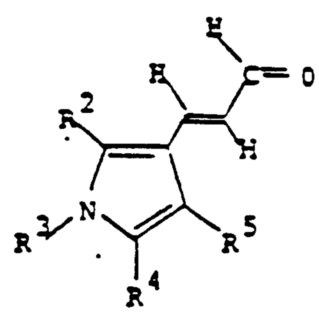

XI

einer Aldolreaktion mit dem Dianion des Acetessigsäuremethylesters unterwirft, wobei eine Verbindung der Formel XII

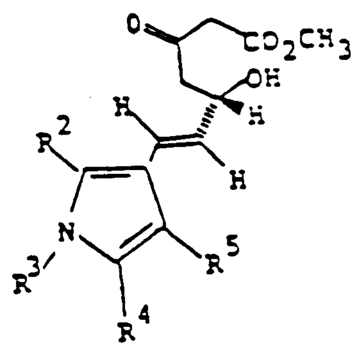

XII

entsteht,

2. in einer Verbindung der Formel XII die Ketogruppe stereoselektiv reduziert zu einer Verbindung der Formel I a

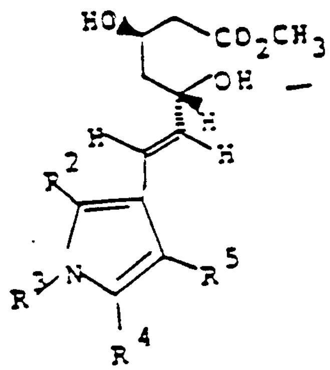

I a

worin $R^2$ bis $R^5$ die angegebenen Bedeutungen haben, $R^1$ die Methylgruppe ist

3. gegebenenfalls eine erhaltene Verbindung der Formel Ia, worin $R^1$ die $CH_3$-Gruppe bedeutet, in eine Verbindung I a überführt, worin $R^1$ bis $R^5$ die zu Formel I angegebenen Bedeutungen haben, und

4. in einer erhaltenen Verbindung der Formel I a die Doppelbindung hydriert zu einer Verbindung der Formel I mit A-B gleich $-CH_2-CH_2-$, und

5. gegebenenfalls eine erhaltene Verbindung der Formel I mit $R^1 = H$ unter Abspaltung von Wasser in ein Lacton der Formel II überführt, oder

C)

1. das Enolat eines Essigsäureesters der Formel XIII

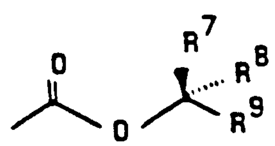

XIII

worin $R^7$ einen Phenylring, der unsubstituiert ist oder substituiert ist mit Fluor, Chlor, Brom, verzweigtem oder unverzweigtem Alkyl mit 1-4 Kohlenstoffatomen, $R^8$ ein H-Atom oder Methyl, und $R^9$ Diphenylhydroxymethyl $Ph_2COH$, wobei die Phenylringe unsubstituiert sind oder substituiert sind mit Fluor, Chlor, Brom, verzweigtem oder unverzweigtem Alkyl mit 1-4 Kohlenstoffatomen, bedeuten, in einer Aldolreaktion mit dem $\alpha,\beta$-ungesättigten Aldehyd der Formel XI

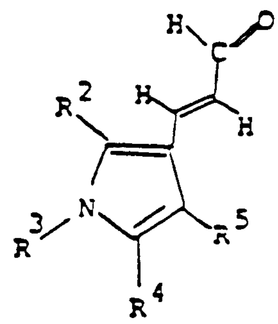

XI

zum Addukt der Formel XIV

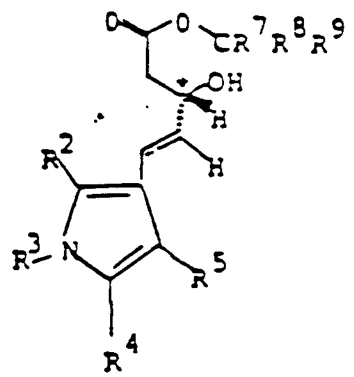

XIV

worin $R^2$ bis $R^5$ die zu Formel I und $R^7$, $R^8$, $R^9$ die zu Formel XIII angegebenen Bedeutungen haben, umsetzt,

2. das Addukt der Formel XIV durch Umesterung in den β-Hydroxymethylester der Formel XV

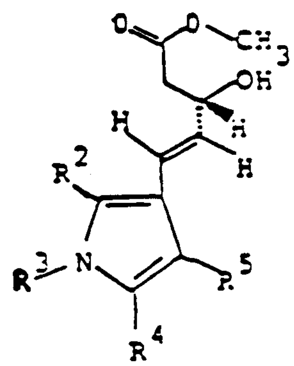

XV

überführt,

3. den erhaltenen Methylester der Formel XV mit dem Enolat eines Essigsäurealkylesters in den β-Keto-δ-(S)-hydroxyester der Formel XVI

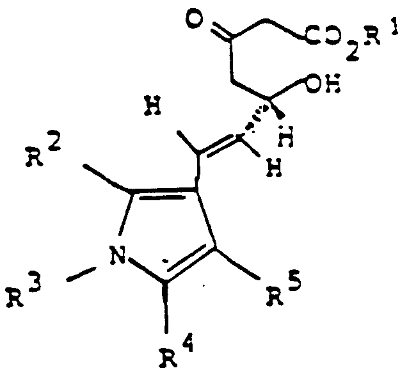

XVI

worin $R^2$ bis $R^5$ die zu Formel I angegebenen Bedeutungen haben und $R^1$ eine Alkylgruppe mit 1-4 C-Atomen ist, überführt und

4. in einer erhaltenen Verbindung der Formel XVI die Ketogruppe stereoselektiv reduziert zu einer Verbindung der Formel Ia

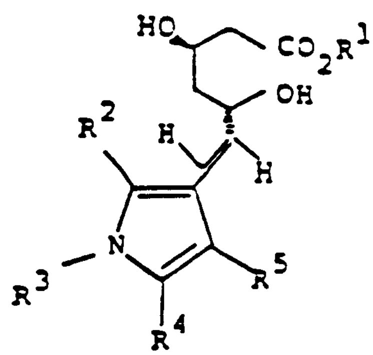

I a

worin $R^2$ bis $R^5$ die zu Formel I angegebenen Bedeutungen haben, $R^1$ eine Alkylgruppe mit 1-4 C-Atomen ist,

5. gegebenenfalls eine erhaltene Verbindung der Formel Ia, worin $R^1$ eine Alkylgruppe mit 1-4 C-Atomen ist, in eine Verbindung der Formel Ia überführt, worin $R^1$ bis $R^5$ die zu Formel I angegebenen Bedeutungen haben, und

6. in einer erhaltenen Verbindung der Formel Ia die Doppelbindung hydriert zu einer Verbindung der Formel I mit A-B- gleich $-CH_2-CH_2-$ und

7. gegebenenfalls eine erhaltene Verbindung der Formel I mit $R^1$ = Wasserstoff unter Abspaltung von Wasser in ein Lacton der Formel II überführt.

**2.** Verfahren gemäß Anspruch 1, dadurch gekennzeichnet, daß man eine Verbindung der Formel I, worin

A-B $-CH_2-CH_2-$ bedeutet

$R^1$ Methyl, Ethyl oder Natrium bedeutet

$R^2$, $R^3$, $R^4$, $R^5$ unabhängig voneinander bedeuten

1) Wasserstoff

2) Methyl, Ethyl, Isopropyl, tert.-Butyl, Cyclohexyl

3) Phenyl, das 1-2 fach substituiert sein kann mit

a) Fluor, Chlor

b) Methyl

bedeuten oder ein entsprechendes δ-Lacton der allgemeinen Formel II herstellt.

**3.** Verfahren zur Herstellung eines pharmazeutischen Präparates, dadurch gekennzeichnet, daß man eine Verbindung der Formel I oder II gemäß Anspruch 1 in eine geeignete Darreichungsform überführt.

**Claims**

**Claims for the following Contracting States : AT, BE, CH, DE, FR, GB, IT, LI, LU, NL, SE**

**1.** 7-[1H-Pyrrol-3-yl]-substituted 3,5-dihydroxyheptanoic acids and their derivatives of the formula I

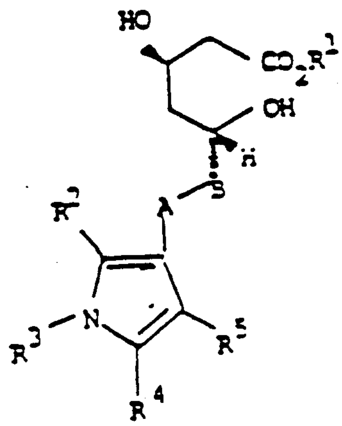

I

and corresponding δ-lactones of the formula II

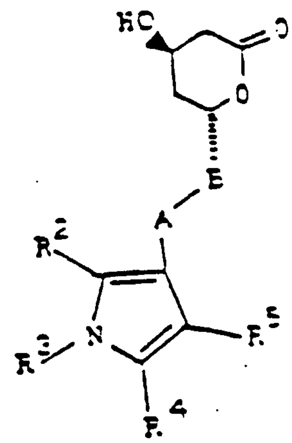

II

in which formulae

| | |
|---|---|
| A-B | denotes an ethanediyl group $-CH_2-CH_2-$, |
| $R^1$ | denotes hydrogen, alkyl having 1 to 4 carbon atoms, phenyl, benzyl or 2,3-dihydroxypropyl, or a pharmacologically tolerated alkali or alkaline earth metal cation, $NH_4$ or an ammonium ion substituted with 1 to 4 alkyl groups each having 1-4 carbon atoms, |
| $R^2$, $R^3$, $R^4$ and $R^5$ | denote, independently of one another,<br>1) hydrogen<br>2) straight-chain or branched alkyl having 1-12 carbon atoms, or cycloalkyl which has 5-7 carbon atoms and is optionally bonded via a straight-chain or branched alkyl chain of 1-5 carbon atoms to the aromatic heterocycle,<br>3) phenyl or benzyl, unsubstituted or substituted 1-3 times with<br>a) fluorine, chlorine, bromine or trifluoromethyl,<br>b) alkyl or alkenyl having up to 5 carbon atoms, it being possible for ortho alkyl or alkenyl substituents to be connected to form a fused-on carbocyclic ring,<br>c) alkoxy having 1-5 carbon atoms or<br>d) alkoxycarbonyl having 2-6 carbon atoms, |

with the exception of the compound of the formula I in which $R^1$ is methyl, $R^2$ is i-propyl, $R^3$ is methyl, $R^4$ is phenyl and $R^5$ is 4-fluorophenyl.

**2.** Compounds as claimed in claim 1, wherein in formula I

A-B denotes $-CH_2-CH_2-$
$R^1$ denotes methyl, ethyl or sodium
$R^2$, $R^3$, $R^4$ and $R^5$ denote, independently of one another,
1) hydrogen
2) methyl, ethyl, isopropyl, tert.-butyl or cyclohexyl, or
3) phenyl which can be substituted once or twice by
a) fluorine or chlorine, or
b) methyl

and the corresponding δ-lactones of the formula II.

**3.** A pharmaceutical product containing a compound as claimed in claim 1.

**4.** A compound as claimed in claim 1 for the prophylaxis and therapy of hypercholesterolemia.

**5.** A process for the preparation of compounds of the formulae I and II, which comprises
A)
1. reduction of a carbaldehyde of the formula III

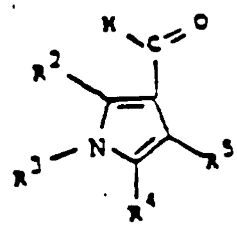

III

to an alcohol of the formula IV

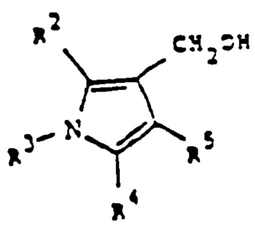

IV

2. conversion of the alcohol IV into the halogen derivative of the formula V

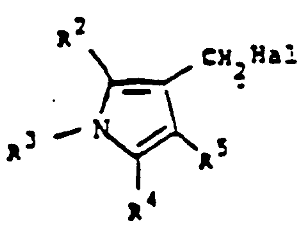

V

in which Hal denotes chlorine, bromine or iodine,
3. conversion of the halogen compounds of the formula V into the phosphonium salt of the formula VI

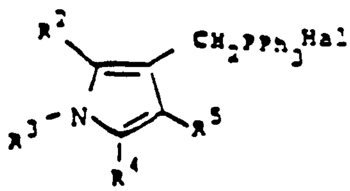

VI

in which Hal denotes chlorine, bromine or iodine,

4. reaction of the phosphonium salt of the formula VI with the compactin aldehyde of the formula VII

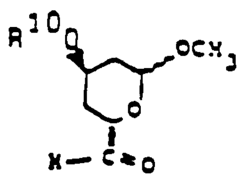

VII

in which $R^{10}$ denotes a protective group which is stable to bases and weak acids, for example t-butyl-diphenylsilyl, to give the lactol ether of the formula VIII

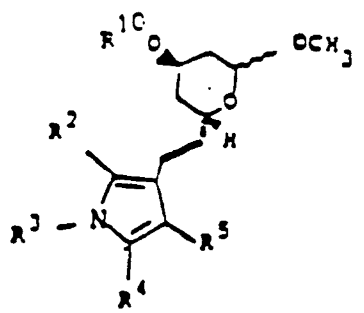

VIII

5. acid hydrolysis of the methyl acetal group in the lactol ether of the formula VIII to give a lactol of the formula IX

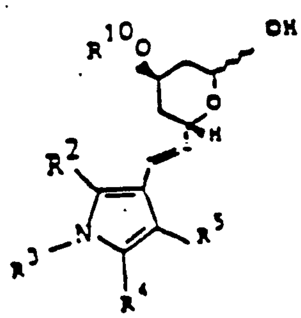

IX

6. oxidation of the lactol of the formula IX to a lactone of the formula X

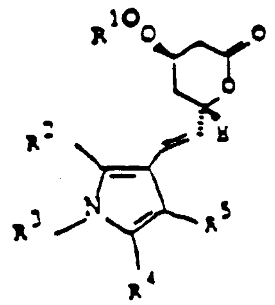

X

7. elimination of the protective group $R^{10}$, for example

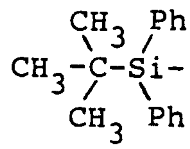

,

in the lactone of the formula X to give a compound of the formula IIa

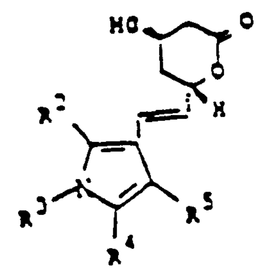

IIa

in which $R^2$ to $R^5$ have the indicated meanings, and

8. where appropriate conversion of a resulting compound of the formula IIa in which $R^2$ to $R^5$ have the indicated meanings into the corresponding dihydroxyhept-6-enoic acid of the formula Ia

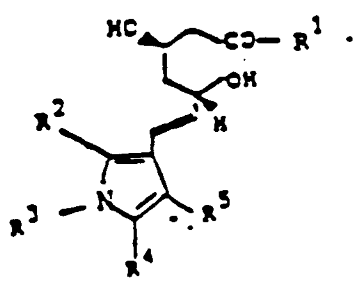

Ia

in which $R^1$ to $R^5$ have the indicated meanings, and

9. hydrogenation of the double bond in a resulting compound of the formula Ia or IIa with CH = CH to give a compound of the formula I or II with A-B equal to $CH_2$-$CH_2$, it being possible for the hydrogenation also to be carried out on the intermediates VIII, IX or X as well as before the conversion of compounds IIa into Ia, or

B)

1) subjecting the $\alpha,\beta$-unsaturated aldehyde of the formula XI

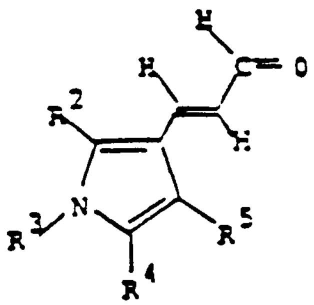

XI

to an aldol reaction with the dianion of methyl acetoacetate, resulting in a compound of the formula XII

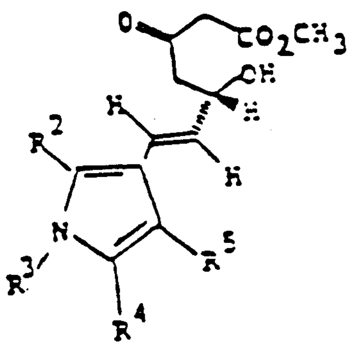

XII

2. stereoselective reduction of the keto group in a compound of the formula XII to give a compound of the formula Ia

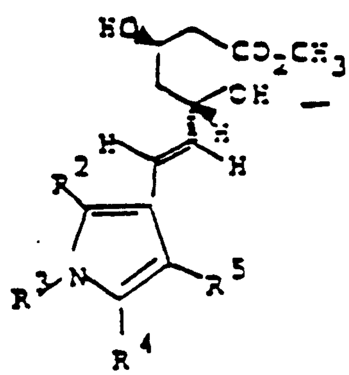

Ia

in which $R^2$ to $R^5$ have the indicated meanings and $R^1$ is the methyl group,
3. where appropriate conversion of a resulting compound of the formula Ia in which $R^1$ denotes the $CH_3$ group into a compound Ia in which $R^1$ to $R^5$ have the meanings indicated for formula I, and
4. hydrogenation of the double bond in a resulting compound of the formula Ia to give a compound of the formula I with A-B equal to $-CH_2-CH_2-$, and
5. where appropriate conversion of a resulting compound of the formula I with $R^1$ = H, with elimination of water, into a lactone of the formula II, or

C)
1. reaction of the enolate of an acetic ester of the formula XIII

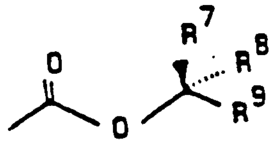

XIII

in which $R^7$ denotes a phenyl ring which is unsubstituted or substituted by fluorine, chlorine, bromine or branched or unbranched alkyl having 1-4 carbon atoms, $R^8$ denotes an H atom or methyl, and $R^9$ denotes diphenylhydroxymethyl $Ph_2COH$, the phenyl rings being unsubstituted or substituted by fluorine, chlorine, bromine or branched or unbranched alkyl having 1-4 carbon atoms, in an aldol reaction with the $\alpha,\beta$-unsaturated aldehyde of the formula XI

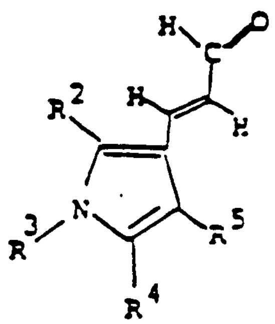

XI

to give the adduct of the formula XIV

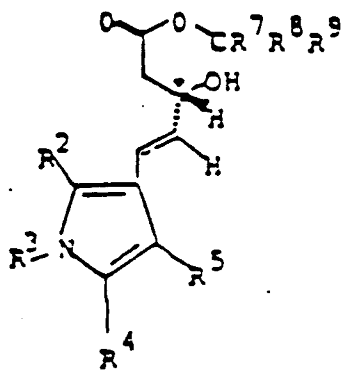

XIV

in which $R^2$ to $R^3$ have the meanings indicated for formula I, and $R^7$, $R^8$ and $R^9$ have the meanings indicated for formula XIII,

2. conversion of the adduct of the formula XIV, by transesterification, into the $\beta$-hydroxy methyl ester of the formula XV

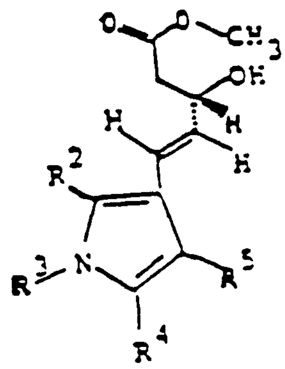

XV

3. conversion of the resulting methyl ester of the formula XV, using the enolate of an alkyl acetate, into the β-keto δ-(S)-hydroxy ester of the formula XVI

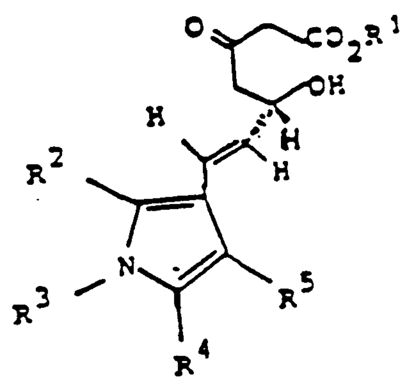

XVI

in which $R^2$ to $R^5$ have the meanings indicated for formula I, and $R^1$ is an alkyl group having 1-4 carbon atoms, and

4. stereoselective reduction of the keto group in a resulting compound of the formula XVI to give a compound of the formula Ia

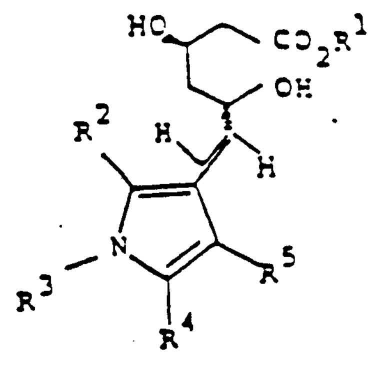

Ia

in which $R^2$ to $R^5$ have the meanings indicated for formula I and $R^1$ is an alkyl group having 1-4 carbon atoms,

5. where appropriate conversion of a resulting compound of the formula Ia in which $R^1$ is an alkyl group having 1-4 carbon atoms into a compound of the formula Ia in which $R^1$ to $R^5$ have the meanings indicated for formula I, and

6. hydrogenation of the double bond in a resulting compound of the formula Ia to give a compound of the formula I with A-B equal to $-CH_2-CH_2-$, and

7. where appropriate conversion of a resulting compound of the formula I with $R^1$ = hydrogen,

with elimination of water, into a lactone of the formula II.

**Claims for the following Contracting States : ES, GR**

**1.** A process for the preparation of 7-[1H-pyrrol-3-yl]-substituted 3,5-dihydroxyheptanoic acids and their derivatives of the formula I

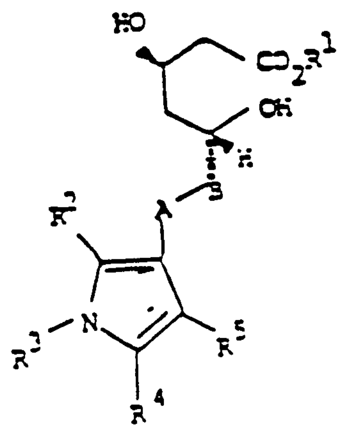

I

and corresponding δ-lactones of the formula II

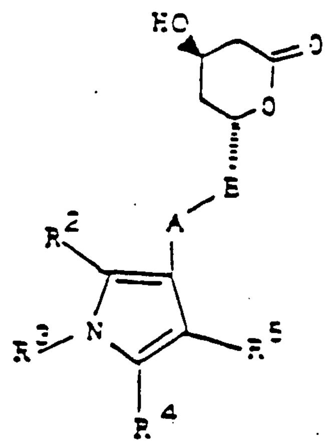

II

in which formulae

| | |
|---|---|
| A-B | denotes an ethanediyl group $-CH_2-CH_2-$, |
| $R^1$ | denotes hydrogen, alkyl having 1 to 4 carbon atoms, phenyl, benzyl or 2,3-dihydroxypropyl, or a pharmacologically tolerated alkali or alkaline earth metal cation, $NH_4$ or an ammonium ion substituted with 1 to 4 alkyl groups each having 1-4 carbon atoms, |
| $R^2$, $R^3$, $R^4$ and $R^5$ | denote, independently of one another,<br>1) hydrogen<br>2) straight-chain or branched alkyl having 1-12 carbon atoms, or cycloalkyl which has 5-7 carbon atoms and is optionally bonded via a straight-chain or branched alkyl chain of 1-5 carbon atoms to the aromatic heterocycle,<br>3) phenyl or benzyl, unsubstituted or substituted 1-3 times with<br>a) fluorine, chlorine, bromine or trifluoromethyl,<br>b) alkyl or alkenyl having up to 5 carbon atoms, it being possible for ortho alkyl or alkenyl substituents to be connected to form a fused-on carbocyclic ring,<br>c) alkoxy having 1-5 carbon atoms or<br>d) alkoxycarbonyl having 1-6 carbon atoms, |

with the exception of the compound of the formula I in which $R^1$ is methyl, $R^2$ is i-propyl, $R^3$ is methyl, $R^4$ is phenyl and $R^5$ is 4-fluorophenyl, which comprises

A)

1. reduction of a carbaldehyde of the formula III

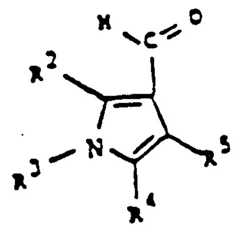

III

to an alcohol of the formula IV

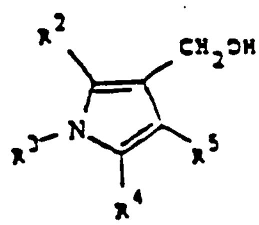

IV

2. conversion of the alcohol IV into the halogen derivative of the formula V

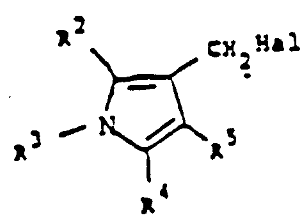

V

in which Hal denotes chlorine, bromine or iodine,

3. conversion of the halogen compounds of the formula V into the phosphonium salt of the formula VI

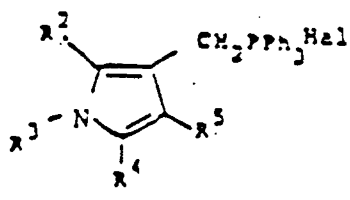

VI

in which Hal denotes chlorine, bromine or iodine,

4. reaction of the phosphonium salt of the formula VI with the compactin aldehyde of the formula VII

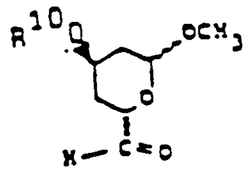

VII

in which $R^{10}$ denotes a protective group which is stable to bases and weak acids, for example t-butyl-diphenylsilyl, to give the lactol ether of the formula VIII

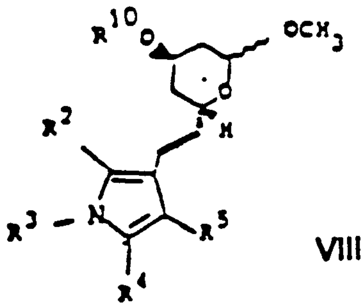

VIII

VIII

5. acid hydrolysis of the methyl acetal group in the lactol ether of the formula VIII to give a lactol of the formula IX

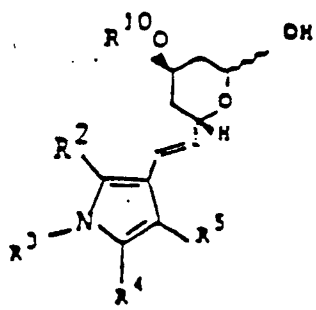

IX

6. oxidation of the lactol of the formula IX to a lactone of the formula X

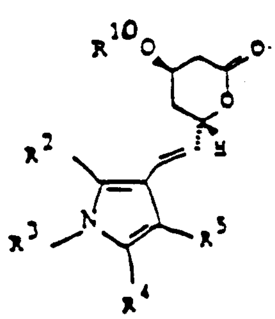

X

7. elimination of the protective group $R^{10}$, for example

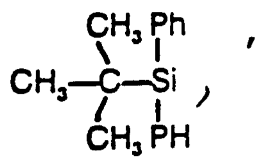

in the lactone of the formula X to give a compound of the formula IIa

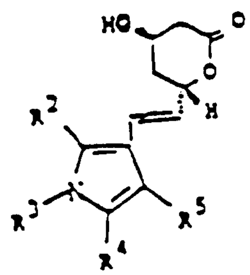

IIa

in which $R^2$ and $R^5$ have the indicated meanings, and

8. where appropriate conversion of a resulting compound of the formula IIa in which $R^2$ to $R^5$ have the indicated meanings into the corresponding dihydroxyhept-6-enoic acid of the formula Ia

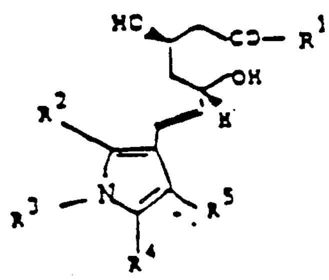

Ia

in which $R^1$ to $R^5$ have the indicated meanings, and

9. hydrogenation of the double bond in a resulting compound of the formula Ia or IIa to give a compound of the formula I or II with A-B equal to $-CH_2-CH_2-$, it being possible for the hydrogenation also to be carried out on the intermediates VIII, IX or X as well as before the conversion of compounds IIa into Ia, or

B)

1) subjecting the $\alpha,\beta$-unsaturated aldehyde of the formula XI

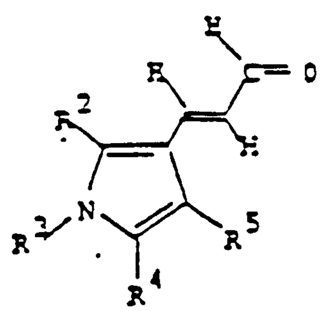

XI

to an aldol reaction with the dianion of methyl acetoacetate, resulting in a compound of the formula XII

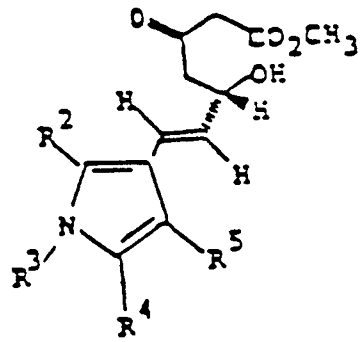

XII

2. stereoselective reduction of the keto group in a compound of the formula XII to give a compound of the formula Ia

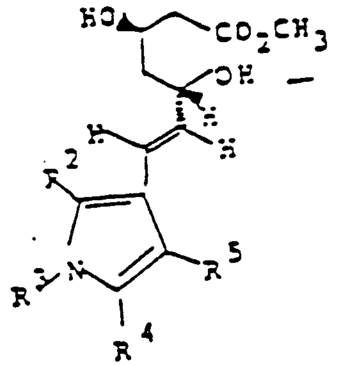

Ia

in which $R^2$ to $R^5$ have the indicated meanings and $R^1$ is the methyl group,

3. where appropriate conversion of a resulting compound of the formula Ia in which $R^1$ denotes the $CH_3$ group into a compound Ia in which $R^1$ to $R^5$ have the meanings indicated for formula I, and

4. hydrogenation of the double bond in a resulting compound of the formula Ia to give a compound of the formula I with A-B equal to $-CH_2-CH_2-$, and

5. where appropriate conversion of a resulting compound of the formula I with $R^1$ = H, with elimination of water, into a lactone of the formula II, or

C)

1. reaction of the enolate of an acetic ester of the formula XIII

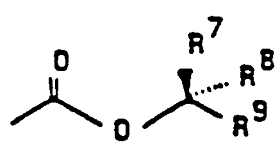

XIII

in which $R^7$ denotes a phenyl ring which is unsubstituted or substituted by fluorine, chlorine, bromine or branched or unbranched alkyl having 1-4 carbon atoms, $R^8$ denotes an H atom or methyl, and $R^9$ denotes diphenylhydroxymethyl $Ph_2COH$, the phenyl rings being unsubstituted or substituted by fluorine, chlorine, bromine or branched or unbranched alkyl having 1-4 carbon atoms, in an aldol reaction with the $\alpha,\beta$-unsaturated aldehyde of the formula XI

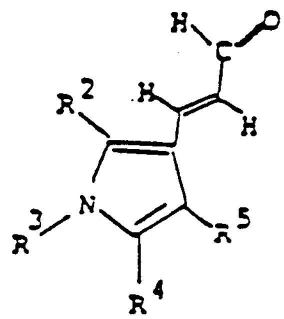

XI

to give the adduct of the formula XIV

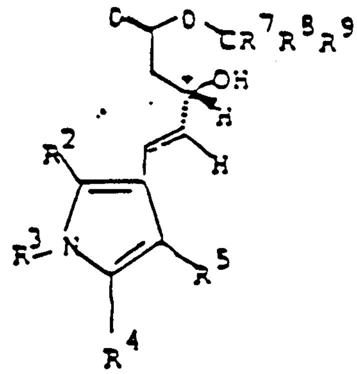

XIV

in which $R^2$ to $R^5$ have the meanings indicated for formula I, and $R^7$, $R^8$ and $R^9$ have the meanings indicated for formula XIII,

2. conversion of the adduct of the formula XIV, by transesterification, into the β-hydroxy methyl ester of the formula XV

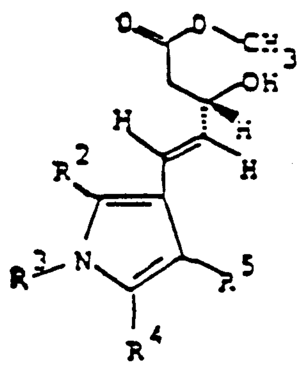

XV

3. conversion of the resulting methyl ester of the formula XV, using the enolate of an alkyl acetate, into the β-keto δ-(S)-hydroxy ester of the formula XVI

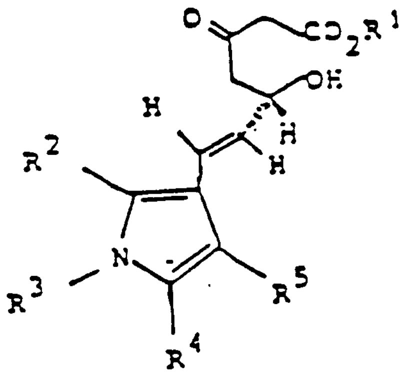

XVI

in which $R^2$ to $R^5$ have the meanings indicated for formula I, and $R^1$ is an alkyl group having 1-4 carbon atoms, and

4. stereoselective reduction of the keto group in a resulting compound of the formula XVI to give a compound of the formula Ia

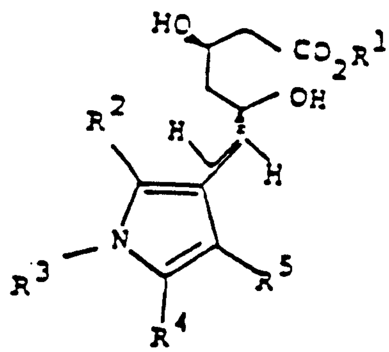

Ia

in which $R^2$ to $R^5$ have the meanings indicated for formula I and $R^1$ is an alkyl group having 1-4 carbon atoms,

5. where appropriate conversion of a resulting compound of the formula Ia in which $R^1$ is an alkyl group having 1-4 carbon atoms into a compound of the formula Ia in which $R^1$ to $R^5$ have the meanings indicated for formula I, and

6. hydrogenation of the double bond in a resulting compound of the formula Ia to give a compound of the formula I with A-B equal to $-CH_2-CH_2-$, and

7. where appropriate conversion of a resulting compound of the formula I with $R^1$ = hydrogen, with elimination of water, into a lactone of the formula II.

**2.** The process as claimed in claim 1, which comprises preparing a compound of the formula I in which

| | |
|---|---|
| A-B | denotes $-CH_2-CH_2-$ |
| $R^1$ | denotes methyl, ethyl or sodium |
| $R^2$, $R^3$, $R^4$ and $R^5$ | denote, independently of one another,<br>1) hydrogen<br>2) methyl, ethyl, isopropyl, tert.-butyl or cyclohexyl, or<br>3) phenyl which can be substituted once or twice by<br>a) fluorine or chlorine, or<br>b) methyl |

or a corresponding δ-lactone of the formula II.

**3.** A process for the production of a pharmaceutical product, which comprises converting a compound of the formula I or II as defined in claim 1 into a form suitable for administration.

**Revendications**

**Revendications pour les Etats contractants suivants : AT, BE, CH, DE, FR, GB, IT, LI, LU, NL, SE**

**1.** Acides 3,5-dihydroxyheptanoïques substitués en position 7 par le groupe 1H-pyrrol-3-yle et leurs dérivés, de formule générale I

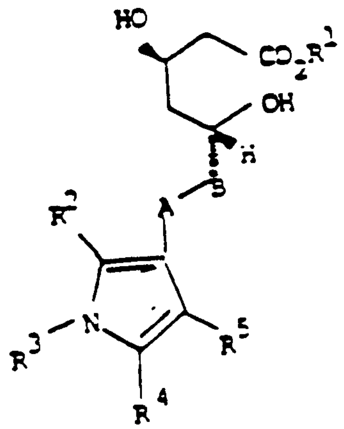

I

ainsi que δ-lactones correspondantes, de formule II

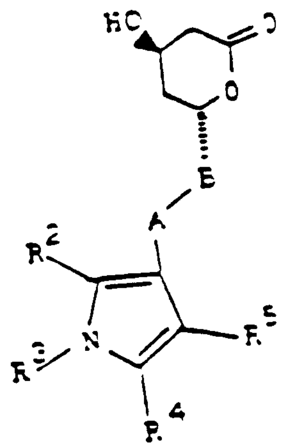

II

dans les formules

| | |
|---|---|
| A-B | représentant un groupe éthanediyle $-CH_2-CH_2-$, |
| $R^1$ | représentant un atome d'hydrogène, un groupe alkyle ayant de 1 à 4 atomes de carbone, phényle, benzyle, 2,3-dihydroxypropyle, un cation alcalin ou alcalinoterreux pharmacologiquement acceptable, $NH_4$ ou un ion ammonium substitué par 1-4 groupes alkyle ayant chacun de 1 à 4 atomes de carbone, |
| $R^2$, $R^3$, $R^4$, $R^5$ | représentant, indépendamment les uns des autres, |

1) un atome d'hydrogène,

2) un groupe alkyle à chaîne droite ou ramifiée ayant 1-12 atomes de carbone, cycloalkyle ayant 5-7 atomes de carbone, qui est éventuellement lié au noyau aromatique hétérocyclique par une chaîne alkyle linéaire ou ramifiée ayant 1-5 atomes de carbone,

3) un radical phényle ou benzyle non substitué ou 1-3 fois substitué par

a) un ou des atomes de fluor, chlore ou brome, ou le groupe trifluorométhyle,

b) un ou des groupes alkyle ou alcényle ayant jusqu'à 5 atomes de carbone, des substituants alkyle ou alcényle en position ortho pouvant être liés avec formation d'un cycle carbocyclique soudé,

c) un ou des groupes alcoxy ayant 1-5 atomes de carbone,

d) un ou des groupes alcoxycarbonyle ayant de 2 à 6 atomes de carbone,

à l'exception du composé de formule I dans lequel $R^1$ représente le groupe méthyle, $R^2$ représente le groupe isopropyle, $R^3$ représente le groupe méthyle, $R^4$ représente le groupe phényle et $R^5$ représente le groupe 4-fluorophényle.

**2.** Composés selon la revendication 1, caractérisés en ce que, dans la formule I,

A-B représente $-CH_2-CH_2-$,

$R^1$ représente le groupe méthyle ou éthyle ou un atome de sodium,

$R^2$, $R^3$, $R^4$, $R^5$ repésentent, indépendamment les uns des autres,

1) un atome d'hydrogène,

2) le groupe méthyle éthyle, isopropyle, tert-butyle, cyclohexyle,

3) un groupe phényle qui peut être 1-2 fois substitué par

a) un ou des atomes de fluor ou de chlore,

b) le groupe méthyle, ainsi que les δ-lactones correspondantes de formule générale II.

**3.** Compositions pharmaceutiques contenant un composé selon la revendication 1.

**4.** Composé selon la revendication 1, pour la prophylaxie et le traitement de l'hypercholestérolémie.

**5.** Procédé pour la préparation des composés de formules I et II, caractérisé en ce que

A)

1. on réduit un carbaldéhyde de formule III

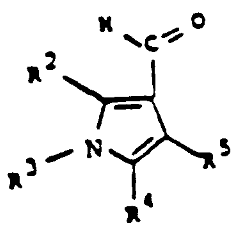

III

en un alcool de formule IV

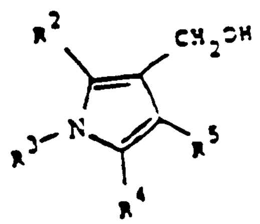

IV

2. on convertit l'alcool IV en le dérivé halogéné de formule V

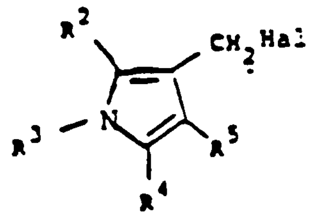

V

dans laquelle Hal représente le chlore, le brome ou l'iode,

3. on convertit les composés halogénés de formule V en le sel de phosphonium de formule VI

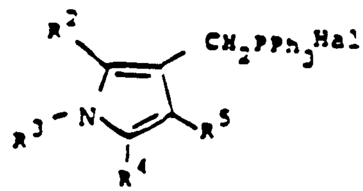

VI

dans laquelle Hal représente le chlore, le brome ou l'iode,

4. on fait réagir le sel de phosphonium de formule VI avec le compactinaldéhyde de formule VII

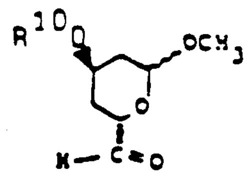

VII

dans laquelle $R^{10}$ représente un groupe protecteur stable à l'égard de bases et d'acides faibles, par exemple le groupe tert-butyldiphénylsilyle, pour aboutir au lactoléther de formule VIII

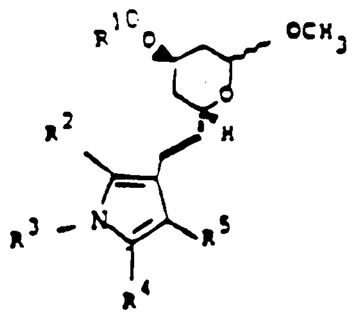

VIII

5. dans le lactoléther de formule VIII, on hydrolyse par voie acide la fonction méthylacétal pour obtenir un lactol de formule IX

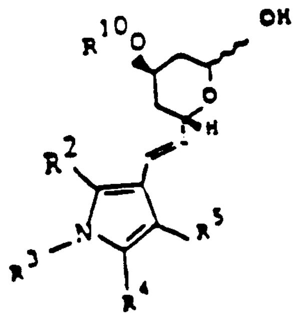

IX

6. on oxyde le lactol de formule IX en une lactone de formule X

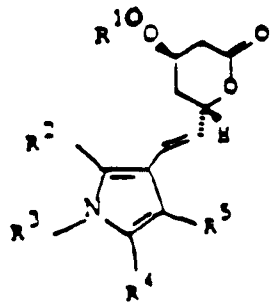

X

7. dans la lactone de formule X, on élimine le groupe protecteur $R^{10}$, par exemple

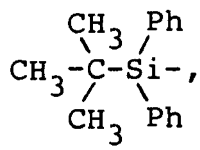

pour aboutir à un composé de formule IIa

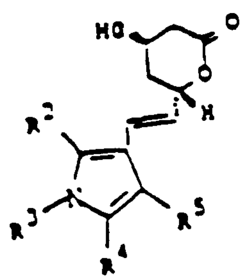

IIa

dans laquelle $R^2$ à $R^5$ ont les significations données, et

8. éventuellement on convertit un composé de formule IIa obtenu, dans lequel $R^2$ à $R^5$ ont les significations données, en l'acide dihydroxy-hept-6-énoïque de formule Ia

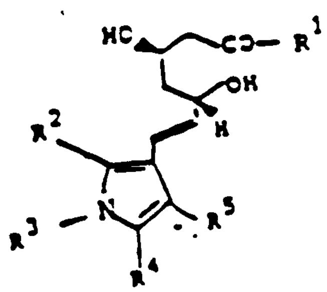

Ia

dans laquelle $R^1$ à $R^5$ ont les significations données, et

9. dans un composé de formule Ia ou IIa obtenu, comportant CH = CH, on soumet la double liaison à une hydrogénation pour aboutir à un composé de formule I ou II dans lequel A-B est $CH_2$-$CH_2$, l'hydrogénation pouvant être également effectuée sur les composés intermédiaires VIII, IX ou X ainsi qu'avant la conversion de composés IIa en Ia, ou

B)

1. on soumet l'aldéhyde $\alpha,\beta$-insaturé de formule XI

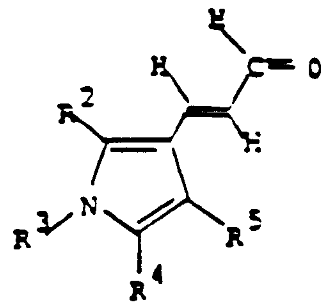

XI

à une réaction d'aldol avec le dianion de l'acétoacétate de méthyle, pour obtenir un composé de formule XII

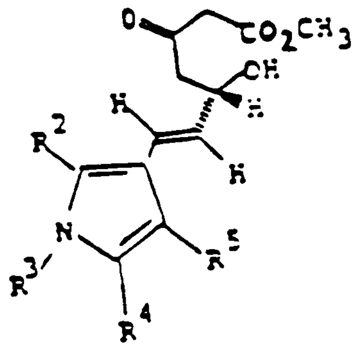

XII

2. dans un composé de formule XII, on réduit stéréosélectivement le groupe céto, pour aboutir à un composé de formule Ia

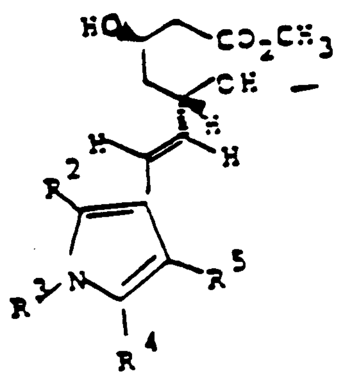

Ia

dans laquelle $R^2$ à $R^5$ ont les significations données, $R^1$ est le groupe méthyle,

3. éventuellement on convertit un composé de formule Ia obtenu, dans lequel $R^1$ représente le groupe $CH_3$, en un composé Ia dans lequel $R^1$ à $R^5$ ont les significations données à propos de la formule I, et

4. dans un composé de formule Ia obtenu, on soumet la double liaison à une hydrogénation pour aboutir à un composé de formule I dans lequel A-B est $-CH_2-CH_2-$, et

5. éventuellement on convertit un composé de formule I obtenu, dans lequel $R^1$ = H, en une lactone de formule II, avec élimination d'eau, ou

C)

1. on fait réagir l'énolate d'un ester acétique de formule XIII

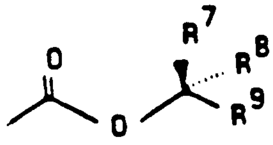

XIII

dans laquelle $R^7$ représente un noyau phényle qui n'est pas substitue ou est substitué par le fluor, le chlore, le brome, ou par un groupe alkyle ramifié ou non ramifié ayant 1-4 atomes de carbone, $R^8$ représente un atome d'hydrogène ou le groupe méthyle, et $R^9$ représente le groupe diphénylhydroxyméthyle $Ph_2COH$, les noyaux phényle n'étant pas substitués ou étant substitués par le fluor, le chlore, le brome, ou par un groupe alkyle ramifié ou non ramifié ayant 1-4 atomes de carbone, dans une réaction aldolique, avec l'aldéhyde $\alpha,\beta$-insaturéde formule XI

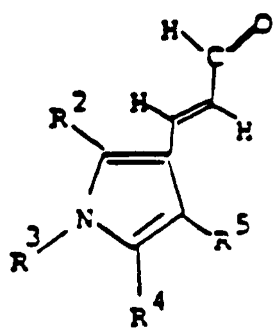

XI

pour aboutir au produit d'addition de formule XIV

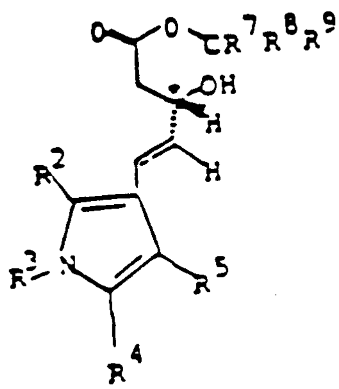

XIV

dans laquelle $R^2$ à $R^5$ ont les significations données à propos de la formule I, et $R^7$, $R^8$, $R^9$ ont les significations données à propos de la formule XIII,

2. on convertit l'adduct de formule XIV, par transestérification, en l'ester $\beta$-hydroxyméthylique de formule XV

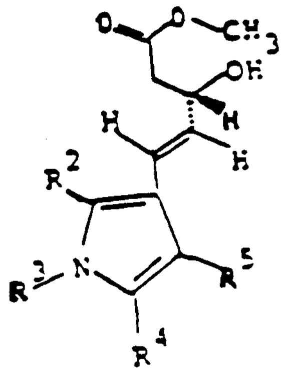

XV

3. on convertit l'ester méthylique de formule XV obtenu, avec l'énolate d'un acétate d'alkyle, en le $\beta$-céto-$\delta$-(S)-hydroxyester de formule XVI

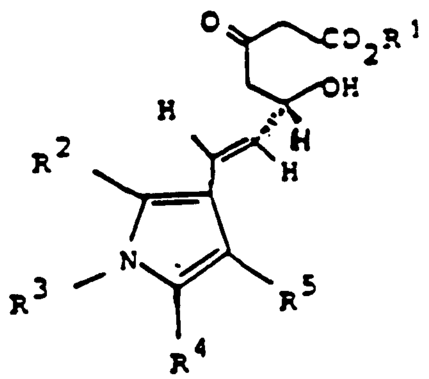

XVI

dans laquelle $R^2$ à $R^5$ ont les significations données à propos de la formule I, et $R^1$ est un groupe alkyle ayant 1-4 atomes de carbone, et
4. dans un composé de formule XVI obtenu, on réduit stéréosélectivement le groupe céto, pour aboutir à un composé de formule Ia

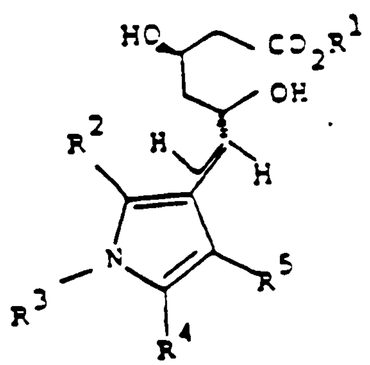

Ia

dans laquelle $R^2$ à $R^5$ ont les significations données à propos de la formule I, $R^1$ est un groupe alkyle ayant 1-4 atomes de carbone,
5. éventuellement on convertit un composé de formule Ia obtenu, dans lequel $R^1$ est un groupe alkyle ayant 1-4 atomes de carbone, en un composé de formule Ia dans lequel $R^1$ à $R^5$ ont les significations données à propos de la formule I, et
6. dans un composé de formule Ia obtenu, on soumet la double liaison à une hydrogénation pour aboutir à un composé de formule Ia dans lequel A-B est $-CH_2-CH_2-$, et
7. éventuellement on convertit un composé de formule I obtenu, dans lequel $R^1$ est un atome d'hydrogène, en une lactone de formule II, avec élimination d'eau.

**Revendications pour les Etats contractants suivants : ES, GR**

**1.** Procédé pour la préparation d'acides 3,5-dihydroxyheptanoïques substitués en position 7 par le groupe 1H-pyrrol-3-yle et de leurs dérivés, de formule générale I

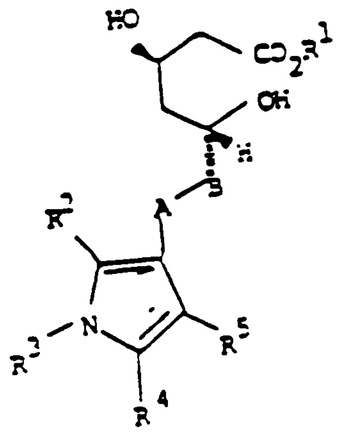

I

ainsi que des δ-lactones correspondantes, de formule II

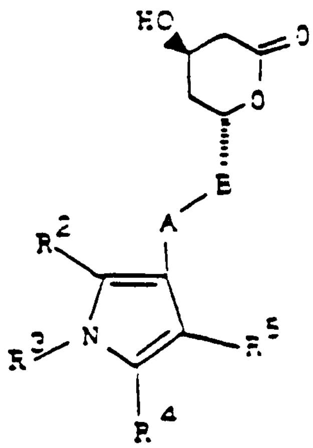

II

dans les formules

| | |
|---|---|
| A-B | représentant un groupe éthanediyle $-CH_2-CH_2-$, |
| $R^1$ | représentant un atome d'hydrogène, un groupe alkyle ayant de 1 à 4 atomes de carbone, phényle, benzyle, 2,3-dihydroxypropyle, un cation alcalin ou alcalinoterreux pharmacologiquement acceptable, $NH_4$ ou un ion ammonium substitué par 1-4 groupes alkyle ayant chacun de 1 à 4 atomes de carbone, |
| $R^2$, $R^3$, $R^4$, $R^5$ | représentant, indépendamment les uns des autres, |

1) un atome d'hydrogène,

2) un groupe alkyle à chaîne droite ou ramifiée ayant 1-12 atomes de carbone, cycloalkyle ayant 5-7 atomes de carbone, qui est éventuellement lié au noyau aromatique hétérocyclique par une chaîne alkyle linéaire ou ramifiée ayant 1-5 atomes de carbone,

3) un radical phényle ou benzyle non substitué ou 1-3 fois substitué par

a) un ou des atomes de fluor, chlore ou brome, ou le groupe trifluorométhyle,

b) un ou des groupes alkyle ou alcényle ayant jusqu'à 5 atomes de carbone, des substituants alkyle ou alcényle en position ortho pouvant être liés avec formation d'un cycle carbocyclique soudé,

c) un ou des groupes alcoxy ayant 1-5 atomes de carbone,

d) un ou des groupes alcoxycarbonyle ayant de 2 à 6 atomes de carbone,

à l'exception du composé de formule I dans lequel $R^1$ représente le groupe méthyle, $R^2$ représente le groupe isopropyle, $R^3$ représente le groupe méthyle, $R^4$ représente le groupe phényle et $R^5$ représente le groupe 4-fluorophényle,

caractérisé en ce que

A)

1. on réduit un carbaldéhyde de formule III

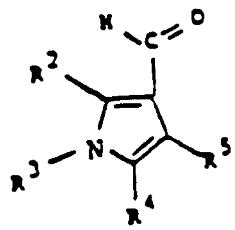

III

en un alcool de formule IV

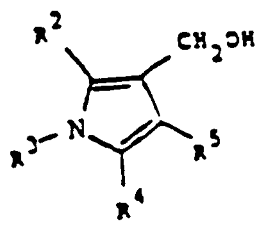

IV

2. on convertit l'alcool IV en le dérivé halogéné de formule V

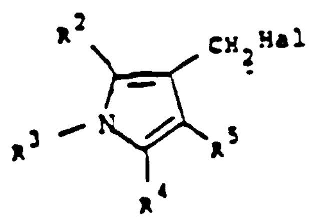

V

dans laquelle Hal représente le chlore, le brome ou l'iode,

3. on convertit les composés halogénés de formule V en le sel de phosphonium de formule VI

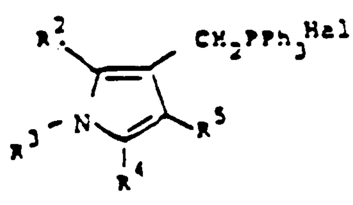

VI

dans laquelle Hal représente le chlore, le brome ou l'iode,

4. on fait réagir le sel de phosphonium de formule VI avec le compactinaldéhyde de formule VII

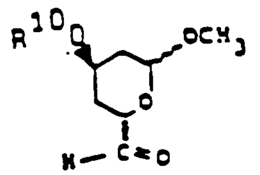

VII

dans laquelle $R^{10}$ représente un groupe protecteur stable à l'égard de bases et d'acides faibles, par exemple le groupe tert-butyldiphénylsilyle, pour aboutir au lactoléther de formule VIII

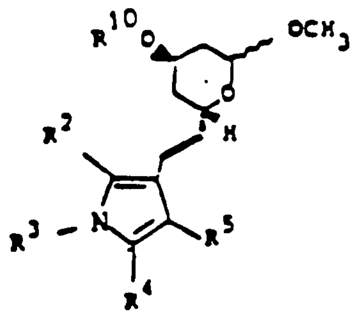

VIII

5. dans le lactoléther de formule VIII, on hydrolyse par voie acide la fonction méthylacétal pour obtenir un lactol de formule IX

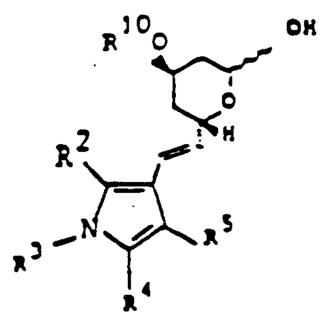

IX

6. on oxyde le lactol de formule IX en une lactone de formule X

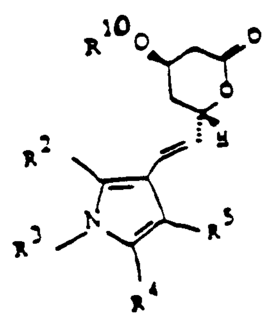

X

7. dans la lactone de formule X, on élimine le groupe protecteur $R^{10}$, par exemple

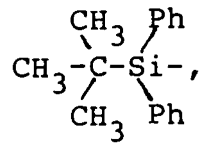

pour aboutir à un composé de formule IIa

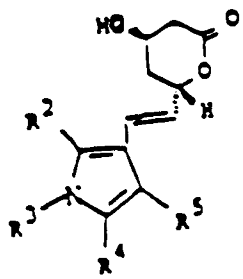

IIa

dans laquelle $R^2$ à $R^5$ ont les significations données, et

8. éventuellement on convertit un composé de formule IIa obtenu, dans lequel $R^2$ à $R^5$ ont les significations données, en l'acide dihydroxy-hept-6-énoïque de formule Ia

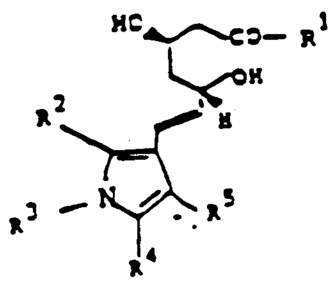

Ia

dans laquelle $R^1$ à $R^5$ ont les significations données, et

9. dans un composé de formule Ia ou IIa obtenu, comportant CH=CH, on soumet la double liaison à une hydrogénation pour aboutir à un composé de formule I ou II dans lequel A-B est $CH_2$-$CH_2$, l'hydrogénation pouvant être également effectuée sur les composés intermédiaires VIII, IX ou X ainsi qu'avant la conversion de composés IIa en Ia, ou

B)

1. on soumet l'aldéhyde $\alpha,\beta$-insaturé de formule XI

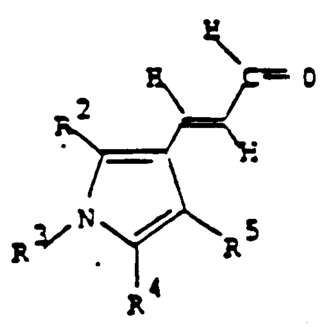

XI

à une réaction d'aldol avec le dianion de l'acétoacétate de méthyle, pour obtenir un composé de formule XII

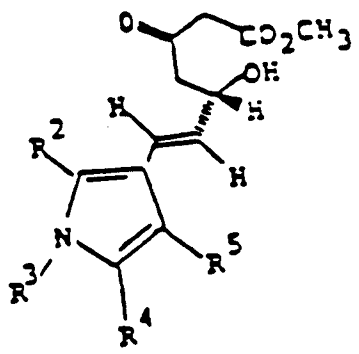

XII

2. dans un composé de formule XII, on réduit stéréosélectivement le groupe céto, pour aboutir à un composé de formule Ia

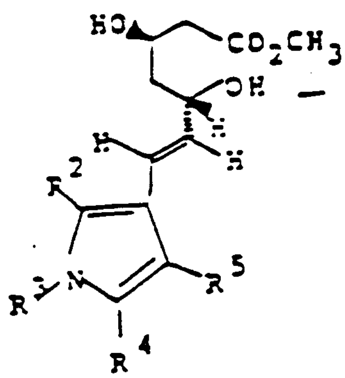

Ia

dans laquelle $R^2$ à $R^5$ ont les significations données, $R^1$ est le groupe méthyle,

3. éventuellement on convertit un composé de formule Ia obtenu, dans lequel $R^1$ représente le groupe $CH_3$, en un composé Ia dans lequel $R^1$ à $R^5$ ont les significations données à propos de la formule I, et

4. dans un composé de formule Ia obtenu, on soumet la double liaison à une hydrogénation pour aboutir à un composé de formule I dans lequel A-B est $-CH_2-CH_2-$, et

5. éventuellement on convertit un composé de formule I obtenu, dans lequel $R^1$ = H, en une lactone de formule II, avec élimination d'eau, ou

C)

1. on fait réagir l'énolate d'un ester acétique de formule XIII

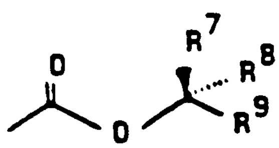

XIII

dans laquelle $R^7$ représente un noyau phényle qui n'est pas substitué ou est substitué par le fluor, le chlore, le brome, ou par un groupe alkyle ramifié ou non ramifié ayant 1-4 atomes de carbone, $R^8$ représente un atome d'hydrogène ou le groupe méthyle, et $R^9$ représente le groupe diphénylhydroxyméthyle $Ph_2COH$, les noyaux phényle n'étant pas substitués ou étant substitués par le fluor, le chlore, le brome, ou par un groupe alkyle ramifié ou non ramifié ayant 1-4 atomes de carbone, dans une réaction d'aldol, avec l'aldéhyde $\alpha,\beta$-insaturé de formule XI

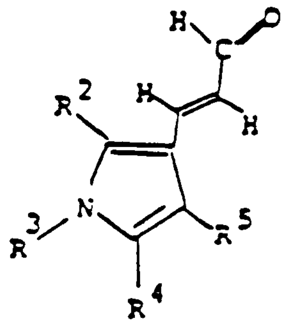

XI

pour aboutir à l'adduct de formule XIV

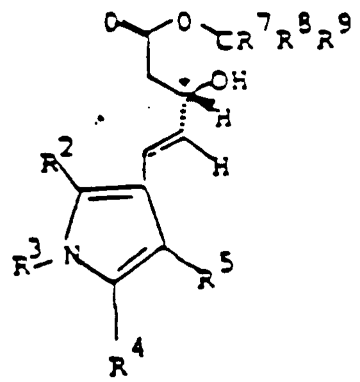

XIV

dans laquelle $R^2$ à $R^5$ ont les significations données à propos de la formule I, et $R^7$, $R^8$, $R^9$ ont les significations données à propos de la formule XIII,

2. on convertit l'adduct de formule XIV, par transestérification, en l'ester $\beta$-hydroxyméthylique de formule XV

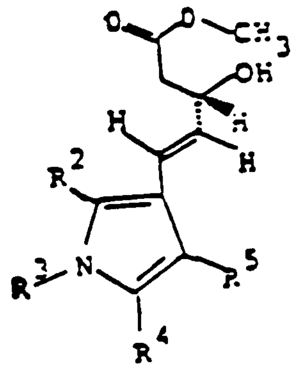

XV

3. on convertit l'ester méthylique de formule XV obtenu, avec l'énolate d'un acétate d'alkyle, en le $\beta$-céto-$\delta$-(S)-hydroxyester de formule XVI

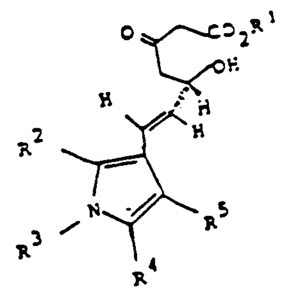

XVI

dans laquelle $R^2$ à $R^5$ ont les significations données à propos de la formule I, et $R^1$ est un groupe alkyle ayant 1-4 atomes de carbone, et

4. dans un composé de formule XVI obtenu, on réduit stéréosélectivement le groupe céto, pour aboutir à un composé de formule Ia

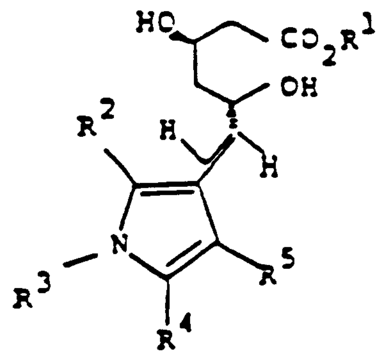

Ia

dans laquelle $R^2$ à $R^5$ ont les significations données à propos de la formule I, $R^1$ est un groupe alkyle ayant 1-4 atomes de carbone,

5. éventuellement on convertit un composé de formule Ia obtenu, dans lequel $R^1$ est un groupe alkyle ayant 1-4 atomes de carbone, en un composé de formule Ia dans lequel $R^1$ à $R^5$ ont les significations données à propos de la formule I, et

6. dans un composé de formule Ia obtenu, on soumet la double liaison à une hydrogénation pour aboutir à un composé de formule Ia dans lequel A-B est $-CH_2-CH_2-$, et

7. éventuellement on convertit un composé de formule I obtenu, dans lequel $R^1$ est un atome d'hydrogène, en une lactone de formule II, avec élimination d'eau.

**2.** Procédé selon la revendication 1, caractérisé en ce que l'on prépare un composé de formule I dans lequel

| | |
|---|---|
| A-B | représente $-CH_2-CH_2-$, |
| $R^1$ | représente le groupe méthyle ou éthyle ou un atome de sodium, |
| $R^2$, $R^3$, $R^4$, $R^5$ | représentent, indépendamment les uns des autres, |

1) un atome d'hydrogène,
2) le groupe méthyle, éthyle, isopropyle, tert-butyle, cyclohexyle,
3) un groupe phényle qui peut être 1-2 fois substitué par
   a) un ou des atomes de fluor ou de chlore,
   b) le groupe méthyle, ou une δ-lactone correspondante de formule générale II.

**3.** Procédé pour la préparation d'une composition pharmaceutique, caractérisé en ce que l'on met sous une forme pharmaceutique appropriée un composé de formule I ou II selon la revendication 1.